# EUROPEAN PATENT APPLICATION

(11) **EP 4 821 497 A1**
(43) Date of publication of application: **14.10.2026**
(21) Application number: 26157088.1
(22) Date of filing: 05.10.2020
(51) Int. Cl.: A61K 31/519, A61K 31/352, A61K 31/436, A61K 31/501, A61K 31/522, A61K 31/4184, A61K 31/4709, A61K 38/13, A61P 13/12, G01N 33/68

(54) **BIOMARKER-BASED TREATMENT OF FOCAL SEGMENTAL GLOMERULOSCLEROSIS AND DIABETIC KIDNEY DISEASE**

(30) Priority: 04.10.2019 US 201962910758 P
(62) Document number(s) of the earlier application(s) in accordance with Art. 76 EPC: 20871404.8 / 4 037 680
(71) Applicant: GFB (ABC), LLC, Foxboro, MA 02035 (US)
(72) Inventor: REILLY, John Francis, Framingham, MA 01701 (US)
(74) Representative: EIP

(57) **Abstract**

Disclosed are compounds having structural formulas (I)-(XI), and related pharmaceutical compositions. Also disclosed are methods of selecting and treating human subjects suffering from a kidney disease, using the compounds of formulas (I)-(XI), and methods of determining the efficacy of TRPC5 inhibitor therapies using the same.

## Description

### RELATED APPLICATIONS

This application claims the benefit of priority to U.S. Provisional Application No. 62/910,758, filed October 4, 2019.

### BACKGROUND

Mammalian TRP channel proteins form six-transmembrane cation-permeable channels which may be grouped into six subfamilies on the basis of amino acid sequence homology (TRPC, TRPV, TRPM, TRPA, TRPP, and TRPML). Recent studies of TRP channels indicate that they are involved in numerous fundamental cell functions and are considered to play an important role in the pathophysiology of many diseases. Many TRPs are expressed in kidney along different parts of the nephron and growing evidence suggest that these channels are involved in hereditary, as well as acquired kidney disorders. TRPC6, TRPM6, and TRPP2 have been implicated in hereditary focal segmental glomerulosclerosis (FSGS), hypomagnesemia with secondary hypocalcemia (HSH), and polycystic kidney disease (PKD), respectively.

Injury and loss of podocytes is a central component to the pathogenesis of FSGS and diabetic kidney disease (DKD) (Jefferson et al. 2014; Weil et al. 2012; Lin et al. 2016). FSGS is considered a primary podocytopathy, indicating that the pathogenesis is the result of podocyte dysfunction and injury. While several mechanisms have been postulated as the inciting cause for podocyte injury, among the recently described genetic causes of the disease are mutations in Rho-GTPases, regulators of actin cytoskeleton dynamics (Wen et al. 2018). Loss of appropriately functioning Rho-GTPases (e.g., mutations in ARHGAP24 and ARHGDIA) leads to the unopposed activation of Rac1 within podocytes, which promotes cytoskeletal remodeling and podocyte death through an elevation of cytoplasmic calcium and reactive oxygen species (Akilesh et al. 2011; Gee et al. 2013; Greka et al. 2011). Many familial and sporadic forms of FSGS have been linked to genetic dysregulation of Rac1, highlighting the importance of Rac1 as a driver in the disease (Lovric et al. 2015).

Experimental data, both in vitro and in vivo, support a role for Rac1 activation in the pathogenesis of DKD. In a diabetic milieu, cultured podocytes undergo cytoskeletal remodeling as well as epithelial-to-mesenchymal transition; both are abrogated by knockdown of Rac1 (Liu et al. 2013). Furthermore, podocyte-specific Rac1-deficient mice are protected from diabetic nephropathy (Liu et al. 2018). The activation of the Rac1 pathway is also mediated directly by the activation of the TRPC5 channel, through either the epithelial growth factor receptor, toll-like receptors (TLR), or AT1R (Liu et al. 2018), all of which are implicated in the pathogenesis of DKD (Greka et al. 2011). Synaptopodin, an actin-associated podocyte protein that is degraded following the activation of TRPC5, has been detected in the urine of patients with DKD (Zheng et al. 2011), further supporting the relevance of Rac1 activation in DKD. Given the role of TRPC5-Rac1 signaling in mediating injury in DKD, inhibition of TRPCS represents a viable treatment option in this area of high unmet medical need.

Based on United States (US) Renal Data System estimates, FSGS accounts for 4% of adult and 12% of pediatric incident ESKD patients (USRDS 2018a; USRDS 2018b). The overall incidence of FSGS is estimated as 0.2/100,000/year and 1.1/100,000/year, with the range attributed to geographic differences in biopsy rates and possibly genetic differences among populations (McGrogan et al. 2011; Rosenberg 2017). In a study of analyzing kidney biopsy diagnosis by glomerular disease subtype in the US and Canada, 19.1% were FSGS (O'Shaughnessy et al. 2018). FSGS is the cause of an estimated 40% of nephrotic syndrome in adults and 20% of nephrotic syndrome in children (Kitiyakara et al. 2003).

Currently, there is no approved therapy in the US with a specific indication for treatment of FSGS. In general, for both pediatric patients and adults, initial therapy comprises renin angiotensin aldosterone system (RAAS) blockade and corticosteroids (KDIGO 2012; D'Agati et al. 2011). In FSGS, the response to corticosteroids is often incomplete or, if remission is achieved, patients frequently relapse if treatment is discontinued and therefore may become dependent on long-term corticosteroid administration. Patients with FSGS who do not respond to corticosteroids are administered calcineurin inhibitors (CNIs), or in some cases other immunomodulating agents, to achieve a reduction in proteinuria (D'Agati et al. 2011; Gipson et al. 2011a; Ochi et al. 2012). Overall, while proteinuria remission may be achieved with corticosteroids or CNIs, the toxicity associated with long-term use limits chronic use of these agents at effective dose levels (Gipson et al. 2011a; Gipson et al. 2007). Where the need for treatment outweighs the associated toxicity, patients, particularly children, may be left with long-term impacts to their health. Given the importance of achieving a meaningful reduction in proteinuria, the limited efficacy of currently available treatments, and the significant side effect profile associated with these agents, there is a need to develop novel therapies to treat patients of all ages with TR-MCD or FSGS.

Diabetes, with an estimated worldwide prevalence of 415 million patients, is a major cause of morbidity and mortality both in the US and across the globe (Ogurtsova et al. 2017). Diabetic kidney disease is a major consequence of longstanding diabetes, is estimated to develop in approximately 40% of diabetic patients, and is associated with significantly increased rates of ESKD, cardiovascular complications, and early mortality (Alicic et al. 2017). Despite overall improvements in control of hyperglycemia and hypertension, patients continue to experience progressive loss of kidney function. Compared to individuals with diabetes but without kidney disease, patients with DKD are at an increased mortality risk, with a standardized 10-year cumulative incidence of mortality of up to 47% (Afkarian et al. 2013). These data support the need for a renewed focus on targeted drug development for patients with diabetes and kidney disease.

### SUMMARY

One aspect of the invention are methods of selecting and treating a human subject suffering from a kidney disease. In some embodiments, the method comprises the steps of:
a. selecting the subject if a urinary level of one or more biomarkers selected from Rac1, Rac1-GTP, phospho-LIM kinase 1, and phospho-cofilin in the subject is above a pre-determined threshold; and
b. administering to the selected subject a pharmaceutical composition comprising a TRPC5 inhibitor or a calcineurin inhibitor; and a pharmaceutically acceptable carrier.

In one aspect, the invention relates to a method of treating a human subject suffering from a kidney disease comprising the step of:
administering to the subject a pharmaceutical composition comprising
   a TRPC5 inhibitor or a calcineurin inhibitor, and
   a pharmaceutically acceptable carrier;
only if the subject is determined to have a pre-treatment urinary level of one or more biomarkers selected from Rac1, Rac1-GTP, phospho-LIM kinase 1, and phospho-cofilin that is above a pre-determined threshold.

In some embodiments, the kidney disease is diabetic nephropathy, focal segmental glomerulosclerosis, minimal change disease, membranoproliferative glomerulonephritis, membranous nephropathy, other hepatitis C virus-associated glomerulopathies, or Alport syndrome.

In one aspect, the invention relates to a method of determining the efficacy of a TRPC5 inhibitor therapy in a human subject suffering from a kidney disease, wherein prior to commencing the therapy the subject was determined to have a pre-treatment urinary level of one or more biomarkers selected from Rac1, Rac1-GTP, phospho-LIM kinase 1, and phospho-cofilin that is above a pre-determined threshold, the method comprising:
a. obtaining the urinary level of the selected biomarker in the human subject at a time after initiation of TRPC5 therapy;
b. comparing the level of the selected biomarker in step a. with the pre-treatment urinary level of the selected biomarker;
c. determining that the TRPC5 inhibitor therapy is efficacious if the level of the selected biomarker in step a. is lower than the pre-treatment urinary level of the selected biomarker.

In one aspect, the invention relates to a method of determining the efficacy of a TRPC5 inhibitor therapy in a human subject suffering from a kidney disease, wherein prior to commencing the therapy the subject was determined to have a pre-treatment urinary level of one or more biomarkers selected from Rac1, Rac1-GTP, phospho-LIM kinase 1, and phospho-cofilin that is above a pre-determined threshold, the method comprising:
a. obtaining the urinary level of the selected biomarker in the human subject at a time after initiation of TRPC5 therapy; and
b. determining that the TRPC5 inhibitor therapy is efficacious if the level of the selected biomarker in step a. is lower than the pre-determined threshold for the selected biomarker.

The methods are effective for a variety of subjects including mammals, e.g., humans and other animals, such as laboratory animals, e.g., mice, rats, rabbits, or monkeys, or domesticated and farm animals, e.g., cats, dogs, goats, sheep, pigs, cows, or horses. In some embodiments, the subject is a human.

The invention provides several advantages. The prophylactic and therapeutic methods described herein are effective in treating kidney disease, e.g., proteinuria, and have minimal, if any, side effects. Further, methods described herein are effective to identify compounds that treat or reduce risk of developing a kidney disease, anxiety, depression, or cancer.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

Other features, objects, and advantages of the invention will be apparent from the detailed description, and from the claims.

### BRIEF DESCRIPTION OF THE DRAWINGS

**FIG. 1A** and **FIG. 1B** depict scatter plots showing urinary Rac1 levels in healthy humans (circles), and in DN (squares), FSGS (diamonds) and Alport syndrome (triangles) human patients (FIG 1A), or urinary Rac1 levels in a greater number of healthy humans (circles), a greater number of DN patients (squares), a greater number of FSGS patients (diamonds), PKD patients (hexagons) and the same number of Alport syndrome patients (triangles) (FIG 1B).
**FIG. 2** depicts the ratio of Rac1:creatinine in the urine of naïve rats over time following treatment with 10 mg/kg Compound 1 or a vehicle control.
**FIG. 3** depicts the ratio of Rac1:creatinine in the urine of DOCA-treated DOCA-salt hypertensive rats over time following treatment with 10 mg/kg Compound 1 or a vehicle control.
**FIG. 4A** and **FIG. 4B** depict the change Rac1:creatinine in the urine of healthy humans over time following treatment with a single oral dose of 20 mg of Compound 1 or a placebo expressed as percent of the pre-treatment Rac1:creatinine ratio (FIG. 4A), or a single, oral dose of either a placebo, 5 mg of Compound 1 as a liquid suspension or 20, 40 or 80 mg of Compound 1 as tablets (FIG. 4B) expressed as percent of the pre-treatment Rac1 concentration.
**FIG. 5** depicts the amount of Rac1 in the urinary extracellular vesicle fraction versus the supernatant in healthy humans.
**FIG. 6** depicts the daily amount of albumin excreted in the urine of ZDSD rats over time following treatment with different doses of Compound 1 (3 mg/kg or 10 mg/kg) or a control vehicle.
**FIG. 7** depicts the daily amount of albumin excreted in the urine of DOCA-treated DOCA-salt hypertensive rats over time following treatment with different doses of Compound 1 (3 mg/kg or 10 mg/kg) or a control vehicle.
**FIG. 8** depicts the urinary protein:creatinine ratio ("UPCR") in COL4A4 knockout mice over time following treatment with different doses of Compound 1 (3 mg/kg or 10 mg/kg) or a control vehicle.
**FIG. 9** depicts the daily amount of albumin excreted in the urine of DOCA-treated DOCA-salt hypertensive rats over time following treatment with different doses of Compound 2 (10 mg/kg or 60 mg/kg stepped up to 100 mg/kg after 1 week) or a control vehicle.
**FIG. 10** depicts the daily amount of albumin excreted in the urine of DOCA-treated DOCA-salt hypertensive rats over time following treatment with different doses of Compound 3 (30 mg/kg), eplerenone (50 mg/kg BID), or a control vehicle.
**FIG. 11** depicts the daily amount of protein excreted in the urine of DOCA-treated DOCA-salt hypertensive rats over time following treatment with different doses of Compound 4 (20 mg/kg, 50 mg/kg, or 100 mg/kg)) or a control vehicle.
**FIG. 12** depicts the daily amount of albumin excreted in the urine of DOCA-treated DOCA-salt hypertensive rats over time following treatment with cyclosporine A (3 mg/kg), tacrolimus (0.3 mg/kg reduced to 0.1 mg/kg after 14 days), or a control vehicle.
**FIG. 13** depicts the urinary Rac1 levels in six human subjects that were diagnosed as having active acute kidney injury following a positive PCR test for COVID-19.

### DETAILED DESCRIPTION

### Definitions

The term "acyl" is art-recognized and refers to a group represented by the general formula hydrocarbylC(O)-, preferably alkylC(O)-.

The term "acylamino" is art-recognized and refers to an amino group substituted with an acyl group and may be represented, for example, by the formula hydrocarbylC(O)NH-.

The term "acyloxy" is art-recognized and refers to a group represented by the general formula hydrocarbylC(O)O-, preferably alkylC(O)O-.

The term "alkoxy" refers to an alkyl group, preferably a lower alkyl group, having an oxygen attached thereto. Representative alkoxy groups include methoxy, trifluoromethoxy, ethoxy, propoxy, tert-butoxy and the like.

The term "alkoxyalkyl" refers to an alkyl group substituted with an alkoxy group and may be represented by the general formula alkyl-O-alkyl.

The term "alkenyl", as used herein, refers to an aliphatic group containing at least one double bond and is intended to include both "unsubstituted alkenyls" and "substituted alkenyls", the latter of which refers to alkenyl moieties having substituents replacing a hydrogen on one or more carbons of the alkenyl group. Such substituents may occur on one or more carbons that are included or not included in one or more double bonds. Moreover, such substituents include all those contemplated for alkyl groups, as discussed below, except where stability is prohibitive. For example, substitution of alkenyl groups by one or more alkyl, carbocyclyl, aryl, heterocyclyl, or heteroaryl groups is contemplated.

An "alkyl" group or "alkane" is a straight chained or branched non-aromatic hydrocarbon which is completely saturated. Typically, a straight chained or branched alkyl group has from 1 to about 20 carbon atoms, preferably from 1 to about 10 unless otherwise defined. Examples of straight chained and branched alkyl groups include methyl, ethyl, n-propyl, iso-propyl, n-butyl, sec-butyl, tert-butyl, pentyl, hexyl, pentyl and octyl. A C₁-C₆ straight chained or branched alkyl group is also referred to as a "lower alkyl" group.

Moreover, the term "alkyl" (or "lower alkyl") as used throughout the specification, examples, and claims is intended to include both "unsubstituted alkyls" and "substituted alkyls", the latter of which refers to alkyl moieties having substituents replacing a hydrogen on one or more carbons of the hydrocarbon backbone. Such substituents, if not otherwise specified, can include, for example, a halogen (e.g., fluoro), a hydroxyl, a carbonyl (such as a carboxyl, an alkoxycarbonyl, a formyl, or an acyl), a thiocarbonyl (such as a thioester, a thioacetate, or a thioformate), an alkoxy, a phosphoryl, a phosphate, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or an aromatic or heteroaromatic moiety. In preferred embodiments, the substituents on substituted alkyls are selected from C₁₋₆ alkyl, C₃₋₆ cycloalkyl, halogen, carbonyl, cyano, or hydroxyl. In more preferred embodiments, the substituents on substituted alkyls are selected from fluoro, carbonyl, cyano, or hydroxyl. It will be understood by those skilled in the art that the moieties substituted on the hydrocarbon chain can themselves be substituted, if appropriate. For instance, the substituents of a substituted alkyl may include substituted and unsubstituted forms of amino, azido, imino, amido, phosphoryl (including phosphonate and phosphinate), sulfonyl (including sulfate, sulfonamido, sulfamoyl and sulfonate), and silyl groups, as well as ethers, alkylthios, carbonyls (including ketones, aldehydes, carboxylates, and esters), -CF₃, -CN and the like. Exemplary substituted alkyls are described below. Cycloalkyls can be further substituted with alkyls, alkenyls, alkoxys, alkylthios, aminoalkyls, carbonyl-substituted alkyls, -CF₃, -CN, and the like.

Unless otherwise specified, "alkylene" by itself or as part of another substituent refers to a saturated straight-chain or branched divalent group having the stated number of carbon atoms and derived from the removal of two hydrogen atoms from the corresponding alkane. Examples of straight chained and branched alkylene groups include -CH₂- (methylene), -CH₂-CH₂-(ethylene), -CH₂-CH₂-CH₂- (propylene), -C(CH₃)₂-, -CH₂-CH(CH₃)-, -CH₂-CH₂-CH₂-CH₂-, -CH₂-CH₂-CH₂-CH₂-CH₂- (pentylene), -CH₂-CH(CH₃)-CH₂-, and -CH₂-C(CH₃)₂-CH₂-.

The term "C_{x-y}" when used in conjunction with a chemical moiety, such as, acyl, acyloxy, alkyl, alkenyl, alkynyl, or alkoxy is meant to include groups that contain from x to y carbons in the chain. For example, the term "C_{x-y} alkyl" refers to substituted or unsubstituted saturated hydrocarbon groups, including straight-chain alkyl and branched-chain alkyl groups that contain from x to y carbons in the chain, including haloalkyl groups. Preferred haloalkyl groups include trifluoromethyl, difluoromethyl, 2,2,2-trifluoroethyl, and pentafluoroethyl. C₀ alkyl indicates a hydrogen where the group is in a terminal position, a bond if internal. The terms "C_{2-y} alkenyl" and "C_{2-y} alkynyl" refer to substituted or unsubstituted unsaturated aliphatic groups analogous in length and possible substitution to the alkyls described above, but that contain at least one double or triple bond respectively.

The term "alkylamino", as used herein, refers to an amino group substituted with at least one alkyl group.

The term "alkylthio", as used herein, refers to a thiol group substituted with an alkyl group and may be represented by the general formula alkylS-.

The term "alkynyl", as used herein, refers to an aliphatic group containing at least one triple bond and is intended to include both "unsubstituted alkynyls" and "substituted alkynyls", the latter of which refers to alkynyl moieties having substituents replacing a hydrogen on one or more carbons of the alkynyl group. Such substituents may occur on one or more carbons that are included or not included in one or more triple bonds. Moreover, such substituents include all those contemplated for alkyl groups, as discussed above, except where stability is prohibitive. For example, substitution of alkynyl groups by one or more alkyl, carbocyclyl, aryl, heterocyclyl, or heteroaryl groups is contemplated.

The term "amide", as used herein, refers to a group 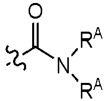 wherein each R^{A} independently represent a hydrogen or hydrocarbyl group, or two R^{A} are taken together with the N atom to which they are attached complete a heterocycle having from 4 to 8 atoms in the ring structure.

The terms "amine" and "amino" are art-recognized and refer to both unsubstituted and substituted amines and salts thereof, e.g., a moiety that can be represented by 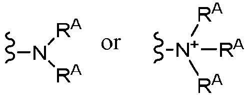 wherein each R^{A} independently represents a hydrogen or a hydrocarbyl group, or two R^{A} are taken together with the N atom to which they are attached complete a heterocycle having from 4 to 8 atoms in the ring structure.

The term "aminoalkyl", as used herein, refers to an alkyl group substituted with an amino group.

The term "aralkyl", as used herein, refers to an alkyl group substituted with an aryl group.

The term "aryl" as used herein include substituted or unsubstituted single-ring aromatic groups in which each atom of the ring is carbon. Preferably, the ring is a 6- or 10-membered ring, more preferably a 6-membered ring. The term "aryl" also includes polycyclic ring systems having two or more cyclic rings in which two or more carbons are common to two adjoining rings wherein at least one of the rings is aromatic, e.g., the other cyclic rings can be cycloalkyls, cycloalkenyls, aryls, heteroaryls, and/or heterocyclyls. Aryl groups include benzene, naphthalene, phenanthrene, phenol, aniline, and the like.

The term "carbamate" is art-recognized and refers to a group 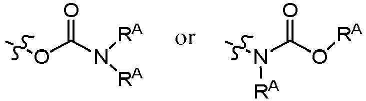 wherein each R^{A} independently represent hydrogen or a hydrocarbyl group, such as an alkyl group, or both R^{A} taken together with the intervening atom(s) complete a heterocycle having from 4 to 8 atoms in the ring structure.

The terms "carbocycle", and "carbocyclic", as used herein, refers to a saturated or unsaturated ring in which each atom of the ring is carbon. The term carbocycle includes both aromatic carbocycles and non-aromatic carbocycles. Non-aromatic carbocycles include both cycloalkane rings, in which all carbon atoms are saturated, and cycloalkene rings, which contain at least one double bond. "Carbocycle" includes 5-7 membered monocyclic and 8-12 membered bicyclic rings. Each ring of a bicyclic carbocycle may be selected from saturated, unsaturated and aromatic rings. Carbocycle includes bicyclic molecules in which one, two or three or more atoms are shared between the two rings. The term "fused carbocycle" refers to a bicyclic carbocycle in which each of the rings shares two adjacent atoms with the other ring. Each ring of a fused carbocycle may be selected from saturated, unsaturated and aromatic rings. In an exemplary embodiment, an aromatic ring, e.g., phenyl, may be fused to a saturated or unsaturated ring, e.g., cyclohexane, cyclopentane, or cyclohexene. Any combination of saturated, unsaturated and aromatic bicyclic rings, as valence permits, is included in the definition of carbocyclic. Exemplary "carbocycles" include cyclopentane, cyclohexane, bicyclo[2.2.1]heptane, 1,5-cyclooctadiene, 1,2,3,4-tetrahydronaphthalene, bicyclo[4.2.0]oct-3-ene, naphthalene and adamantane. Exemplary fused carbocycles include decalin, naphthalene, 1,2,3,4-tetrahydronaphthalene, bicyclo[4.2.0]octane, 4,5,6,7-tetrahydro-1H-indene and bicyclo[4.1.0]hept-3-ene. "Carbocycles" may be substituted at any one or more positions capable of bearing a hydrogen atom.

A "cycloalkyl" group is a cyclic hydrocarbon which is completely saturated. "Cycloalkyl" includes monocyclic and bicyclic rings. Typically, a monocyclic cycloalkyl group has from 3 to about 10 carbon atoms, more typically 3 to 8 carbon atoms unless otherwise defined. The second ring of a bicyclic cycloalkyl may be selected from saturated, unsaturated and aromatic rings. Cycloalkyl includes bicyclic molecules in which one, two or three or more atoms are shared between the two rings. The term "fused cycloalkyl" refers to a bicyclic cycloalkyl in which each of the rings shares two adjacent atoms with the other ring. The second ring of a fused bicyclic cycloalkyl may be selected from saturated, unsaturated and aromatic rings. A "cycloalkenyl" group is a cyclic hydrocarbon containing one or more double bonds.

The term "carbocyclylalkyl", as used herein, refers to an alkyl group substituted with a carbocycle group.

The term "carbonate" is art-recognized and refers to a group -OCO₂-R^{A}, wherein R^{A} represents a hydrocarbyl group.

The term "carboxy", as used herein, refers to a group represented by the formula -CO₂H.

The term "ester", as used herein, refers to a group -C(O)OR^{A} wherein R^{A} represents a hydrocarbyl group.

The term "ether", as used herein, refers to a hydrocarbyl group linked through an oxygen to another hydrocarbyl group. Accordingly, an ether substituent of a hydrocarbyl group may be hydrocarbyl-O-. Ethers may be either symmetrical or unsymmetrical. Examples of ethers include, but are not limited to, heterocycle-O-heterocycle and aryl-O-heterocycle. Ethers include "alkoxyalkyl" groups, which may be represented by the general formula alkyl-O-alkyl.

The terms "halo" and "halogen" as used herein means halogen and includes chloro, fluoro, bromo, and iodo.

The terms "hetaralkyl" and "heteroaralkyl", as used herein, refers to an alkyl group substituted with a hetaryl group.

The term "heteroalkyl", as used herein, refers to a saturated or unsaturated chain of carbon atoms and at least one heteroatom, wherein no two heteroatoms are adjacent.

The terms "heteroaryl" and "hetaryl" include substituted or unsubstituted aromatic single ring structures, preferably 5- to 7-membered rings, more preferably 5- to 6-membered rings, whose ring structures include at least one heteroatom, preferably one to four heteroatoms, more preferably one or two heteroatoms. The terms "heteroaryl" and "hetaryl" also include polycyclic ring systems having two or more cyclic rings in which two or more carbons are common to two adjoining rings wherein at least one of the rings is heteroaromatic, e.g., the other cyclic rings can be cycloalkyls, cycloalkenyls, aryls, heteroaryls, and/or heterocyclyls. Heteroaryl groups include, for example, pyrrole, furan, thiophene, imidazole, oxazole, thiazole, pyrazole, pyridine, pyrazine, pyridazine, and pyrimidine, and the like.

The term "heteroatom" as used herein means an atom of any element other than carbon or hydrogen. Preferred heteroatoms are nitrogen, oxygen, and sulfur.

The terms "heterocyclyl", "heterocycle", and "heterocyclic" refer to substituted or unsubstituted non-aromatic ring structures, preferably 3- to 10-membered rings, more preferably 3- to 7-membered rings, whose ring structures include at least one heteroatom, preferably one to four heteroatoms, more preferably one or two heteroatoms. The terms "heterocyclyl" and "heterocyclic" also include polycyclic ring systems having two or more cyclic rings in which two or more carbons are common to two adjoining rings wherein at least one of the rings is heterocyclic, e.g., the other cyclic rings can be cycloalkyls, cycloalkenyls, aryls, heteroaryls, and/or heterocyclyls. Heterocyclyl groups include, for example, piperidine, piperazine, pyrrolidine, tetrahydropyran, tetrahydrofuran, morpholine, lactones, lactams, and the like.

The term "heterocyclylalkyl" or "heterocycloalkyl", as used herein, refers to an alkyl group substituted with a heterocycle group.

The term "hydrocarbyl", as used herein, refers to a group that is bonded through a carbon atom that does not have a =O or =S substituent, and typically has at least one carbon-hydrogen bond and a primarily carbon backbone, but may optionally include heteroatoms. Thus, groups like methyl, ethoxyethyl, 2-pyridyl, and trifluoromethyl are considered to be hydrocarbyl for the purposes of this application, but substituents such as acetyl (which has a =O substituent on the linking carbon) and ethoxy (which is linked through oxygen, not carbon) are not. Hydrocarbyl groups include, but are not limited to aryl, heteroaryl, carbocycle, heterocyclyl, alkyl, alkenyl, alkynyl, and combinations thereof.

The term "hydroxyalkyl", as used herein, refers to an alkyl group substituted with a hydroxy group.

The term "lower" when used in conjunction with a chemical moiety, such as, acyl, acyloxy, alkyl, alkenyl, alkynyl, or alkoxy is meant to include groups where there are ten or fewer non-hydrogen atoms in the substituent, preferably six or fewer. A "lower alkyl", for example, refers to an alkyl group that contains ten or fewer carbon atoms, preferably six or fewer. In certain embodiments, acyl, acyloxy, alkyl, alkenyl, alkynyl, or alkoxy substituents defined herein are respectively lower acyl, lower acyloxy, lower alkyl, lower alkenyl, lower alkynyl, or lower alkoxy, whether they appear alone or in combination with other substituents, such as in the recitations hydroxyalkyl and aralkyl (in which case, for example, the atoms within the aryl group are not counted when counting the carbon atoms in the alkyl substituent).

The terms "polycyclyl", "polycycle", and "polycyclic" refer to two or more rings (e.g., cycloalkyls, cycloalkenyls, aryls, heteroaryls, and/or heterocyclyls) in which two or more atoms are common to two adjoining rings, e.g., the rings are "fused rings". Each of the rings of the polycycle can be substituted or unsubstituted. In certain embodiments, each ring of the polycycle contains from 3 to 10 atoms in the ring, preferably from 5 to 7.

The term "silyl" refers to a silicon moiety with three hydrocarbyl moieties attached thereto.

The term "substituted" refers to moieties having substituents replacing a hydrogen on one or more carbons of the backbone. It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, etc. As used herein, the term "substituted" is contemplated to include all permissible substituents of organic compounds. In a broad aspect, the permissible substituents include acyclic and cyclic, branched and unbranched, carbocyclic and heterocyclic, aromatic and non-aromatic substituents of organic compounds. The permissible substituents can be one or more and the same or different for appropriate organic compounds. For purposes of this invention, the heteroatoms such as nitrogen may have hydrogen substituents and/or any permissible substituents of organic compounds described herein which satisfy the valences of the heteroatoms. Substituents can include any substituents described herein, for example, a halogen, a hydroxyl, a carbonyl (such as a carboxyl, an alkoxycarbonyl, a formyl, or an acyl), a thiocarbonyl (such as a thioester, a thioacetate, or a thioformate), an alkoxy, a phosphoryl, a phosphate, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or an aromatic or heteroaromatic moiety. In preferred embodiments, the substituents on substituted alkyls are selected from C₁₋₆ alkyl, C₃₋₆ cycloalkyl, halogen, carbonyl, cyano, or hydroxyl. In more preferred embodiments, the substituents on substituted alkyls are selected from fluoro, carbonyl, cyano, or hydroxyl. It will be understood by those skilled in the art that substituents can themselves be substituted, if appropriate. Unless specifically stated as "unsubstituted," references to chemical moieties herein are understood to include substituted variants. For example, reference to an "aryl" group or moiety implicitly includes both substituted and unsubstituted variants.

The term "sulfate" is art-recognized and refers to the group -OSO₃H, or a pharmaceutically acceptable salt thereof.

The term "sulfonamide" is art-recognized and refers to the group represented by the general formulae 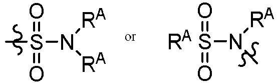 wherein each R^{A} independently represents hydrogen or hydrocarbyl, such as alkyl, or both R^{A} taken together with the intervening atom(s) complete a heterocycle having from 4 to 8 atoms in the ring structure.

The term "sulfoxide" is art-recognized and refers to the group -S(O)-R^{A}, wherein R^{A} represents a hydrocarbyl.

The term "sulfonate" is art-recognized and refers to the group SO₃H, or a pharmaceutically acceptable salt thereof.

The term "sulfone" is art-recognized and refers to the group -S(O)₂-R^{A}, wherein R^{A} represents a hydrocarbyl.

The term "thioalkyl", as used herein, refers to an alkyl group substituted with a thiol group.

The term "thioester", as used herein, refers to a group -C(O)SR^{A} or -SC(O)R^{A} wherein R^{A} represents a hydrocarbyl.

The term "thioether", as used herein, is equivalent to an ether, wherein the oxygen is replaced with a sulfur.

The term "urea" is art-recognized and may be represented by the general formula 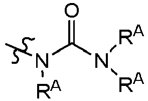 wherein each R^{A} independently represents hydrogen or a hydrocarbyl, such as alkyl, or any occurrence of R^{A} taken together with another and the intervening atom(s) complete a heterocycle having from 4 to 8 atoms in the ring structure.

"Protecting group" refers to a group of atoms that, when attached to a reactive functional group in a molecule, mask, reduce or prevent the reactivity of the functional group. Typically, a protecting group may be selectively removed as desired during the course of a synthesis. Examples of protecting groups can be found in Greene and Wuts, Protective Groups in Organic Chemistry, 3rd Ed., 1999, John Wiley & Sons, NY and Harrison et al., Compendium of Synthetic Organic Methods, Vols. 1-8, 1971-1996, John Wiley & Sons, NY. Representative nitrogen protecting groups include, but are not limited to, formyl, acetyl, trifluoroacetyl, benzyl, benzyloxycarbonyl ("CBZ"), tert-butoxycarbonyl ("Boc"), trimethylsilyl ("TMS"), 2-trimethylsilyl-ethanesulfonyl ("TES"), trityl and substituted trityl groups, allyloxycarbonyl, 9-fluorenylmethyloxycarbonyl ("FMOC"), nitro-veratryloxycarbonyl ("NVOC") and the like. Representative hydroxyl protecting groups include, but are not limited to, those where the hydroxyl group is either acylated (esterified) or alkylated such as benzyl and trityl ethers, as well as alkyl ethers, tetrahydropyranyl ethers, trialkylsilyl ethers (e.g., TMS or TIPS groups), glycol ethers, such as ethylene glycol and propylene glycol derivatives and allyl ethers.

As used herein, a therapeutic that "prevents" or "reduces the risk of developing" a disease, disorder, or condition refers to a compound that, in a statistical sample, reduces the occurrence of the disease, disorder, or condition in the treated sample relative to an untreated control sample, or delays the onset or reduces the severity of one or more symptoms of the disorder or condition relative to the untreated control sample.

The term "treating" includes prophylactic and/or therapeutic treatments. The term "prophylactic or therapeutic" treatment is art-recognized and includes administration to the host of one or more of the subject compositions. If it is administered prior to clinical manifestation of the unwanted condition (e.g., disease or other unwanted state of the host animal) then the treatment is prophylactic (i.e., it protects the host against developing the unwanted condition), whereas if it is administered after manifestation of the unwanted condition, the treatment is therapeutic, (i.e., it is intended to diminish, ameliorate, or stabilize the existing unwanted condition or side effects thereof).

The phrases "conjoint administration" and "administered conjointly" refer to any form of administration of two or more different therapeutic compounds such that the second compound is administered while the previously administered therapeutic compound is still effective in the body (*e.g.,* the two compounds are simultaneously effective in the patient, which may include synergistic effects of the two compounds). For example, the different therapeutic compounds can be administered either in the same formulation or in a separate formulation, either concomitantly or sequentially. In certain embodiments, the different therapeutic compounds can be administered within one hour, 12 hours, 24 hours, 36 hours, 48 hours, 72 hours, or a week of one another. Thus, an individual who receives such treatment can benefit from a combined effect of different therapeutic compounds.

The term "prodrug" is intended to encompass compounds which, under physiologic conditions, are converted into the therapeutically active agents of the present invention. A common method for making a prodrug is to include one or more selected moieties which are hydrolyzed under physiologic conditions to reveal the desired molecule. In other embodiments, the prodrug is converted by an enzymatic activity of the host animal. For example, esters or carbonates (e.g., esters or carbonates of alcohols or carboxylic acids) are preferred prodrugs of the present invention. In certain embodiments, some or all of the compounds of the invention in a formulation represented above can be replaced with the corresponding suitable prodrug, e.g., wherein a hydroxyl in the parent compound is presented as an ester or a carbonate or carboxylic acid present in the parent compound is presented as an ester.

As used herein, "small molecules" refers to small organic or inorganic molecules of molecular weight below about 3,000 Daltons. In general, small molecules useful for the invention have a molecular weight of less than 3,000 Daltons (Da). The small molecules can be, e.g., from at least about 100 Da to about 3,000 Da (e.g., between about 100 to about 3,000 Da, about 100 to about 2500 Da, about 100 to about 2,000 Da, about 100 to about 1,750 Da, about 100 to about 1,500 Da, about 100 to about 1,250 Da, about 100 to about 1,000 Da, about 100 to about 750 Da, about 100 to about 500 Da, about 200 to about 1500, about 500 to about 1000, about 300 to about 1000 Da, or about 100 to about 250 Da).

In some embodiments, a "small molecule" refers to an organic, inorganic, or organometallic compound typically having a molecular weight of less than about 1000. In some embodiments, a small molecule is an organic compound, with a size on the order of 1 nm. In some embodiments, small molecule drugs of the invention encompass oligopeptides and other biomolecules having a molecular weight of less than about 1000.

An "effective amount" is an amount sufficient to effect beneficial or desired results. For example, a therapeutic amount is one that achieves the desired therapeutic effect. This amount can be the same or different from a prophylactically effective amount, which is an amount necessary to prevent onset of disease or disease symptoms. An effective amount can be administered in one or more administrations, applications or dosages. A therapeutically effective amount of a composition depends on the composition selected. The compositions can be administered from one or more times per day to one or more times per week, including once every other day. The skilled artisan will appreciate that certain factors may influence the dosage and timing required to treat effectively a subject, including but not limited to the severity of the disease or disorder, previous treatments, the general health and/or age of the subject, and other diseases present. Moreover, treatment of a subject with a therapeutically effective amount of the compositions described herein can include a single treatment or a series of treatments.

### Compounds of the Invention

One aspect of the invention provides methods of selecting and treating a human subject suffering from a kidney disease comprising the steps of:
a. selecting the subject if a urinary level of one or more biomarkers selected from Rac1, Rac1-GTP, phospho-LIM kinase **1,** and phospho-cofilin in the subject is above a pre-determined threshold; and
b. administering to the selected subject a pharmaceutical composition comprising a TRPC5 inhibitor or a calcineurin inhibitor; and a pharmaceutically acceptable carrier.

In one aspect, the invention relates to a method of treating a human subject suffering from a kidney disease comprising the step of
administering to the subject a pharmaceutical composition comprising
   a TRPC5 inhibitor or a calcineurin inhibitor, and
   a pharmaceutically acceptable carrier;
only if the subject is determined to have a pre-treatment urinary level of one or more biomarkers selected from Rac1, Rac1-GTP, phospho-LIM kinase **1,** and phospho-cofilin that is above a pre-determined threshold.

In some embodiments, the TRPC5 inhibitor is a small molecule inhibitor of TRPCS. In some embodiments, the TRPC5 inhibitor is:
a. a compound of Formula (I) or Formula (II): 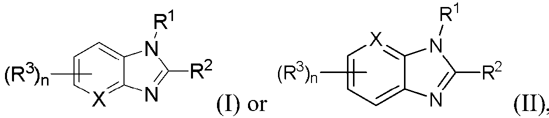 or a pharmaceutically acceptable salt of either of the foregoing, wherein:
   X is CH, C(R³), or N;
   R¹ is selected from the group consisting of H; alkyl; cycloalkyl; heterocycloalkyl; alkenyl; aryl; heteroaryl; alkylene-aryl; alkylene-heteroaryl; -CH₂(O)N(R)-heteroaryl; -CH₂(O)N(R)-alkyl; alkylene-N(alkyl)₂; heterocycloalkyl; alkylene-O-alkyl; alkylene-O-aryl; alkylene-N(R)- C(O)-aryl; alkylene-N(R)-C(O)-alkyl; alkylene-C(O)-N(R)-alkyl; alkylene-C(O)-N(R)-aryl; alkylene-C(O)-cycloalkyl; and alkylene-C(O)-N(R)-heteroaryl;
   R² is selected from the group consisting of H; NH₂, alkyl; cycloalkyl; aryl; heteroaryl; alkylene-aryl, alkylene-N(alkyl)₂; alkylene-heterocycloalkyl; alkylene-cycloalkyl; -N(R)-alkyl; -N(R)-aryl; -N(R)-alkylene-aryl; -N(R)-cycloalkyl; -N(R)-heterocycloalkyl; -O-aryl; alkylene-O-aryl; heterocycloalkyl; -N=C(R)-aryl; -N(R)-alkylene-heteroaryl; -N(R)-alkylene-OH; -S-alkylene-C(O)N(R)-aryl; -S-alkylene-C(O)N(R)-heteroaryl; alkylene-C(O)-heterocycloalkyl; alkylene-N(R)-alkyl; alkylene-N(R)-aryl; and -S-alkyl;
   R³ is independently selected from alkyl, halogen, -CN, -OMe, -OH, -NO₂, -NH₂, -N(Me)₂, -CF₃, -OCF₃, -CHF₂, -OCHF₂, and -O-alkylene-OH;
   R is H, or Me; and
      n is 0, 1, 2, 3, or 4;
b. a compound of Formula (III), (IV), or (V): 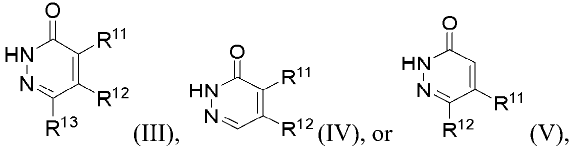 or a tautomer or a pharmaceutically acceptable salt of any of the foregoing, wherein:
   R¹¹ and R¹³ are independently selected from the group consisting of H, alkyl, alkenyl, alkynyl, aryl, heterocyclyl, heteroaryl, halogen, -OH, -CN, -cycloalkyl, -O-alkyl, -O-cycloalkyl, -O-aryl, -aryl-O-aryl -CF₃, -C(H)F₂, alkylene-CF₃, alkylene-C(H)F₂, -SO₂-alkyl, and -O-alkylene-O-alkyl, -heterocyclyl-L-R⁴, and -heteroaryl-L-R⁴;
   R¹² is -heterocyclyl-L-R¹⁴;
   R¹⁴ is absent or selected from the group consisting of alkyl, cycloalkyl, aryl, alkylene-aryl, alkylene-heteroaryl, heteroaryl, heterocyclyl, -C(O)N(R¹⁵)₂, and CF₃;
   R¹⁵ is independently H or alkyl;
   R¹⁶ is selected from the group consisting of alkyl, cycloalkyl, aryl, heterocyclyl, heteroaryl, alkylene-aryl, -C(O)N(R¹⁵)₂, and CF₃;
   L is absent or selected from the group consisting of methylene, -C(O)-, -SO₂-, -CH₂N(Me)-, -N(R¹⁵)(R¹⁶)-, -C(R¹⁵)(R¹⁶)-, and -O-R¹⁶; and
   one and only one of R¹¹, R¹², and R¹³ is -heterocyclyl-L-R¹⁴ or -heteroaryl-L-R¹⁴;
c. a compound of Formula (VI) or (VII): 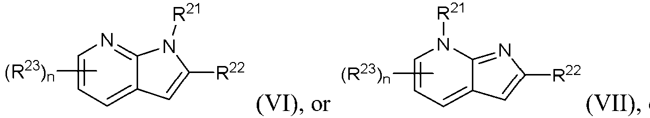 or a pharmaceutically acceptable salt thereof, wherein:
   R²¹ is selected from the group consisting of alkyl; cycloalkyl; heterocycloalkyl; aryl; heteroaryl; alkylene-aryl; alkylene-heteroaryl; alkylene-O-aryl; alkylene-N(alkyl)₂; alkylene-heterocycloalkyl; alkylene-cycloalkyl; -N(alkyl)_{2;} and -C(O)-aryl;
   R²² is selected from the group consisting of alkyl; cycloalkyl; heterocycloalkyl; aryl; heteroaryl; alkylene-N(alkyl)₂; alkylene-heterocycloalkyl; alkylene-cycloalkyl; alkylene-heterocycloalkyl; and alkylene-OR';
   R²³ is independently selected from alkyl, halogen, OMe, OH, N(Me)₂, CF₃, or OCF_{3,} -O- and alkylene-OH;
   R is H, or Me;
   R' is H, methyl, ethyl, or isopropyl; and
   n is 0, 1, 2, 3, or 4; or
d. a compound of Formula (VIII) or (IX): 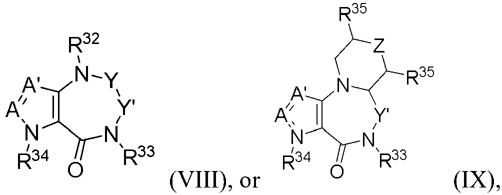 or a pharmaceutically acceptable salt of either of the foregoing, wherein:
   A and A' are independently selected from CR^{a} and N;
   R^{a} is L-R³¹;
   L is absent, CH₂, O, SO₂, or NR³²;
   R³¹ is selected from optionally substituted alkyl, optionally substituted aryl, and optionally substituted heteroaryl;
   each R³² is independently **H,** or alkyl;
   R³³ is selected from optionally substituted alkyl, optionally substituted alkylene-OR³², optionally substituted cycloalkylene-OR³², optionally substituted alkylene-N(R³⁷)₂, optionally substituted cycloalkylene-N(R³⁷)₂, optionally substituted alkylene-C(O)N(R³²)₂, optionally substituted cycloalkylene-C(O)N(R³²)₂, optionally substituted alkylene-S(O)₂N(R³²)₂, and optionally substituted cycloalkylene-S(O)₂N(R³²)₂;
   R³⁴ is selected from alkyl, optionally substituted alkylene-aryl, and optionally substituted alkylene-heteroaryl;
   each R³⁵ is independently selected from **H,** N(R³²)₂, OR³²;
   each R³⁷ is independently selected from **H,** alkyl, (alkyl)C(O)-, (aryl)C(O)-, (alkyl)S(O)₂-, and (aryl)S(O)₂-;
   Y is -C(O)-, CH₂, CHR³⁶, C(R³⁶)₂;
   each R³⁶ is independently selected from **H,** alkyl, and optionally substituted alkylene-OH;
   Y' is -C(O)-, CH₂, CHR^{33'}, C(R^{33'})₂, or Y' is taken together with R³³ to form a 5- or 6-membered ring;
   each R^{33'} is independently selected from optionally substituted alkyl, optionally substituted alkylene-OR³², optionally substituted cycloalkylene-OR³², optionally substituted alkylene-N(R³⁷)₂, optionally substituted cycloalkylene-N(R³⁷)₂, optionally substituted alkylene-C(O)N(R³²)₂, optionally substituted cycloalkylene-C(O)N(R³²)₂, optionally substituted alkyleneS(O)₂N(R³²)₂, and optionally substituted cycloalkylene-S(O)₂N(R³²)₂; and
   Z is absent, CH₂, CHR³⁵, O, -NR³²-, or -SO₂-;
   provided that Y and Y' are not both -C(O)-.

In some embodiments, the TRPC5 inhibitor a compound of structural formula X: 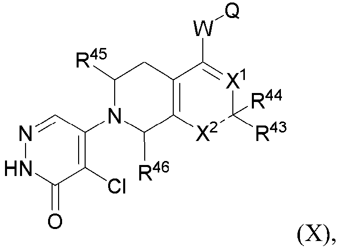 or a pharmaceutically acceptable salt thereof, wherein:
"---" is a single bond or a double bond;
X¹ is CH or N;
when "---" is a double bond, X² is CH or N;
when "---" is a single bond, X² is N(CH₃),
when X¹ is CH, X² is N or N(CH₃);
W is -O-, -N(CH₃)-, -N(CH₂CH₂OH)-, cyclopropan-1,1-diyl, or -CH(CH₃)-;
Q is 2-trifluoromethyl-4-fluorophenyl, 2-difluoromethyl-4-fluorophenyl, 2-trifluoromethylphenyl, 2-methyl-4-fluorophenyl, 2-chloro-4-fluorophenyl, 2-chlorophenyl, 1-(benzyl)-4-methylpiperidin-3-yl, 4-trifluoromethylpyridin-3-yl, 2-trifluoromethyl-6-fluorophenyl, 2-trifluoromethyl-3-cyanophenyl, 2-ethyl-3-fluorophenyl, 2-chloro-3-cyanophenyl, 2-trifluoromethyl-5-fluorophenyl, or 2-difluoromethylphenyl;
R⁴³ is hydrogen, -CH₂OH, -CH(OH)-CH₂OH, -NH₂, -CH(OH)CH₃, -OCH₃, or -NH-(CH₂)₂OH; and when "---" is a double bond, R⁴⁴ is absent;
and when "---" is a single bond, R⁴³ and R⁴⁴ are taken together to form =O; and
each of R⁴⁵ and R⁴⁶ is independently hydrogen or -CH₃.

In some embodiments, the TRPC5 inhibitor is a compound of Formula XI: 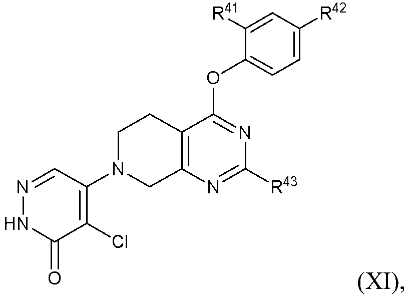 or a pharmaceutically acceptable salt thereof; wherein:
R⁴¹ is chloro, -CF₃, -CHF₂, or -CH₃;
R⁴² is hydrogen or fluoro; and
R⁴³ is hydrogen, -NH₂, -CH₂OH, or CH(OH)-CH₂OH.

Compounds of Formulas I-XI can be synthesized using methods known to those of skill in the art, e.g., methods disclosed in WO 2019/055966, the entire contents of which are hereby incorporated herein by reference.

In some embodiments, the TRPC5 inhibitor is a compound of Formula (H-I): 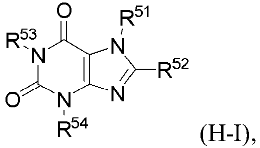 or a pharmaceutically acceptable salt thereof, wherein:
R⁵¹ is C₁-C₆ alkyl, C₂-C₆ alkenyl or C₂-C₆ alkynyl, each of which is optionally substituted with 1-4 R⁵⁵;
R⁵² is C₁-C₆ alkyl, C₁-C₆ heteroalkyl, C₂-C₆ alkenyl, C₂-C₆ alkynyl, C₁-C₆ haloalkyl, halo, C₁-C₆ haloalkoxy, hydroxyl, C₁-C₆ alkoxy, C₃-C₇ cycloalkyloxy, C₆-C₁₀ aryl, C₆-C₁₀ aryloxy, C₇-C₁₆ arylalkoxy, amino, C₁-C₆ akylamino, C₂-C₁₂ dialkylamino, -S-, -S-C₁-C₆ alkyl, - S(O)-,S(O)₂-, heterocycloalkyl, heteroaryl, heteroaryloxy, sulfonamidyl, amido, urea, sulfonylurea, acyl, nitro, cyano,
   wherein each of C₁-C₆ alkyl, C₁-C₆ heteroalkyl, C₂-C₆ alkenyl, C₂-C₆ alkynyl, C₁-C₆ haloalkyl, C₁-C₆ haloalkoxy, hydroxyl, C₁-C₆ alkoxy, C₃-C₇ cycloalkyloxy, C₆-C₁₀ aryl, C₆-C₁₀ aryloxy, C₇-C₁₆ arylalkoxy, amino, C₁-C₆ akylamino, C₂-C₁₂ dialkylamino, - S-, -S-C₁-C₆ alkyl, -S(O)-, -S(O)₂-, heterocycloalkyl, heteroaryl, heteroaryloxy, sulfonamidyl, amido, urea, sulfonylurea, acyl, is optionally substituted with 1-3 R⁵⁶;
R⁵³ is C₁-C₆ alkyl, C₁-C₆ heteroalkyl, C₃-C₇ cycloalkyl, C₂-C₆ alkenyl, C₂-C₆ alkynyl, C₂-C₆ hydroxyalkyl, or C₁-C₆ alkoxy, each of which is optionally substituted with 1-4 R⁵⁷;
R⁵⁴ is C₁-C₆ alkyl, C₁-C₆ heteroalkyl, C₂-C₆ alkenyl or C₂-C₆ alkynyl, each of which is optionally substituted with 1-4 R⁵⁸;
R⁵⁵, R⁵⁶, R⁵⁷, and R⁵⁸ are each independently C₁-C₆ alkyl, C₁-C₆ heteroalkyl, halo, C₁-C₆ haloalkyl, C₁-C₆ haloalkoxy, hydroxyl, C₁-C₆ alkoxy, amino, C₁-C₆ alkylamino, C₂-C₁₂ dialkylamino, cyano, nitro, amido, C₁-C₆ alkylamido, C₂-C₁₂ dialkylamido, -S-, -S(O)₂-, -C(O)O-, -C(O)-, -C(O)O-C₁-C₆ alkyl, C₃-C₇ cycloalkyl, C₆-C₁₀ aryl, heterocycloalkyl, or heteroaryl,
   wherein each of C₁-C₆ alkyl, C₁-C₆ heteroalkyl, C₁-C₆ haloalkyl, C₁-C₆ haloalkoxy, hydroxyl, C₁-C₆ alkoxy, amino, C₁-C₆ alkylamino, C₂-C₁₂ dialkylamino, amido, C₁-C₆ alkylamido, C₂-C₁₂ dialkylamido, -S-, -S(O)₂-, -C(O)O-, -C(O)-, -C(O)O-C₁-C₆ alkyl, C₃-C₇ cycloalkyl, C₆-C₁₀ aryl, heterocycloalkyl, or heteroaryl is optionally substituted with 1-3 R⁵⁹; and
each R⁵⁹ is independently C₁-C₆ alkyl, C₁-C₆ heteroalkyl, C₁-C₆ haloalkyl, C₁-C₆ haloalkoxy, heterocycloalkyl, C₆-C₁₀ aryl, heteroaryl, C₄-C₁₀ cycloalkylalkyl, heterocycloalkyl-C₁-C₆ alkyl, C₇-C₁₆ arylalkyl, heteroaryl-C₁-C₆ alkyl, halo, hydroxyl, C₁-C₆ alkoxy, C₆-C₁₀ aryloxy, C₇-C₁₆ arylalkoxy, C₂-C₈ alkoxyalkoxyl, amino, C₁-C₆ akylamino, C₂-C₁₂ dialkylamino, C₁-C₆ akyl-amino-C₁-C₆ akyl, C1-C6 akyl-amino-C₂-C₁₂ dialkyl, -S-, -S-C₁-C₆ alkyl, -S(O)₂-C₁-C₆ alkyl, sulfonamidyl, amido, urea, sulfonylurea, acyl, -C(O)-C₆-C₁₀ aryl, -NHC(O)-C₆-C₁₀ aryl, -C(O)NH-C₆-C₁₀ aryl, -C(O)OH, -C(O)O-C₁-C₆ alkyl, -C(O)-C₁-C₆ alkyl acyl, nitro, or cyano.

In some embodiments, the TRPC5 inhibitor is a compound of formula H-Ia: 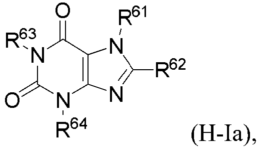 or a pharmaceutically acceptable salt thereof, wherein:
R⁶¹ is C₁-C₆ alkyl, C₂-C₆ alkenyl or C₂-C₆ alkynyl, each of which is optionally substituted with 1-4 R⁶⁵;
R⁶² is C₁-C₆ alkyl, C₁-C₆ heteroalkyl, C₂-C₆ alkenyl, C₂-C₆ alkynyl, C₁-C₆ haloalkyl, halo, C₁-C₆ haloalkoxy, hydroxyl, C₁-C₆ alkoxy, C₃-C₇ cycloalkyloxy, C₆-C₁₀ aryl, C₆-C₁₀ aryloxy, C₇-C₁₆ arylalkoxy, amino, C₁-C₆ akylamino, C₂-C₁₂ dialkylamino, -S-, -S-C₁-C₆ alkyl, -S(O)-,S(O)₂-, heterocycloalkyl, heteroaryl, heteroaryloxy, sulfonamidyl, amido, urea, sulfonylurea, acyl, nitro, cyano,
   wherein each of C₁-C₆ alkyl, C₁-C₆ heteroalkyl, C₂-C₆ alkenyl, C₂-C₆ alkynyl, C₁-C₆ haloalkyl, C₁-C₆ haloalkoxy, hydroxyl, C₁-C₆ alkoxy, C₃-C₇ cycloalkyloxy, C₆-C₁₀ aryl, C₆-C₁₀ aryloxy, C₇-C₁₆ arylalkoxy, amino, C₁-C₆ akylamino, C₂-C₁₂ dialkylamino, -S-, -S-C₁-C₆ alkyl, -S(O)-, -S(O)₂-, heterocycloalkyl, heteroaryl, heteroaryloxy, sulfonamidyl, amido, urea, sulfonylurea, acyl, is optionally substituted with 1-3 R⁶⁶;
R⁶³ is C₂-C₆ hydroxyalkyl or C₁-C₆ heteroalkyl;
R⁶⁴ is C₁-C₆ alkyl, C₁-C₆ heteroalkyl, C₂-C₆ alkenyl or C₂-C₆ alkynyl, each of which is optionally substituted with 1-4 R⁶⁸;
R⁶⁵, R⁶⁶, and R⁶⁸ are each independently C₁-C₆ alkyl, C₁-C₆ heteroalkyl, halo, C₁-C₆ haloalkyl, C₁-C₆ haloalkoxy, hydroxyl, C₁-C₆ alkoxy, amino, C₁-C₆ alkylamino, C₂-C₁₂ dialkylamino, cyano, nitro, amido, C₁-C₆ alkylamido, C₂-C₁₂ dialkylamido, -S-, -S(O)₂-, -C(O)O-, -C(O)-, -C(O)O-C₁-C₆ alkyl, C₃-C₇ cycloalkyl, C₆-C₁₀ aryl, heterocycloalkyl, or heteroaryl,
   each of C₁-C₆ alkyl, C₁-C₆ heteroalkyl, C₁-C₆ haloalkyl, C₁-C₆ haloalkoxy, hydroxyl, C₁-C₆ alkoxy, amino, C₁-C₆ alkylamino, C₂-C₁₂ dialkylamino, amido, C₁-C₆ alkylamido, C₂-C₁₂ dialkylamido, -S-, -S(O)₂-, -C(O)O-, -C(O)-, -C(O)O-C₁-C₆ alkyl, C₃-C₇ cycloalkyl, C₆-C₁₀ aryl, heterocycloalkyl, or heteroaryl is optionally substituted with 1-3 R⁶⁹; and
each R⁶⁹ is independently C₁-C₆ alkyl, C₁-C₆ heteroalkyl, C₁-C₆ haloalkyl, C₁-C₆ haloalkoxy, heterocycloalkyl, C₆-C₁₀ aryl, heteroaryl, C₄-C₁₀ cycloalkylalkyl, heterocycloalkyl-C₁-C₆ alkyl, C₇-C₁₆ arylalkyl, heteroaryl-C₁-C₆ alkyl, halo, hydroxyl, C₁-C₆ alkoxy, C₆-C₁₀ aryloxy, C₇-C₁₆ arylalkoxy, C₂-C₈ alkoxyalkoxyl, amino, C₁-C₆ akylamino, C₂-C₁₂ dialkylamino, C₁-C₆ akyl-amino-C₁-C₆ akyl, C₁-C₆ akyl-amino-C₂-C₁₂ dialkyl, -S-, -S-C₁-C₆ alkyl, -S(O)₂-C₁-C₆ alkyl, sulfonarnidyl, amido, urea, sulfonylurea, acyl, -C(O)-C₆-C₁₀ aryl, -NHC(O)-C₆-C₁₀ aryl, -C(O)NH-C₆-C₁₀ aryl, -C(O)OH, -C(O)O-C₁-C₆ alkyl, -C(O)-C₁-C₆ alkyl acyl, nitro, or cyano.

In some embodiments, the TRPC5 inhibitor is a compound of Formula H-II: 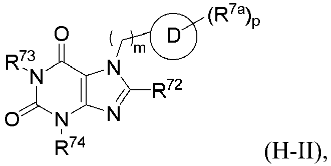 or a pharmaceutically acceptable salt thereof, wherein:
Ring D is phenyl, pyridyl, thiazolyl, pyrimidinyl, or oxazolyl;
R⁷² is C₁-C₆ alkyl, C₁-C₆ heteroalkyl, C₂-C₆ alkenyl, C₂-C₆ alkynyl, C₁-C₆ haloalkyl, halo, C₁-C₆ haloalkoxy, hydroxyl, C₁-C₆ alkoxy, C₃-C₇ cycloalkyloxy, C₆-C₁₀ aryl, C₆-C₁₀ aryloxy, C₇-C₁₆ arylalkoxy, amino, C₁-C₆ akylamino, C₂-C₁₂ dialkylamino, -S-, -S-C₁-C₆ alkyl, -S(O)-,S(O)₂-, heterocycloalkyl, heteroaryl, heteroaryloxy, sulfonamidyl, amido, urea, sulfonylurea, acyl, nitro, cyano,
   wherein each of C₁-C₆ alkyl, C₁-C₆ heteroalkyl, C₂-C₆ alkenyl, C₂-C₆ alkynyl, C₁-C₆ haloalkyl, C₁-C₆ haloalkoxy, hydroxyl, C₁-C₆ alkoxy, C₃-C₇ cycloalkyloxy, C₆-C₁₀ aryl, C₆-C₁₀ aryloxy, C₇-C₁₆ arylalkoxy, amino, C₁-C₆ akylamino, C₂-C₁₂ dialkylamino, -S-, -S-C₁-C₆ alkyl, -S(O)-, -S(O)₂-, heterocycloalkyl, heteroaryl, heteroaryloxy, sulfonamidyl, amido, urea, sulfonylurea, acyl, is optionally substituted with 1-3 R⁷⁶;
R⁷³ is C₁-C₆ alkyl, C₁-C₆ heteroalkyl, C₂-C₆ alkenyl, C₂-C₆ alkynyl, C₂-C₆ hydroxyalkyl, or C₁-C₆ alkoxy, each of which is optionally substituted with 1-4 R⁷⁷;
R⁷⁴ is C₁-C₆ alkyl, C₁-C₆ heteroalkyl, C₂-C₆ alkenyl or C₂-C₆ alkynyl, each of which is optionally substituted with 1-4 R⁷⁸;
R⁷⁶, R⁷⁷, and R⁷⁸ are each independently C₁-C₆ alkyl, C₁-C₆ heteroalkyl, halo, C₁-C₆ haloalkyl, C₁-C₆ haloalkoxy, hydroxyl, C₁-C₆ alkoxy, amino, C₁-C₆ alkylamino, C₂-C₁₂ dialkylamino, cyano, nitro, amido, C₁-C₆ alkylamido, C₂-C₁₂ dialkylamido, -S-, -S(O)₂-, -C(O)O-, -C(O)-, -C(O)O-C₁-C₆ alkyl, C₃-C₇ cycloalkyl, C₆-C₁₀ aryl, heterocycloalkyl, or heteroaryl,
   wherein each of C₁-C₆ alkyl, C₁-C₆ heteroalkyl, C₁-C₆ haloalkyl, C₁-C₆ haloalkoxy, hydroxyl, C₁-C₆ alkoxy, amino, C₁-C₆ alkylamino, C₂-C₁₂ dialkylamino, amido, C₁-C₆ alkylamido, C₂-C₁₂ dialkylamido, -S-, -S(O)₂-, -C(O)O-, -C(O)-, -C(O)O-C₁-C₆ alkyl, C₃-C₇ cycloalkyl, C₆-C₁₀ aryl, heterocycloalkyl, or heteroaryl is optionally substituted with 1-3 R⁷⁹;
each R⁷⁹ is independently C₁-C₆ alkyl, C₁-C₆ heteroalkyl, C₁-C₆ haloalkyl, C₁-C₆ haloalkoxy, heterocycloalkyl, C₆-C₁₀ aryl, heteroaryl, C₄-C₁₀ cycloalkylalkyl, heterocycloalkyl C₁-C₆ alkyl, C₇-C₁₆ arylalkyl, heteroaryl-C₁-C₆ alkyl, halo, hydroxyl, C₁-C₆ alkoxy, C₆-C₁₀ aryloxy, C₇-C₁₆ arylalkoxy, C₂-C₈ alkoxyalkoxyl, amino, C₁-C₆ akylamino, C₂-C₁₂ dialkylamino, C₁-C₆ akyl-amino-C₁-C₆ akyl, C₁-C₆ akyl-amino-C₂-C₁₂ dialkyl, -S-, -S-C₁-C₆ alkyl, -S(O)₂-C₁-C₆ alkyl, sulfonamidyl, amido, urea, sulfonylurea, acyl, -C(O)-C₆-C₁₀ aryl, -NHC(O)-C₆-C₁₀ aryl, -C(O)NH-C₆-C₁₀ aryl, -C(O)OH, -C(O)O-C₁-C₆ alkyl, -C(O)-C₁-C₆ alkyl acyl, nitro, or cyano;
each R⁷⁸ is C₁-C₆ alkyl, C₁-C₆ haloalkyl, halo;
p is 1 or 2; and
m is 1, 2, or 3.

In some embodiments, the TRPC5 inhibitor is a compound of formula H-III: 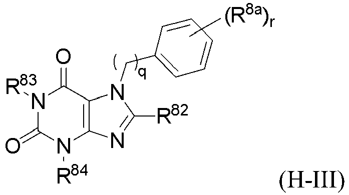 or a pharmaceutically acceptable salt thereof, wherein:
R⁸² is C₁-C₆ alkoxy or C₆-C₁₀ aryloxy substituted with 1-3 R⁸⁶;
R⁸³ is C₁-C₆ heteroalkyl or C₂-C₆ hydroxyalkyl;
R⁸⁴ is C₁-C₆ alkyl;
R⁸⁶ is independently C₁-C₆ alkyl, halo, C₁-C₆ haloalkyl, C₁-C₆ haloalkoxy, or C₁-C₆ alkoxy;
each R^{8a} is C₁-C₆ alkyl, C₁-C₆ haloalkyl, halo;
r is 1 or 2; and
q is 1, 2, or 3.

Compounds of formulas (H-I), (H-Ia), (H-II), and (H-III) can be synthesized using methods known to those of skill in the art, e.g., methods disclosed in International Patent Application Publication No. WO 2014/143799, the entire contents of which are hereby incorporated herein by reference.

In some embodiments, the TRPC5 inhibitor is: 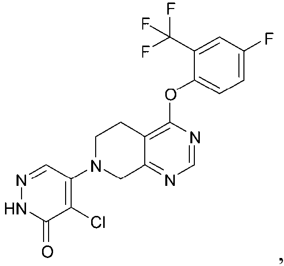 or a pharmaceutically acceptable salt thereof.

In some embodiments, the TRPC5 inhibitor is 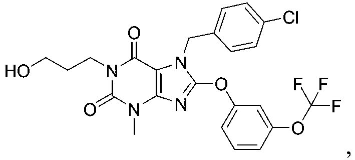 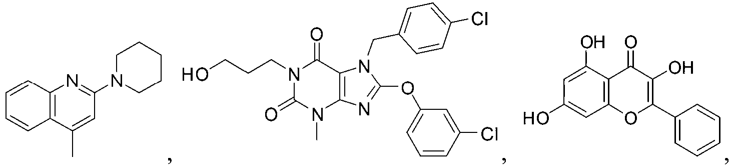 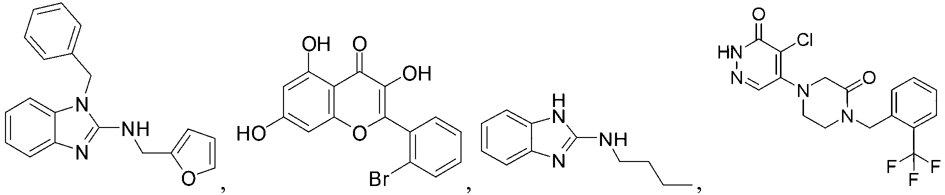 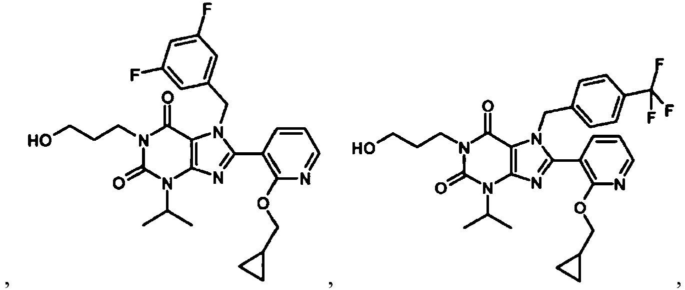 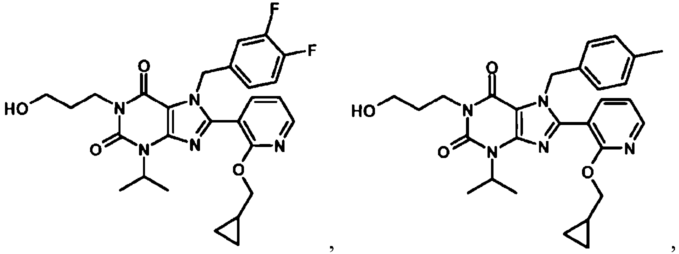 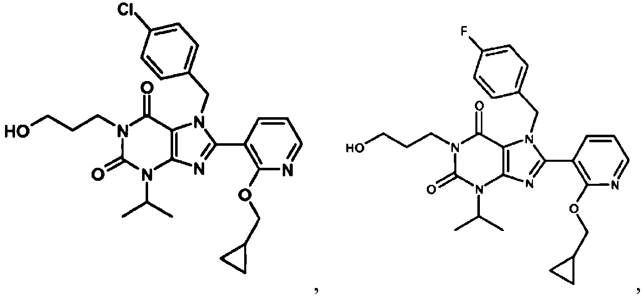 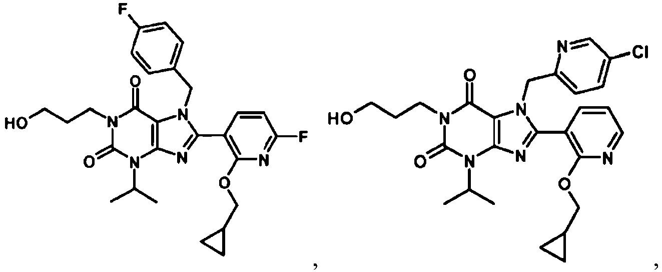 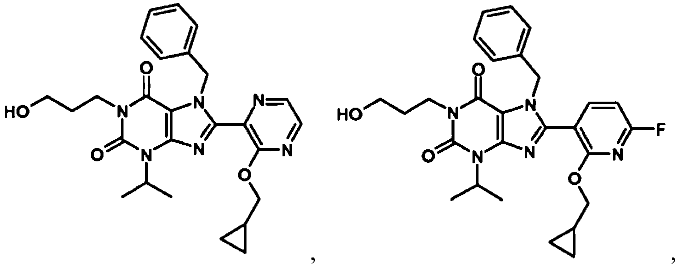 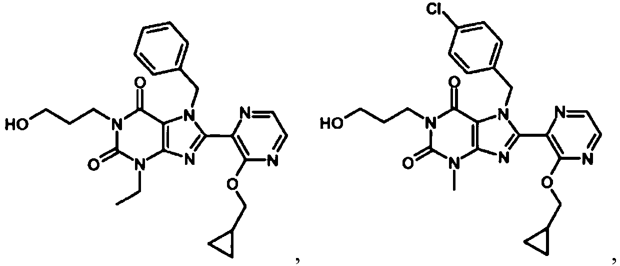 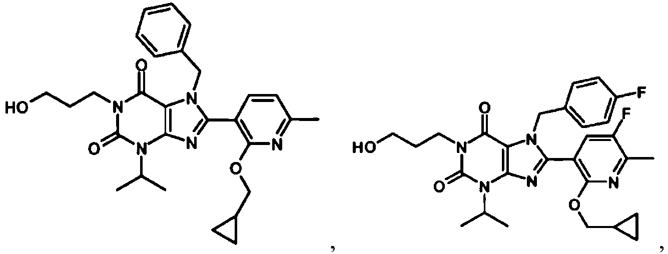 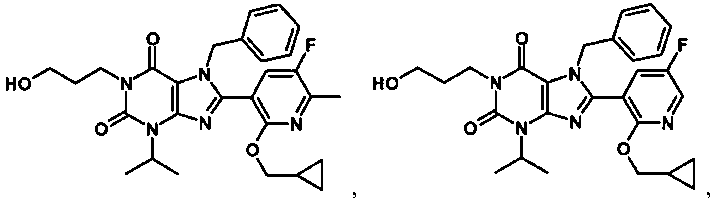 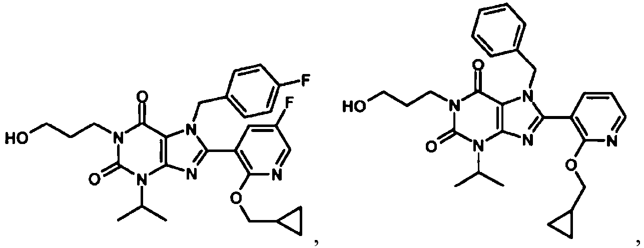 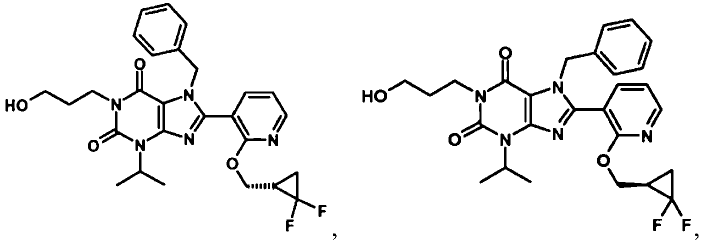 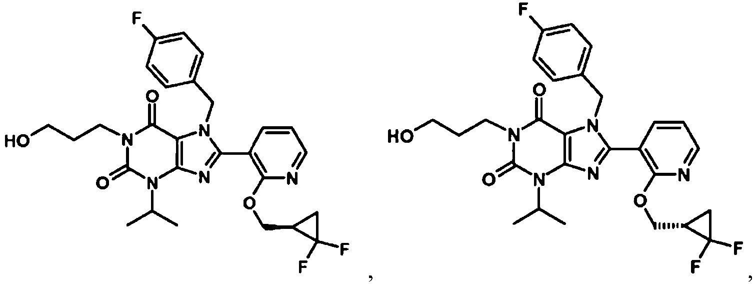 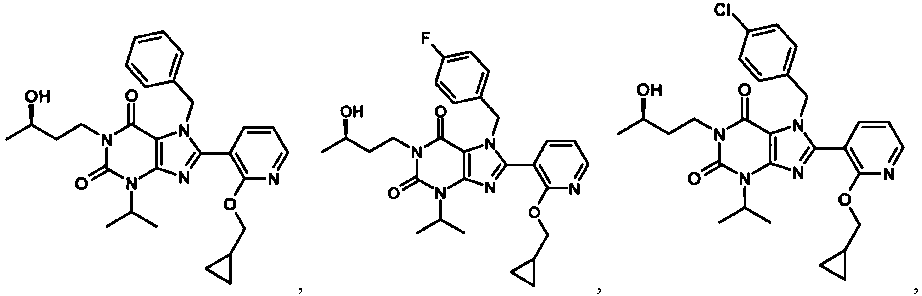 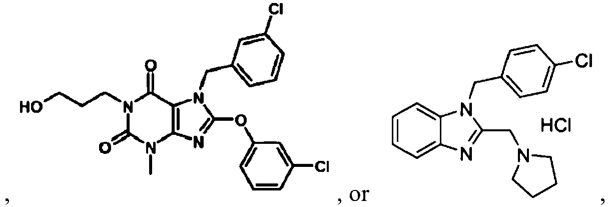 or a pharmaceutically acceptable salt thereof.

These compounds can be synthesized by methods known to those skilled in the art, e.g., methods disclosed in International Patent Application Publication No. WO 2019/011802 (incorporated by reference), in Rubaiy et al., Br. J. Pharmacol. (2019) 176:832-846, and in Miller et al., J. Biol. Chem. (2011) 286(38):33436-33446. 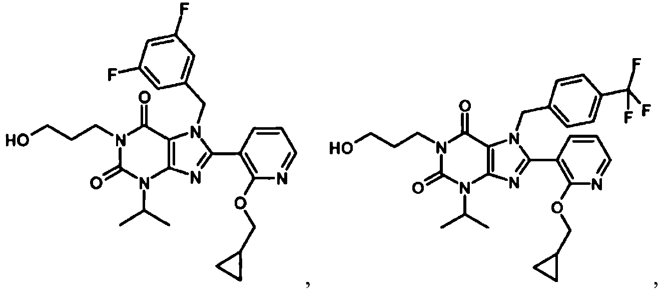 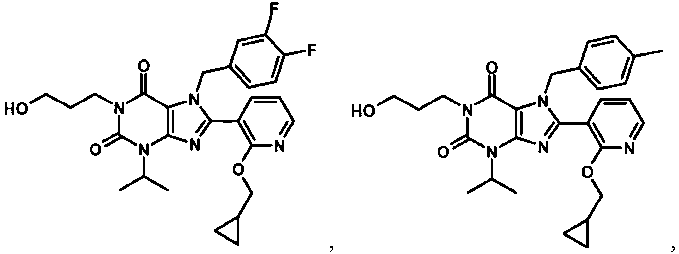 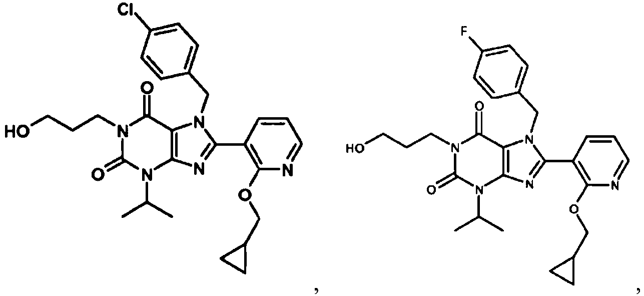 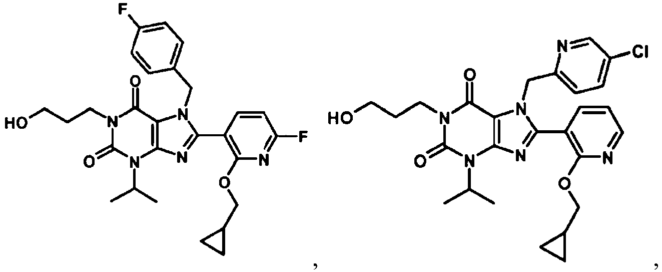 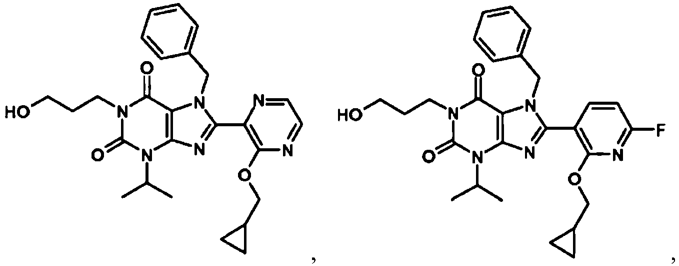 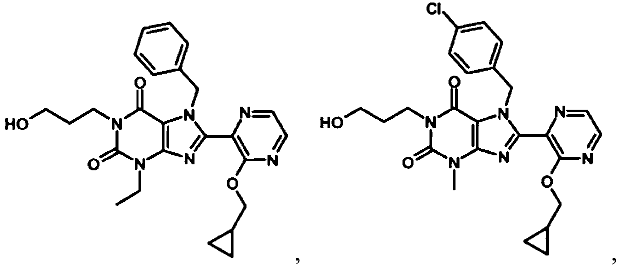 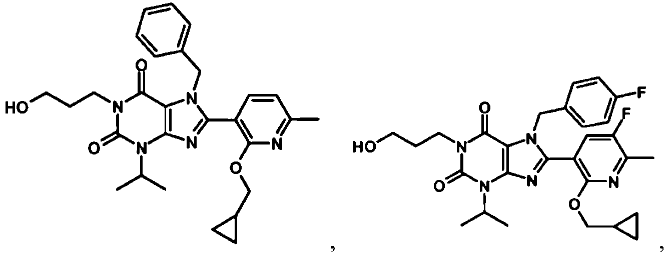 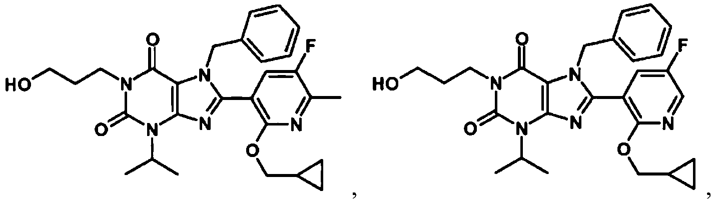 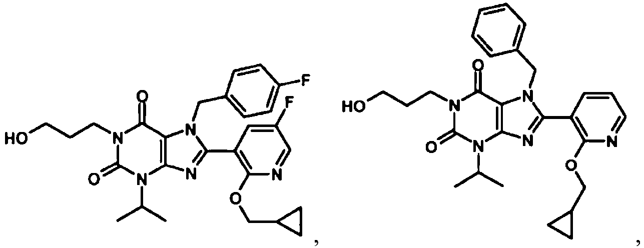 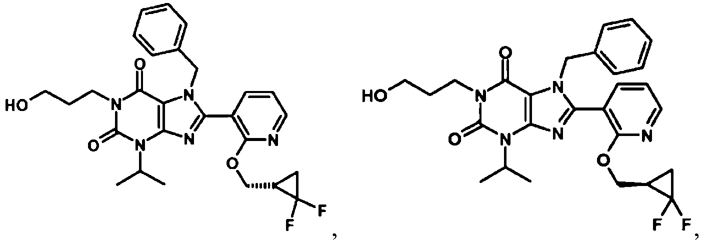 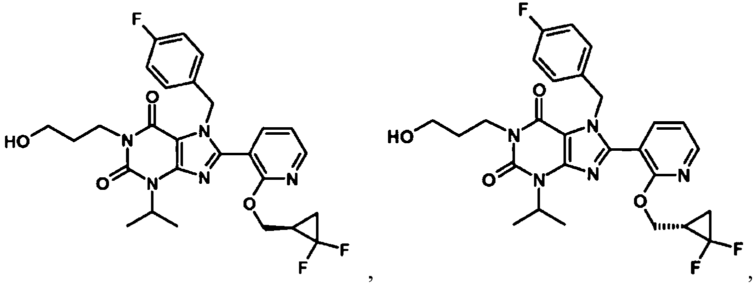 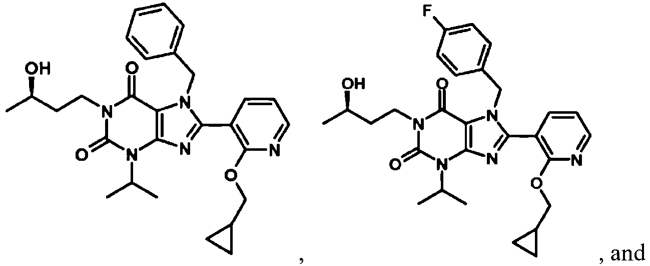 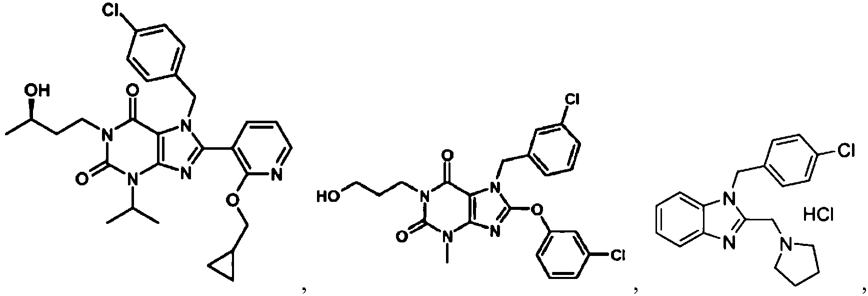 and

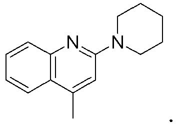

In some embodiments, the TRPC5 inhibitor is 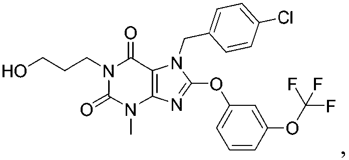 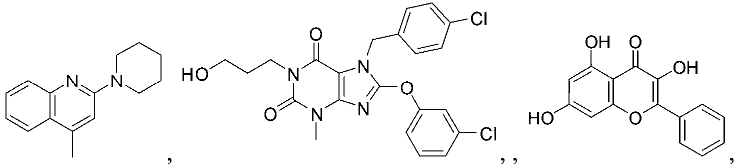 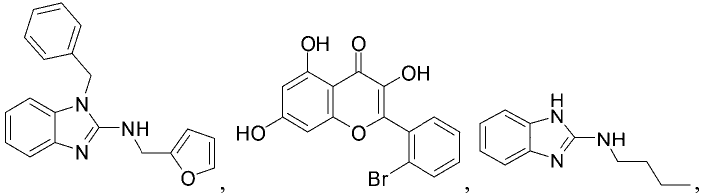 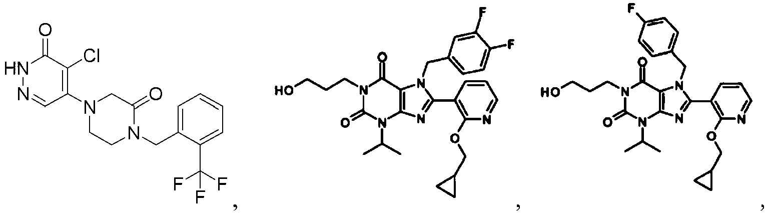 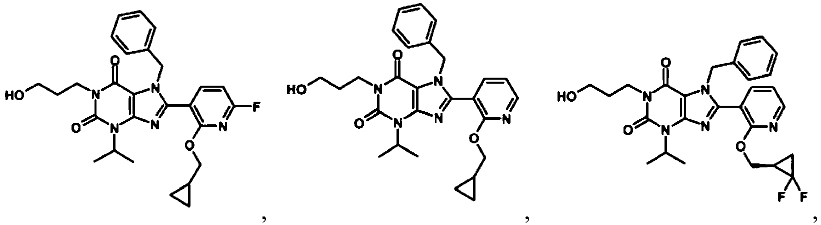 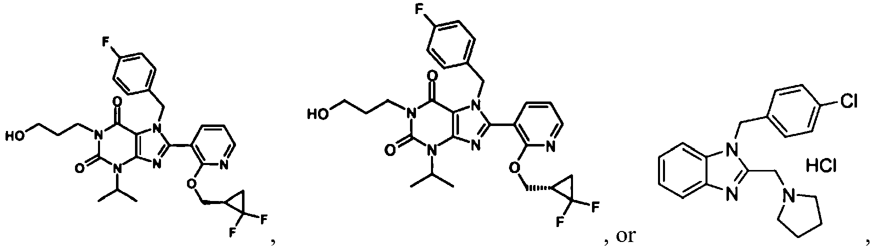 , or a pharmaceutically acceptable salt thereof.

In some embodiments, the calcineurin inhibitor is a small molecule inhibitor of calcineurin. In certain embodiments, the calcineurin inhibitor is cyclosporin A, tacrolimus, or voclosporin, or a pharmaceutically acceptable salt thereof. Cyclosporin A has the following structure: 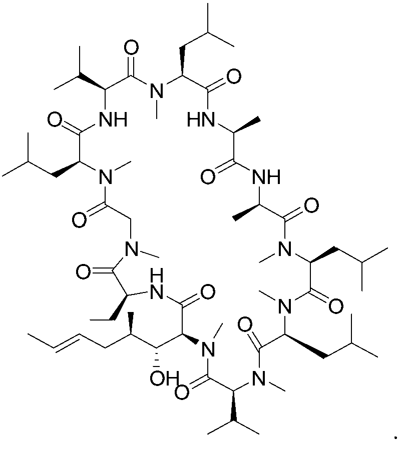 Tacrolimus has the following structure: 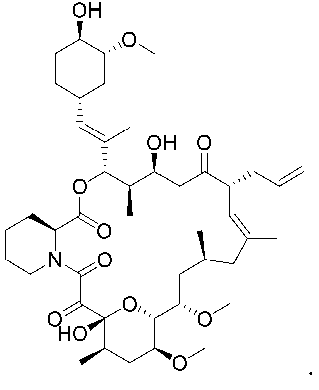 Voclosporin has the following structure:

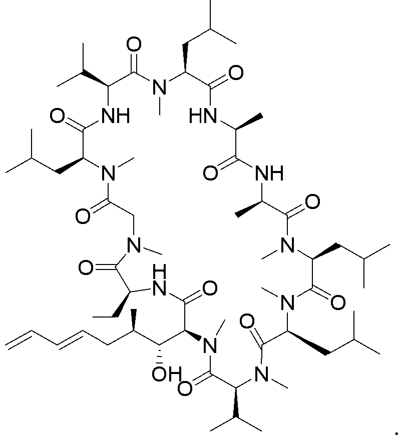

In certain embodiments, the compounds of the invention may be racemic. In certain embodiments, the compounds of the invention may be enriched in one enantiomer. For example, a compound of the invention may have greater than 30% ee, 40% ee, 50% ee, 60% ee, 70% ee, 80% ee, 90% ee, or even 95% or greater ee.

The compounds of the invention have more than one stereocenter. Accordingly, the compounds of the invention may be enriched in one or more diastereomers. For example, a compound of the invention may have greater than 30% de, 40% de, 50% de, 60% de, 70% de, 80% de, 90% de, or even 95% or greater de. In certain embodiments, the compounds of the invention have substantially one isomeric configuration at one or more stereogenic centers, and have multiple isomeric configurations at the remaining stereogenic centers.

In certain embodiments, the enantiomeric excess of the stereocenter is at least 40% ee, 50% ee, 60% ee, 70% ee, 80% ee, 90% ee, 92% ee, 94% ee, 95% ee, 96% ee, 98% ee or greater ee.

As used herein, single bonds drawn without stereochemistry do not indicate the stereochemistry of the compound.

As used herein, hashed or bolded non-wedge bonds indicate relative, but not absolute, stereochemical configuration (e.g., do not distinguish between enantiomers of a given diastereomer).

As used herein, hashed or bolded wedge bonds indicate absolute stereochemical configuration.

In some embodiments, the invention relates to pharmaceutical composition comprising a compound of the invention and a pharmaceutically acceptable carrier. In certain embodiments, a therapeutic preparation or pharmaceutical composition of the compound of the invention may be enriched to provide predominantly one enantiomer of a compound. An enantiomerically enriched mixture may comprise, for example, at least 60 mol percent of one enantiomer, or more preferably at least 75, 90, 95, or even 99 mol percent. In certain embodiments, the compound enriched in one enantiomer is substantially free of the other enantiomer, wherein substantially free means that the substance in question makes up less than 10%, or less than 5%, or less than 4%, or less than 3%, or less than 2%, or less than 1% as compared to the amount of the other enantiomer, e.g., in the composition or compound mixture. For example, if a composition or compound mixture contains 98 grams of a first enantiomer and 2 grams of a second enantiomer, it would be said to contain 98 mol percent of the first enantiomer and only 2% of the second enantiomer.

In certain embodiments, a therapeutic preparation or pharmaceutical composition may be enriched to provide predominantly one diastereomer of the compound of the invention. A diastereomerically enriched mixture may comprise, for example, at least 60 mol percent of one diastereomer, or more preferably at least 75, 90, 95, or even 99 mol percent.

### Pharmaceutical Compositions

The compositions and methods of the present invention may be utilized to treat a subject in need thereof. In certain embodiments, the subject is a mammal such as a human, or a non-human mammal. When administered to subject, such as a human, the composition or the compound is preferably administered as a pharmaceutical composition comprising, for example, a compound of the invention and a pharmaceutically acceptable carrier. Pharmaceutically acceptable carriers are well known in the art and include, for example, aqueous solutions such as water or physiologically buffered saline or other solvents or vehicles such as glycols, glycerol, oils such as olive oil, or injectable organic esters. In preferred embodiments, when such pharmaceutical compositions are for human administration, particularly for invasive routes of administration (i.e., routes, such as injection or implantation, that circumvent transport or diffusion through an epithelial barrier), the aqueous solution is pyrogen-free, or substantially pyrogen-free. The excipients can be chosen, for example, to effect delayed release of an agent or to selectively target one or more cells, tissues or organs. The pharmaceutical composition can be in dosage unit form such as tablet, capsule (including sprinkle capsule and gelatin capsule), granule, lyophile for reconstitution, powder, solution, syrup, suppository, injection or the like. The composition can also be present in a transdermal delivery system, e.g., a skin patch. The composition can also be present in a solution suitable for topical administration, such as an eye drop.

A pharmaceutically acceptable carrier can contain physiologically acceptable agents that act, for example, to stabilize, increase solubility or to increase the absorption of a compound such as a compound of the invention. Such physiologically acceptable agents include, for example, carbohydrates, such as glucose, sucrose or dextrans, antioxidants, such as ascorbic acid or glutathione, chelating agents, low molecular weight proteins or other stabilizers or excipients. The choice of a pharmaceutically acceptable carrier, including a physiologically acceptable agent, depends, for example, on the route of administration of the composition. The preparation or pharmaceutical composition can be a self-emulsifying drug delivery system or a self-microemulsifying drug delivery system. The pharmaceutical composition (preparation) also can be a liposome or other polymer matrix, which can have incorporated therein, for example, a compound of the invention. Liposomes, for example, which comprise phospholipids or other lipids, are nontoxic, physiologically acceptable and metabolizable carriers that are relatively simple to make and administer.

The phrase "pharmaceutically acceptable" is employed herein to refer to those compounds, materials, compositions, and/or dosage forms which are, within the scope of sound medical judgment, suitable for use in contact with the tissues of a subject without excessive toxicity, irritation, allergic response, or other problem or complication, commensurate with a reasonable benefit/risk ratio.

"Pharmaceutically acceptable salt" is used herein to refer to an acid addition salt or a basic addition salt which is suitable for or compatible with the treatment of patients.

The term "pharmaceutically acceptable acid addition salt" as used herein means any non-toxic organic or inorganic salt of the disclosed compounds. Illustrative inorganic acids which form suitable salts include hydrochloric, hydrobromic, sulfuric and phosphoric acids, as well as metal salts such as sodium monohydrogen orthophosphate and potassium hydrogen sulfate. Illustrative organic acids that form suitable salts include mono-, di-, and tricarboxylic acids such as glycolic, lactic, pyruvic, malonic, succinic, glutaric, fumaric, malic, tartaric, bitartaric, citric, ascorbic, maleic, benzoic, phenylacetic, cinnamic, salicylic, and sulfosalicylic acids, as well as sulfonic acids such as p-toluene sulfonic and methanesulfonic acids. Either the mono or di-acid salts can be formed, and such salts may exist in either a hydrated, solvated or substantially anhydrous form. In general, the acid addition salts of compounds disclosed herein are more soluble in water and various hydrophilic organic solvents, and generally demonstrate higher melting points in comparison to their free base forms. The selection of the appropriate salt will be known to one skilled in the art. Other non-pharmaceutically acceptable salts, e.g., oxalates, may be used, for example, in the isolation of compounds disclosed herein for laboratory use, or for subsequent conversion to a pharmaceutically acceptable acid addition salt.

The term "pharmaceutically acceptable basic addition salt" as used herein means any non-toxic organic or inorganic base addition salt of any acid compounds disclosed herein. Illustrative inorganic bases which form suitable salts include lithium, sodium, potassium, calcium, magnesium, or barium hydroxide. Illustrative organic bases which form suitable salts include aliphatic, alicyclic, or aromatic organic amines such as methylamine, trimethylamine and picoline or ammonia. The selection of the appropriate salt will be known to a person skilled in the art.

The phrase "pharmaceutically acceptable carrier" as used herein means a pharmaceutically acceptable material, composition or vehicle, such as a liquid or solid filler, diluent, excipient, solvent or encapsulating material. Each carrier must be "acceptable" in the sense of being compatible with the other ingredients of the formulation and not injurious to the subject. Some examples of materials which can serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

A pharmaceutical composition (preparation) can be administered to a subject by any of a number of routes of administration including, for example, orally (for example, drenches as in aqueous or non-aqueous solutions or suspensions, tablets, capsules (including sprinkle capsules and gelatin capsules), boluses, powders, granules, pastes for application to the tongue); absorption through the oral mucosa (e.g., sublingually); anally, rectally or vaginally (for example, as a pessary, cream or foam); parenterally (including intramuscularly, intravenously, subcutaneously or intrathecally as, for example, a sterile solution or suspension); nasally; intraperitoneally; subcutaneously; transdermally (for example as a patch applied to the skin); and topically (for example, as a cream, ointment or spray applied to the skin, or as an eye drop). The compound may also be formulated for inhalation. In certain embodiments, a compound may be simply dissolved or suspended in sterile water. Details of appropriate routes of administration and compositions suitable for same can be found in, for example, U.S. Pat. Nos. 6,110,973, 5,763,493, 5,731,000, 5,541,231, 5,427,798, 5,358,970 and 4,172,896, as well as in patents cited therein.

The formulations may conveniently be presented in unit dosage form and may be prepared by any methods well known in the art of pharmacy. The amount of active ingredient which can be combined with a carrier material to produce a single dosage form will vary depending upon the subject being treated, the particular mode of administration. The amount of active ingredient that can be combined with a carrier material to produce a single dosage form will generally be that amount of the compound which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Methods of preparing these formulations or compositions include the step of bringing into association an active compound, such as a compound of the invention, with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules (including sprinkle capsules and gelatin capsules), cachets, pills, tablets, lozenges (using a flavored basis, usually sucrose and acacia or tragacanth), lyophile, powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. Compositions or compounds may also be administered as a bolus, electuary or paste.

To prepare solid dosage forms for oral administration (capsules (including sprinkle capsules and gelatin capsules), tablets, pills, dragees, powders, granules and the like), the active ingredient is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; (10) complexing agents, such as, modified and unmodified cyclodextrins; and (11) coloring agents. In the case of capsules (including sprinkle capsules and gelatin capsules), tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions, such as dragees, capsules (including sprinkle capsules and gelatin capsules), pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions that can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions that can be used include polymeric substances and waxes. The active ingredient can also be in micro-encapsulated form, if appropriate, with one or more of the above-described excipients.

Liquid dosage forms useful for oral administration include pharmaceutically acceptable emulsions, lyophiles for reconstitution, microemulsions, solutions, suspensions, syrups and elixirs. In addition to the active ingredient, the liquid dosage forms may contain inert diluents commonly used in the art, such as, for example, water or other solvents, cyclodextrins and derivatives thereof, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions for rectal, vaginal, or urethral administration may be presented as a suppository, which may be prepared by mixing one or more active compounds with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations of the pharmaceutical compositions for administration to the mouth may be presented as a mouthwash, or an oral spray, or an oral ointment.

Alternatively or additionally, compositions can be formulated for delivery via a catheter, stent, wire, or other intraluminal device. Delivery via such devices may be especially useful for delivery to the bladder, urethra, ureter, rectum, or intestine.

Formulations which are suitable for vaginal administration also include pessaries, tampons, creams, gels, pastes, foams or spray formulations containing such carriers as are known in the art to be appropriate.

Dosage forms for the topical or transdermal administration include powders, sprays, ointments, pastes, creams, lotions, gels, solutions, patches and inhalants. The active compound may be mixed under sterile conditions with a pharmaceutically acceptable carrier, and with any preservatives, buffers, or propellants that may be required.

The ointments, pastes, creams and gels may contain, in addition to an active compound, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to an active compound, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the present invention to the body. Such dosage forms can be made by dissolving or dispersing the active compound in the proper medium. Absorption enhancers can also be used to increase the flux of the compound across the skin. The rate of such flux can be controlled by either providing a rate controlling membrane or dispersing the compound in a polymer matrix or gel.

Ophthalmic formulations, eye ointments, powders, solutions and the like, are also contemplated as being within the scope of this invention. Exemplary ophthalmic formulations are described in U.S. Publication Nos. 2005/0080056, 2005/0059744, 2005/0031697 and 2005/004074 and U.S. Patent No. 6,583,124, the contents of which are incorporated herein by reference. If desired, liquid ophthalmic formulations have properties similar to that of lacrimal fluids, aqueous humor or vitreous humor or are compatible with such fluids. A preferred route of administration is local administration (e.g., topical administration, such as eye drops, or administration via an implant).

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, intraocular, subcapsular, subarachnoid, intraspinal and intrasternal injection and infusion.

Pharmaceutical compositions suitable for parenteral administration comprise one or more active compounds in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers that may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents that delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution, which, in turn, may depend upon crystal size and crystalline form. Alternatively, delayed absorption of a parenterally administered drug form is accomplished by dissolving or suspending the drug in an oil vehicle.

Injectable depot forms are made by forming microencapsulated matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions that are compatible with body tissue.

For use in the methods of this invention, active compounds can be given per se or as a pharmaceutical composition containing, for example, about 0.1 to about 99.5% (more preferably, about 0.5 to about 90%) of active ingredient in combination with a pharmaceutically acceptable carrier.

Methods of introduction may also be provided by rechargeable or biodegradable devices. Various slow release polymeric devices have been developed and tested *in vivo* in recent years for the controlled delivery of drugs, including proteinaceous biopharmaceuticals. A variety of biocompatible polymers (including hydrogels), including both biodegradable and non-degradable polymers, can be used to form an implant for the sustained release of a compound at a particular target site.

Actual dosage levels of the active ingredients in the pharmaceutical compositions may be varied so as to obtain an amount of the active ingredient that is effective to achieve the desired therapeutic response for a particular patient, composition, and mode of administration, without being toxic to the patient.

The selected dosage level will depend upon a variety of factors including the activity of the particular compound or combination of compounds employed, or the ester, salt or amide thereof, the route of administration, the time of administration, the rate of excretion of the particular compound(s) being employed, the duration of the treatment, other drugs, compounds and/or materials used in combination with the particular compound(s) employed, the age, sex, weight, condition, general health and prior medical history of the subject being treated, and like factors well known in the medical arts.

A physician or veterinarian having ordinary skill in the art can readily determine and prescribe the therapeutically effective amount of the pharmaceutical composition required. For example, the physician or veterinarian could start doses of the pharmaceutical composition or compound at levels lower than that required in order to achieve the desired therapeutic effect and gradually increase the dosage until the desired effect is achieved. By "therapeutically effective amount" is meant the concentration of a compound that is sufficient to elicit the desired therapeutic effect. It is generally understood that the effective amount of the compound will vary according to the weight, sex, age, and medical history of the subject. Other factors which influence the effective amount may include, but are not limited to, the severity of the subject's condition, the disorder being treated, the stability of the compound, and, if desired, another type of therapeutic agent being administered with the compound of the invention. A larger total dose can be delivered by multiple administrations of the agent. Methods to determine efficacy and dosage are known to those skilled in the art (Isselbacher et al. (1996) Harrison's Principles of Internal Medicine 13 ed., 1814-1882, herein incorporated by reference).

In general, a suitable daily dose of an active compound used in the compositions and methods of the invention will be that amount of the compound that is the lowest dose effective to produce a therapeutic effect. Such an effective dose will generally depend upon the factors described above.

If desired, the effective daily dose of the active compound may be administered as one, two, three, four, five, six or more sub-doses administered separately at appropriate intervals throughout the day, optionally, in unit dosage forms. In certain embodiments of the present invention, the active compound may be administered two or three times daily. In certain embodiments, the active compound will be administered once daily.

In certain embodiments, compounds of the invention may be used alone or conjointly administered with another type of therapeutic agent. As used herein, the phrase "conjoint administration" refers to any form of administration of two or more different therapeutic compounds such that the second compound is administered while the previously administered therapeutic compound is still effective in the body *(e.g.,* the two compounds are simultaneously effective in the subject, which may include synergistic effects of the two compounds). For example, the different therapeutic compounds can be administered either in the same formulation or in a separate formulation, either concomitantly or sequentially. In certain embodiments, the different therapeutic compounds can be administered within one hour, 12 hours, 24 hours, 36 hours, 48 hours, 72 hours, or a week of one another. Thus, a subject who receives such treatment can benefit from a combined effect of different therapeutic compounds.

In certain embodiments, conjoint administration of compounds of the invention with one or more additional therapeutic agent(s) provides improved efficacy relative to each individual administration of the compound of the invention or the one or more additional therapeutic agent(s). In certain such embodiments, the conjoint administration provides an additive effect, wherein an additive effect refers to the sum of each of the effects of individual administration of the compound of the invention and the one or more additional therapeutic agent(s).

This invention includes the use of pharmaceutically acceptable salts of compounds of the invention in the compositions and methods of the present invention. In certain embodiments, contemplated salts of the invention include, but are not limited to, alkyl, dialkyl, trialkyl or tetraalkyl ammonium salts. In certain embodiments, contemplated salts of the invention include, but are not limited to, L-arginine, benenthamine, benzathine, betaine, calcium hydroxide, choline, deanol, diethanolamine, diethylamine, 2-(diethylamino)ethanol, ethanolamine, ethylenediamine, N-methylglucamine, hydrabamine, 1H-imidazole, lithium, L-lysine, magnesium, 4-(2-hydroxyethyl)morpholine, piperazine, potassium, 1-(2-hydroxyethyl)pyrrolidine, sodium, triethanolamine, tromethamine, and zinc salts. In certain embodiments, contemplated salts of the invention include, but are not limited to, Na, Ca, K, Mg, Zn or other metal salts.

The pharmaceutically acceptable acid addition salts can also exist as various solvates, such as with water, methanol, ethanol, dimethylformamide, and the like. Mixtures of such solvates can also be prepared. The source of such solvate can be from the solvent of crystallization, inherent in the solvent of preparation or crystallization, or adventitious to such solvent.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: (1) water-soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal-chelating agents, such as citric acid, ethylenediamine tetraacetic acid (EDTA), sorbitol, tartaric acid, phosphoric acid, and the like.

### Methods of Treatment

The non-selective Ca²⁺-permeable Transient Receptor Potential (TRP) channels act as sensors that transduce extracellular cues to the intracellular environment in diverse cellular processes, including actin remodeling and cell migration (Greka et al., Nat Neurosci 6, 837-845, 2003; Ramsey et al., Annu Rev Physiol 68, 619-647, 2006; Montell, Pflugers Arch 451, 19-28, 2005; Clapham, Nature 426, 517-524, 2003). Dynamic rearrangement of the actin cytoskeleton relies on spatiotemporally regulated Ca²⁺ influx (Zheng and Poo, Annu Rev Cell Dev Biol 23, 375-404, 2007); Brandman and Meyer, Science 322, 390-395, 2008); Collins and Meyer, Dev Cell 16, 160-161, 2009) and the small GTPases RhoA and Rac1 serve as key modulators of these changes (Etienne-Manneville and Hall, Nature 420, 629-635, 2002); Raftopoulou and Hall, Dev Biol 265, 23-32, 2004). RhoA induces stress fiber and focal adhesion formation, while Rac1 mediates lamellipodia formation (Etienne-Manneville and Hall, Nature 420, 629-635, 2002). The Transient Receptor Potential Cation Channel, subfamily C, member 5 (TRPC5) acts in concert with TRPC6 to regulate Ca²⁺ influx, actin remodeling, and cell motility in kidney podocytes and fibroblasts. TRPC5-mediated Ca²⁺ influx increases Rac1 activity, whereas TRPC6-mediated Ca²⁺ influx promotes RhoA activity. Gene silencing of TRPC6 channels abolishes stress fibers and diminishes focal contacts, rendering a motile, migratory cell phenotype. In contrast, gene silencing of TRPC5 channels rescues stress fiber formation, rendering a contractile cell phenotype. The results described herein unveil a conserved signaling mechanism whereby TRPC5 and TRPC6 channels control a tightly regulated balance of cytoskeletal dynamics through differential coupling to Rac1 and RhoA.

Ca²⁺-dependent remodeling of the actin cytoskeleton is a dynamic process that drives cell migration (Wei et al., Nature 457, 901-905, 2009). RhoA and Rac1 act as switches responsible for cytoskeletal rearrangements in migrating cells (Etienne-Manneville and Hall, Nature 420, 629-635, 2002); Raftopoulou and Hall, Dev Biol 265, 23-32, 2004). Activation of Rac1 mediates a motile cell phenotype, whereas RhoA activity promotes a contractile phenotype (Etienne-Manneville and Hall, Nature 420, 629-635, 2002). Ca²⁺ plays a central role in small GTPase regulation (Aspenstrom et al., Biochem J 377, 327-337, 2004). Spatially and temporally restricted flickers of Ca²⁺ are enriched near the leading edge of migrating cells (Wei et al., Nature 457, 901-905, 2009). Ca2+microdomains have thus joined local bursts in Rac1 activity (Gardiner et al., Curr Biol 12, 2029-2034, 2002; Machacek et al., Nature 461, 99-103, 2009) as critical events at the leading edge. To date, the sources of Ca²⁺ influx responsible for GTPase regulation remain largely elusive. TRP (Transient Receptor Potential) channels generate time and space-limited Ca²⁺ signals linked to cell migration in fibroblasts and neuronal growth cones0. Specifically, TRPC5 channels are known regulators of neuronal growth cone guidance1 and their activity in neurons is dependent on PI3K and Rac1 activity (Bezzerides et al., Nat Cell Biol 6, 709-720, 2004).

Podocytes are neuronal-like cells that originate from the metanephric mesenchyme of the kidney glomerulus and are essential to the formation of the kidney filtration apparatus (Somlo and Mundel, Nat Genet. 24, 333-335, 2000; Fukasawa et al., J Am Soc Nephrol 20, 1491-1503, 2009). Podocytes possess an exquisitely refined repertoire of cytoskeletal adaptations to environmental cues (Somlo and Mundel, Nat Genet 24, 333-335, 2000; Garg et al., Mol Cell Biol 27, 8698-8712, 2007; Verma et al., J Clin Invest 116, 1346-1359, 2006; Verma et al., J Biol Chem 278, 20716-20723, 2003; Barletta et al., J Biol Chem 278, 19266-19271, 2003; Holzman et al., Kidney Int 56, 1481-1491, 1999; Ahola et al., Am J Pathol 155, 907-913, 1999; Tryggvason and Wartiovaara, N Engl J Med 354, 1387-1401, 2006; Schnabel and Farquhar, J Cell Biol 111, 1255-1263, 1990; Kurihara et al., Proc Natl Acad Sci USA 89, 7075-7079, 1992). Early events of podocyte injury are characterized by dysregulation of the actin cytoskeleton (Faul et al., Trends Cell Biol 17, 428-437, 2007; Takeda et al., J Clin Invest 108, 289-301, 2001; Asanuma et al., Nat Cell Biol 8, 485-491, 2006) and Ca²⁺ homeostasis (Hunt et al., J Am Soc Nephrol 16, 1593-1602, 2005; Faul et al., Nat Med 14, 931-938, 2008). These changes are associated with the onset of proteinuria, the loss of albumin into the urinary space, and ultimately kidney failure (Tryggvason and Wartiovaara, N Engl J Med 354, 1387-1401, 2006). The vasoactive hormone Angiotensin II induces Ca²⁺ influx in podocytes, and prolonged treatment results in loss of stress fibers (Hsu et al., J Mol Med 86, 1379-1394, 2008). While there is a recognized link between Ca²⁺ influx and cytoskeletal reorganization, the mechanisms by which the podocyte senses and transduces extracellular cues that modulate cell shape and motility remain elusive. TRP Canonical 6 (TRPC6) channel mutations have been linked to podocyte injury (Winn et al., Science 308, 1801-1804, 2005; Reiser et al., Nat Genet 37, 739-744, 2005; Moller et al., J Am Soc Nephrol 18, 29-36, 2007; Hsu et al., Biochim Biophys Acta 1772, 928-936, 2007), but little is known about the specific pathways that regulate this process. Moreover, TRPC6 shares close homology with six other members of the TRPC channel family (Ramsey et al., Annu Rev Physiol 68, 619-647, 2006; Clapham, Nature 426, 517-524, 2003). TRPC5 channels antagonize TRPC6 channel activity to control a tightly regulated balance of cytoskeletal dynamics through differential coupling to distinct small GTPases.

### Proteinuria

Proteinuria is a pathological condition wherein protein is present in the urine. Albuminuria is a type of proteinuria. Microalbuminuria occurs when the kidney leaks small amounts of albumin into the urine. In a properly functioning body, albumin is not normally present in urine because it is retained in the bloodstream by the kidneys. Microalbuminuria is diagnosed either from a 24-hour urine collection (20 to 200 µg/min) or, more commonly, from elevated concentrations (30 to 300 mg/L) on at least two occasions. Microalbuminuria can be a forerunner of diabetic nephropathy. An albumin level above these values is called macroalbuminuria. Subjects with certain conditions, e.g., diabetic nephropathy, can progress from microalbuminuria to macroalbuminuria and reach a nephrotic range (>3.5 g/24 hours) as kidney disease reaches advanced stages.

### Causes of Proteinuria

Proteinuria can be associated with a number of conditions, including focal segmental glomerulosclerosis, IgA nephropathy, diabetic nephropathy, lupus nephritis, membranoproliferative glomerulonephritis, progressive (crescentic) glomerulonephritis, and membranous glomerulonephritis. Each of these conditions may be treated by the patient stratification methods described herein.

Some of the kidney disorders that may be treated by the methods described herein are detailed below.

### A. Focal Segmental Glomerulosclerosis (FSGS)

Focal Segmental Glomerulosclerosis (FSGS) is a disease that attacks the kidney's filtering system (glomeruli) causing serious scarring. FSGS is one of the many causes of a disease known as Nephrotic Syndrome, which occurs when protein in the blood leaks into the urine (proteinuria). Primary FSGS, when no underlying cause is found, usually presents as nephrotic syndrome. Secondary FSGS, when an underlying cause is identified, usually presents with kidney failure and proteinuria. FSGS can be genetic; there are currently several known genetic causes of the hereditary forms of FSGS.

Very few treatments are available for patients with FSGS. Many patients are treated with steroid regimens, most of which have very harsh side effects. Some patients have shown to respond positively to immunosuppressive drugs as well as blood pressure drugs which have shown to lower the level of protein in the urine. To date, there is no commonly accepted effective treatment or cure and there are no FDA approved drugs to treat FSGS. Therefore, more effective methods to reduce or inhibit proteinuria are desirable.

### B. Diabetic Nephropathy

Diabetic nephropathy, also known as Kimmelstiel-Wilson syndrome and intercapillary glomerulonephritis, is a progressive kidney disease caused by angiopathy of capillaries in the kidney glomeruli. It is characterized by nephrotic syndrome and diffuse glomerulosclerosis. It is due to longstanding diabetes mellitus and is a prime cause for dialysis. The earliest detectable change in the course of diabetic nephropathy is a thickening in the glomerulus. At this stage, the kidney may start allowing more serum albumin than normal in the urine. As diabetic nephropathy progresses, increasing numbers of glomeruli are destroyed by nodular glomerulosclerosis and the amount of albumin excreted in the urine increases.

### C. Membranoproliferative Glomerulonephritis IIIIIIII

Membranoproliferative glomerulonephritis is a type of glomerulonephritis caused by deposits in the kidney glomerular mesangium and basement membrane thickening, activating complement and damaging the glomeruli. There are three types of membranoproliferative glomerulonephritis. Type I is caused by immune complexes depositing in the kidney and is believed to be associated with the classical complement pathway. Type II is similar to Type I, however, it is believed to be associated with the alternative complement pathway. Type III is very rare and it is characterized by a mixture of subepithelial deposits and the typical pathological findings of Type I disease.

There are two major types of MPGN, which are based upon immunofluorescence microscopy: immune complex-mediated and complement-mediated. Hypocomplementemia is common in all types of MPGN. In immune complex-mediated MPGN, complement activation occurs via the classic pathway and is typically manifested by a normal or mildly decreased serum C3 concentration and a low serum C4 concentration. In complement-mediated MPGN, there are usually low serum C3 and normal C4 levels due to activation of the alternate pathway. However, complement-mediated MPGN is not excluded by a normal serum C3 concentration, and it is not unusual to find a normal C3 concentration in adults with dense deposit disease (DDD) or C3 glomerulonephritis (C3GN).

C3 glomerulonephritis (C3GN) shows a glomerulonephritis on light microscopy (LM), bright C3 staining and the absence of C1q, C4 and immunoglobulins (Ig) on immunofluorescence microscopy (IF), and mesangial and/or subendothelial electron dense deposits on electron microscopy (EM). Occasional intramembranous and subepithelial deposits are also frequently present. The term 'C3 glomerulopathy' is often used to include C3GN and Dense Deposit Disease (DDD), both of which result from dysregulation of the alternative pathway (AP) of complement. C3GN and DDD may be difficult to distinguish from each other on LM and IF studies. However, EM shows mesangial and/or subendothelial, intramembranous and subepithelial deposits in C3GN, while dense osmiophilic deposits are present along the glomerular basement membranes (GBM) and in the mesangium in DDD. Both C3GN and DDD are distinguished from immune-complex mediated glomerulonephritis by the lack of immunoglobulin staining on IF. (Sethi et al., Kidney Int. (2012) 82(4):465-473).

### D. Membranous Glomerulonephritis

Membranous glomerulonephritis (MGN) is a slowly progressive disease of the kidney affecting mostly patients between ages of 30 and 50 years, usually Caucasian. It can develop into nephrotic syndrome. MGN is caused by circulating immune complex. Current research indicates that the majority of the immune complexes are formed via binding of antibodies to antigens in situ to the glomerular basement membrane. The said antigens may be endogenous to the basement membrane, or deposited from systemic circulation.

### E. Alport syndrome

Alport syndrome is a genetic disorder affecting around 1 in 5,000-10,000 children, characterized by glomerulonephritis, end-stage kidney disease, and hearing loss. Alport syndrome can also affect the eyes, though the changes do not usually affect sight, except when changes to the lens occur in later life. Blood in urine is universal. Proteinuria is a feature as kidney disease progresses.

### F. Minimal Change Disease

Minimal change disease (also known as MCD, minimal change glomerulopathy, and nil disease, among others) is a disease affecting the kidneys which causes a nephrotic syndrome. The clinical signs of minimal change disease are proteinuria (abnormal excretion of proteins, mainly albumin, into the urine), edema (swelling of soft tissues as a consequence of water retention), weight gain, and hypoalbuminemia (low serum albumin). These signs are referred to collectively as nephrotic syndrome. The first clinical sign of minimal change disease is usually edema with an associated increase in weight. The swelling may be mild but patients can present with edema in the lower half of the body, periorbital edema, swelling in the scrotal/labial area and anasarca in more severe cases. **In** older adults, patients may also present with acute kidney injury (20-25% of affected adults) and high blood pressure. Due to the disease process, patients with minimal change disease are also at risk of blood clots and infections.

### G. Membranous nephropathy

Membranous nephropathy refers to the deposition of immune complexes on the glomerular basement membrane (GBM) with GBM thickening. The cause is usually unknown (idiopathic), although secondary causes include drugs, infections, autoimmune disorders, and cancer. Manifestations include insidious onset of edema and heavy proteinuria with benign urinary sediment, normal renal function, and normal or elevated blood pressure. Membranous nephropathy is diagnosed by renal biopsy. Spontaneous remission is common. Treatment of patients at high risk of progression is usually with corticosteroids and cyclophosphamide or chlorambucil.

### H. Postinfectious Glomerulonephritis

Acute proliferative glomerulonephritis is a disorder of the glomeruli (glomerulonephritis), or small blood vessels in the kidneys. It is a common complication of bacterial infections, typically skin infection by Streptococcus bacteria types 12, 4 and 1 (impetigo) but also after streptococcal pharyngitis, for which it is also known as postinfectious or poststreptococcal glomerulonephritis. It can be a risk factor for future albuminuria. In adults, the signs and symptoms of infection may still be present at the time when the kidney problems develop, and the terms infection-related glomerulonephritis or bacterial infection-related glomerulonephritis are also used. Acute glomerulonephritis resulted in 19,000 deaths in 2013 down from 24,000 deaths in 1990 worldwide. Acute proliferative glomerulonephritis (poststreptococcal glomerulonephritis) is caused by an infection with streptococcus bacteria, usually three weeks after infection, usually of the pharynx or the skin, given the time required to raise antibodies and complement proteins. The infection causes blood vessels in the kidneys to develop inflammation, this hampers the renal organs ability to filter urine. [Eison et al., "Poststreptococcal acute glomerulonephritis in children: clinical features and pathogenesis," Pediatr. Nephrol. 2011, 26:165-180] Acute proliferative glomerulonephritis most commonly occurs in children. In addition, glomerulopathies, such as glomerulonephritis, has also been associated with bacterial endocarditis, hepatitis C infection, HIV infection.

### I. Goodpasture Syndrome

Goodpasture syndrome, also known as anti-glomerular basement membrane disease, is a rare autoimmune disease in which antibodies attack the basement membrane in lungs and kidneys, leading to bleeding from the lungs and kidney failure. It is thought to attack the alpha-3 subunit of type IV collagen, which has therefore been referred to as Goodpasture's antigen. Goodpasture syndrome may quickly result in permanent lung and kidney damage, often leading to death.

### J. IgA Nephropathy

IgA nephropathy (also known as IgA nephritis, IgAN, Berger's disease, and synpharyngitic glomerulonephritis) is a form of glomerulonephritis (inflammation of the glomeruli of the kidney). IgA nephropathy is the most common glomerulonephritis throughout the world. Primary IgA nephropathy is characterized by deposition of the IgA antibody in the glomerulus. There are other diseases associated with glomerular IgA deposits, the most common being Henoch-Schönlein purpura (HSP), which is considered by many to be a systemic form of IgA nephropathy. Henoch-Schönlein purpura presents with a characteristic purpuric skin rash, arthritis, and abdominal pain and occurs more commonly in young adults (16-35 yrs old). HSP is associated with a more benign prognosis than IgA nephropathy. **In** IgA nephropathy there is a slow progression to chronic renal failure in 25-30% of cases during a period of 20 years.

### K. Lupus Nephritis

Lupus nephritis is a kidney disorder that is a complication of systemic lupus erythematosus. Lupus nephritis occurs when antibodies and complement build up in the kidneys, causing inflammation. It often causes proteinuria and may progress rapidly to renal failure. Nitrogen waste products build up in the bloodstream. Systemic lupus erythematosus causes various disorders of the internal structures of the kidney, including interstitial nephritis. Lupus nephritis affects approximately 3 out of 10,000 people.

### 1. Polycystic Kidney Disease

Polycystic kidney disease (PKD, also known as polycystic kidney syndrome) is a genetic disorder in which the renal tubules become structurally abnormal, resulting in the development and growth of multiple cysts within the kidney. These cysts may begin to develop in utero, in infancy, in childhood, or in adulthood. Cysts are non-functioning tubules filled with fluid pumped into them, which range in size from microscopic to enormous, crushing adjacent normal tubules and eventually rendering them non-functional as well. PKD is caused by abnormal genes that produce a specific abnormal protein; this protein has an adverse effect on tubule development. PKD is a general term for two types, each having their own pathology and genetic cause: autosomal dominant polycystic kidney disease (ADPKD) and autosomal recessive polycystic kidney disease (ARPKD). PKD affects about 500,000 people in the United States.

### Measurement of Urine Protein Levels

Protein levels in urine can be measured using methods known in the art. Until recently, an accurate protein measurement required a 24-hour urine collection. In a 24-hour collection, the patient urinates into a container, which is kept refrigerated between trips to the bathroom. The patient is instructed to begin collecting urine after the first trip to the bathroom in the morning. Every drop of urine for the rest of the day is to be collected in the container. The next morning, the patient adds the first urination after waking and the collection is complete.

More recently, researchers have found that a single urine sample can provide the needed information. In the newer technique, the amount of albumin in the urine sample is compared with the amount of creatinine, a waste product of normal muscle breakdown. The measurement is called a urine albumin-to-creatinine ratio (UACR). A urine sample containing more than 30 milligrams of albumin for each gram of creatinine (30 mg/g) is a warning that there may be a problem. If the laboratory test exceeds 30 mg/g, another UACR test should be performed 1 to 2 weeks later. If the second test also shows high levels of protein, the person has persistent proteinuria, a sign of declining kidney function, and should have additional tests to evaluate kidney function.

Tests that measure the amount of creatinine in the blood will also show whether a subject's kidneys are removing wastes efficiently. Too much creatinine in the blood is a sign that a person has kidney damage. A physician can use the creatinine measurement to estimate how efficiently the kidneys are filtering the blood. This calculation is called the estimated glomerular filtration rate, or eGFR. Chronic kidney disease is present when the eGFR is less than 60 milliliters per minute (mL/min).

### TRPC5

TRPC is a family of transient receptor potential cation channels in animals. TRPC5 is subtype of the TRPC family of mammalian transient receptor potential ion channels. Three examples of TRPC5 are highlighted below in Table 1.

**TABLE 1**

| The TRPC5 orthologs from three different species along with their GenBank Ref Seq Accession Numbers. | | | |
|---|---|---|---|
| Species | Nucleic Acid | Amino Acid | GeneID |
| *Homo sapiens* | NM_012471.2 | NP_036603.1 | 7224 |
| *Mus musculus* | NM_009428.2 | NP_033454.1 | 22067 |
| *Rattus norvegicus* | NM_080898.2 | NP_543174.1 | 140933 |

The transient receptor potential channel 5 (TRPC5) is a calcium-permeable nonspecific cation channel predominantly expressed in the brain where it can form heterotetrameric complexes with TRPC1 and TRPC4 channel subunits. TRPC5 is also expressed in the kidney, more specifically in podocytes where it is involved in the regulation of the podocyte actin cytoskeleton.

Accordingly, in certain embodiments, the invention provides methods for treating a subject suffering from, or the reducing risk of developing, a kidney disease selected from diabetic nephropathy, focal segmental glomerulosclerosis, minimal change disease, membranoproliferative glomerulonephritis (including post-streptococcal glomerulonephritis and bacterial endocarditis-associated glomerulonephritis), membranous nephropathy, other hepatitis C virus-associated glomerulopathies, HIV-associated glomerulopathies, COVID-19-associated acute kidney injury, Alport syndrome, polycystic kidney disease (both autosomal dominant and autosomal recessive), IgA nephropathy, other genetic nephropathies or ciliopathies (e.g., HNF1beta, nephronophthisis, autosomal dominant cystic/tubular kidney disease), lupus nephritis, Goodpasture's syndrome (anti-GBM disease), and other complement- or immune-mediated kidney diseases, wherein the subject has urinary levels of one or more biomarkers selected from Rac1, Rac1-GTP, phospho-LIM kinase 1, and phospho-cofilin above a pre-determined threshold, the method comprising administering to the subject in need thereof a therapeutically effective amount of a compound of the invention (e.g., a compound of structural formula I, II, III, IV, V, VI, VII, VIII, IX, X, or XI, or a calcineurin inhibitor) or a pharmaceutical composition comprising said compound. In some aspects of these embodiments, the kidney disease is selected from diabetic nephropathy, focal segmental glomerulosclerosis, minimal change disease, membranoproliferative glomerulonephritis, membranous nephropathy, other hepatitis C virus-associated glomerulonephropathies, and Alport syndrome.

In some embodiments, the kidney disease is diabetic nephropathy, or focal segmental glomerulosclerosis.

### Subjects to be Treated

In one aspect of the invention, a subject is selected on the basis that they have urinary levels of one or more biomarkers selected from Rac1, Rac1-GTP, phospho-LIM kinase 1, and phospho-cofilin above a pre-determined threshold; and have or are at risk of developing, a kidney disease, such as diabetic nephropathy, focal segmental glomerulosclerosis, minimal change disease, membranoproliferative glomerulonephritis (including post-streptococcal glomerulonephritis and bacterial endocarditis-associated glomerulonephritis), membranous nephropathy, other hepatitis C virus-associated glomerulopathies, HIV-associated glomerulopathies, COVID-19-associated acute kidney injury, Alport syndrome, polycystic kidney disease (both autosomal dominant and autosomal recessive), IgA nephropathy, other genetic nephropathies or ciliopathies (e.g., HNF1beta, nephronophthisis, autosomal dominant cystic/tubular kidney disease), lupus nephritis, Goodpasture's syndrome (anti-GBM disease), and other complement- or immune-mediated kidney diseases.

In some specific aspects, the subject to be treated has or is at risk of developing diabetic nephropathy, focal segmental glomerulosclerosis, minimal change disease, membranoproliferative glomerulonephritis, membranous nephropathy, other hepatitis C virus-associated glomerulopathies, or Alport syndrome. Subjects that have, or are at risk of developing, proteinuria include those with diabetes, hypertension, or certain family backgrounds. In the United States, diabetes is the leading cause of end-stage renal disease (ESRD). In both type 1 and type 2 diabetes, albumin in the urine is one of the first signs of deteriorating kidney function. As kidney function declines, the amount of albumin in the urine increases. Another risk factor for developing proteinuria is hypertension. Proteinuria in a person with high blood pressure is an indicator of declining kidney function. If the hypertension is not controlled, the person can progress to full kidney failure. African Americans are more likely than Caucasians to have high blood pressure and to develop kidney problems from it, even when their blood pressure is only mildly elevated. Other groups at risk for proteinuria are American Indians, Hispanics/Latinos, Pacific Islander Americans, older adults, and overweight subjects.

In one aspect of the invention, a subject is selected on the basis that they have urinary levels of one or more biomarkers selected from Rac1, Rac1-GTP, phospho-LIM kinase **1,** and phospho-cofilin above a pre-determined threshold; and have, or are at risk of developing proteinuria. A subject that has, or is at risk of developing, proteinuria is one having one or more symptoms of the condition. Symptoms of proteinuria are known to those of skill in the art and include, without limitation, large amounts of protein in the urine, which may cause it to look foamy in the toilet. Loss of large amounts of protein may result in edema, where swelling in the hands, feet, abdomen, or face may occur. These are signs of large protein loss and indicate that kidney disease has progressed. Laboratory testing is the only way to find out whether protein is in a subject's urine before extensive kidney damage occurs.

The methods are effective for a variety of subjects including mammals, e.g., humans and other animals, such as laboratory animals, e.g., mice, rats, rabbits, or monkeys, or domesticated and farm animals, e.g., cats, dogs, goats, sheep, pigs, cows, or horses. In some embodiments, the subject is a mammal. In some embodiments, the subject is a human.

In one aspect, the invention relates to a method of selecting and treating a human subject suffering from a kidney disease, the method comprising the steps of:
a. selecting the subject if a urinary level of one or more biomarkers selected from Rac1, Rac1-GTP, phospho-LIM kinase 1, and phospho-cofilin in the subject is above a pre-determined threshold; and
b. administering to the selected subject a pharmaceutical composition comprising a TRPC5 inhibitor or a calcineurin inhibitor; and a pharmaceutically acceptable carrier.

In one aspect, the invention relates to a method of treating a human subject suffering from a kidney disease comprising the step of
administering to the subject a pharmaceutical composition comprising
   a TRPC5 inhibitor or a calcineurin inhibitor, and
   a pharmaceutically acceptable carrier;
only if the subject is determined to have a pre-treatment urinary level of one or more biomarkers selected from Rac1, Rac1-GTP, phospho-LIM kinase 1, and phospho-cofilin that is above a pre-determined threshold.

In accordance with these aspects, a subject whose pre-treatment urinary level of one or more biomarkers selected from Rac1, Rac1-GTP, phospho-LIM kinase 1, and phospho-cofilin is below a pre-determined threshold for that biomarker is not treated with a TRPC5 inhibitor or a calcineurin inhibitor. In some specific aspects, a subject whose pre-treatment urinary level of Rac1 is below a pre-determined threshold is not treated with a TRPC5 inhibitor or a calcineurin inhibitor. In other specific aspects, a subject whose pre-treatment urinary level of Rac1-GTP is below a pre-determined threshold is not treated with a TRPC5 inhibitor or a calcineurin inhibitor. In still other specific aspects, a subject whose pre-treatment urinary level of phospho-LIM kinase 1 is below a pre-determined threshold is not treated with a TRPC5 inhibitor or a calcineurin inhibitor. In yet other specific aspects, a subject whose pre-treatment urinary level of phospho-cofilin is below a pre-determined threshold is not treated with a TRPC5 inhibitor or a calcineurin inhibitor.

### Rac1 and Rac1-GTP

Rac1, also known as Ras-related C3 botulinum toxin substrate 1, is a small (~21 kDa) signaling G protein (more specifically a GTPase) found in human cells, and is a member of the Rac subfamily of the family Rho family of GTPases. Members of this superfamily appear to regulate a diverse array of cellular events, including the control of GLUT4 translocation to glucose uptake, cell growth, cytoskeletal reorganization, antimicrobial cytotoxicity, and the activation of protein kinases. Rac1 is expressed in significant amounts in insulin sensitive tissues, such as adipose tissue and skeletal muscle. Here Rac1 regulated the translocation of glucose transporting GLUT4 vesicles from intracellular compartments to the plasma membrane. In response to insulin, this allows for blood glucose to enter the cell to lower blood glucose. In conditions of obesity and type 2 diabetes, Rac1 signaling in skeletal muscle is dysfunctional, suggesting that Rac1 contributes to the progression of the disease. Rac1 protein is also necessary for glucose uptake in skeletal muscle activated by exercise and muscle stretching. RAC1 has two conformations, active (RAC1-GTP) and inactive (RAC1-GDP). [Laboratory Investigation (2018) 98:989-998; Cell Mol Life Sci. (2009) 66:370-4].

### phospho-LIM kinase 1

LIM kinase-1 (LIMK1) and LIM kinase-2 (LIMK2) are actin-binding kinases that phosphorylate members of the ADF/cofilin family of actin binding and filament severing proteins. ADF/cofilin are the only substrates yet identified for the LIM kinases. LIM kinases directly phosphorylate and inactivate members of the cofilin family, resulting in stabilization of filamentous (F)-actin. Lim kinases are activated by signaling through small GTPases of the Rho family. LIM domains are highly conserved cysteine-rich structures containing 2 zinc fingers. Although zinc fingers usually function by binding to DNA or RNA, the LIM motif probably mediates protein-protein interactions. LIM kinase-1 and LIM kinase-2 belong to a small subfamily with a unique combination of 2 N-terminal LIM motifs and a C-terminal protein kinase domain.

### phospho-cofilin

Cofilin and actin-depolymerization factor (ADF) are members of a family of essential conserved small actin-binding proteins that play pivotal roles in cytokinesis, endocytosis, embryonic development, stress response, and tissue regeneration (Carlier, M.F. et al. (1999) J Biol Chem 274, 33827-30.). In response to stimuli, cofilin promotes the regeneration of actin filaments by severing preexisting filaments (Condeelis, J. (2001) Trends Cell Biol 11, 288-93.). The severing activity of cofilin is inhibited by LIMK or TESK phosphorylation at Ser3 of cofilin (Arber, S. et al. (1998) Nature 393, 805-9; Yang, N. et al. (1998) Nature 393, 809-12; Toshima, J. et al. (2001) J Biol Chem 276, 31449-58.). Phosphorylation at Ser3 also regulates cofilin translocation from the nucleus to the cytoplasm (Nebl, G. et al. (1996) J Biol Chem 271, 26276-80.). [https://www.cellsignal.com/products/primary-antibodies/phospho-cofilin-ser3-77g2-rabbit-mab/3313]

### Measuring Urinary Levels of Biomarkers

Protein levels in urine can be measured using methods known in the art as described above, and biomarkers can be measured in urine samples that have been concenrated. Antibodies specific for Rac1, Rac1-GTP, phospho-LIM kinase 1, and phospho-cofilin are commercially available. Urine samples can be treated with one or more of these antibodies according to methods known to those of ordinary skill.

In some emodiments, the subject is selected on the basis of having a urinary level of Rac1 above a pre-determined threshold. The threshold can be adjusted based on the Rac1 (or other metabolite) level in subjects having a specific kidney disease (e.g., for FSGS versus membranous nephropathy) and/or based on clinical trial results.

In some embodiments, the urinary Rac1 level in a subject is measured in a fraction of urine comprising extracellular vesicles. When active Rac1 localizes to the plasma membrane, microvesicles are a class of extracellular vesicles that form from budding off the plasma membrane. Microvesicle release is increased by calcium increase and cytoskeletal disruption, events which are central to podocyte damage in FSGS and DN. Extracellular vesicles can be fractionated and isolated from urine, according to certain embodiments, using ultracentrifugation.

In some embodiments, the pre-determined threshold level is established by determining the range of urinary levels of the selected biomarker in a population of healthy humans; and establishing the pre-determined threshold level for the selected biomarker at a level above the 75^{th} percentile in the population. As used herein, the "n^{th} percentile" refers to a value on a scale of 100 that indicates a percent of a distribution that is equal to or below it. For example, a biomarker level above the 75^{th} percentile in a population refers to the level of a biomarker that is present at a concentration greater than its value in the lower 75% of the population. As used herein, a "population" is a group or cohort of subjects (e.g., mammals, felines, canines, primates, or humans); for example, in some embodiments, the pre-determined threshold level is established by determining the range of urinary levels of the selected biomarker in a population of healthy humans (i.e., in a group or cohort of healthy humans); and establishing the pre-determined threshold level for the selected biomarker at a level above the 75^{th} percentile in the population.

In some embodiments, the pre-determined threshold level is at a level above the 90^{th} percentile in the population. In some embodiments, the pre-determined threshold level is at a level above the 95^{th} percentile in the population.

In some embodiments, the pre-determined threshold level for urinary Rac1 is between 100-500 pg/mL.

### Methods for Determining Efficacy of Therapy

In one aspect, the present invention relates to a method of determining the efficacy of a TRPC5 inhibitor therapy in a human subject suffering from a kidney disease, wherein prior to commencing the therapy the subject was determined to have a pre-treatment urinary level of one or more biomarkers selected from Rac1, Rac1-GTP, phospho-LIM kinase 1, and phospho-cofilin that is above a pre-determined threshold, the method comprising:
a. obtaining the urinary level of the selected biomarker in the human subject at a time after initiation of TRPC5 therapy;
b. comparing the level of the selected biomarker in step a. with the pre-treatment urinary level of the selected biomarker;
c. determining that the TRPC5 inhibitor therapy is efficacious if the level of the selected biomarker in step a. is lower than the pre-treatment urinary level of the selected biomarker.

In one aspect, the invention relates to a method of determining the efficacy of a TRPC5 inhibitor therapy in a human subject suffering from a kidney disease, wherein prior to commencing the therapy the subject was determined to have a pre-treatment urinary level of one or more biomarkers selected from Rac1, Rac1-GTP, phospho-LIM kinase 1, and phospho-cofilin that is above a pre-determined threshold, the method comprising:
a. obtaining the urinary level of the selected biomarker in the human subject at a time after initiation of TRPC5 therapy; and
b. determining that the TRPC5 inhibitor therapy is efficacious if the level of the selected biomarker in step a. is lower than the pre-determined threshold for the selected biomarker.

### EXAMPLES

The invention is further described in the following examples, which do not limit the scope of the invention described in the claims.

### Example 1. TRPC5 Activity Assay

ICLN-1633 cells (HEK-TREx hTRPC5) expressing TRPC5 were generated as follows. Commercially available HekTrex-293 cells were seeded at 0.7x10⁶ cells/well in a 1x6-well plate 24 hrs prior to transfection using 2 mL cell growth media containing no antibiotics (1x DMEM/high glucose (Hyclone #SH30022.02); 10% fetal bovine serum (Sigma) 2mM sodium pyruvate, 10 mM HEPES). The human TRPC5 coding sequence (NM_012471 with a silent T478C mutation) was cloned into pcDNA5/TO (Invitrogen; Cat No. V103320) using hygromycin as the resistance gene and the plasmid (SEQ ID NO:2) propagated using T-Rex-293 cells (Invitrogen; Cat No. R71007) following manufacturer's directions. On day 2, 2 µg of plasmid DNA plus 6 µl of Xtreme-GENE HP reagent in Optimem (200 µl total volume) was prepared and incubated for 15 min at room temperature. This plasmid solution was then gently overlayed dropwise onto each well and the plate was gently swirled to mix complex with the media for approximately 30 seconds. Transfected cells were incubated at 37 °C in a 10% CO₂ incubator for 24 hrs. The transfected cells were harvested and transferred into 2 x 150mm dishes containing cell growth media with no antibiotics at 37 °C

The next day selection was initiated to generate a stable pool by adding cell growth media containing 150 µg/mL Hygromycin and 5 µg/mL Blasticidin and cells were allowed to grow. Media with the selection agent was changed every 1-2 days as needed to remove dead cells. After 7 days, the hygromycin concentration was reduced to 75 µg/mL and cells growth was allowed to continue.

Single clones were selected as follows. The stable pool was diluted to 10 cells/mL and seeded (100 µl/well) into 24 x 96 well plates (~1 cell/well) and allowed to grow for 7 days in cell growth media. Fresh media (100 µl) was added and the cells allowed to grow for another 1-2 weeks and then stored frozen or used immediately.

Compounds were made up to, or supplied as a 10 mM stock solution generally using DMSO as the vehicle. 10-point dose response curves were generated using the Echo-550 acoustic dispenser. Compound source plates were made by serially diluting compound stocks to create 10 mM, 1 mM, and 0.1 mM solutions in DMSO into Echo certified LDV plates. The Echo then serially spotted 100% DMSO stock solutions into source dose response plates to generate a 4-fold dilution scheme. 100% DMSO was added to the spotted dose response plates to bring the final volume to 5 µl. 300 nl of the dose response stock plate was then spotted into pre-incubation and stimulation assay plates. 50 µl of pre-incubation buffer and 100µl of stimulation buffer was then added to the plates resulting in a final assay test concentration range of 30 µM to 0.0001 µM with a final DMSO concentration of 0.3%.

Human ICLN-1633 cells expressing were plated onto 384 well, black PDL-coated microplates and maintained in TRPC5 growth media the day prior to use for experiments. TRPC5 expression was induced by the application of 1 µg/mL tetracycline at the time of plating. Media is removed from the plates and 10µl of 4µM of Fluo-4 AM (mixed with equal volume of Pluronic F-127) in EBSS is added to the cells. Cells are incubated at room temperature, protected from light, for 60-90 minutes. After the incubation period, the dye is removed and replaced with 10µl of EBSS. Cell, pre-incubation and stimulation plates are loaded onto the FLIPR-II and the assay is initiated. The FLIPR measures a 10 second baseline and then adds 10µl of 2X compounds (or controls). Changes in fluorescence are monitored for an additional 5 minutes. After the 5 minute pre-incubation, 20 µl of 2X Riluzole (with 1X compound or controls) is added to the cell plate. The final Riluzole stimulation concentration in the assay is 30µM. After the Riluzole addition, changes in fluorescence are monitored for an additional 5 minutes.

Compound modulation of TRPC5 calcium response was determined as follows. After the Englerin A, fluorescence was monitored for a 5-minute period. The maximum relative fluorescence response (minus the control response of 1 µM of an internal control compound known to maximally block TRPC5 calcium response, the "REF INHIB" in the formula below) was captured and exported from the FLIPR.

Compound effect is calculated as % inhibition using the following formula: $\begin{matrix}% inhibition \\ = \frac{RFU TEST AGENT - Plate Average RFU REF INHIB}{Plate Average RFU CONTROL - Plate Average RFU REF INHIB} x 100\end{matrix}$ wherein "RFU" is the relative fluorescent units.

The results of these assays are shown in Table 2, below, wherein "A" indicates an IC₅₀ of less than or equal to 50 nM; "B" an IC₅₀ of greater than 50 nM and less than or equal to 500 nM; "C" an IC₅₀ of greater than 500 nM and less than 1µM; "D" an IC₅₀ of 1 µM or greater; and "NT" indicates that the compound was not tested.

### Example 2. Urinary Rac1 analysis in healthy volunteers, DN, FSGS, PKD and Alport syndrome patients

The aim of this study was to measure the amount of Rac1 protein in the urine of healthy volunteers and patients with diabetic nephropathy ("DN"), FSGS, polycystic kidney disease ("PKD") and Alport syndrome.

Urine samples were obtained from healthy volunteers, and from DN, FSGS, PKD and Alport patients. Samples were concentrated using a Pierce protein concentrator with 10 kDa MWCO (Cat# 88516, Thermo Scientific, USA) and spun at 6000 x g for 30 min at 4 °C using rotor JA 14.50 (Beckman Coulter, USA) in an AVANTI-JE centrifuge, (Beckman Coulter). A second centrifugation at 6000 x g for 30 min at 4 °C was performed for samples that were concentrated to a volume that was >1 mL, to achieve a volume of <1 mL for all samples. The concentrated urine was collected and stored in -80 °C. Next, the samples were analyzed for urinary Rac1 by ELISA (Cat# abx253084, Abbexa Ltd, UK) following standard procedures according to the manufacturer's instructions.

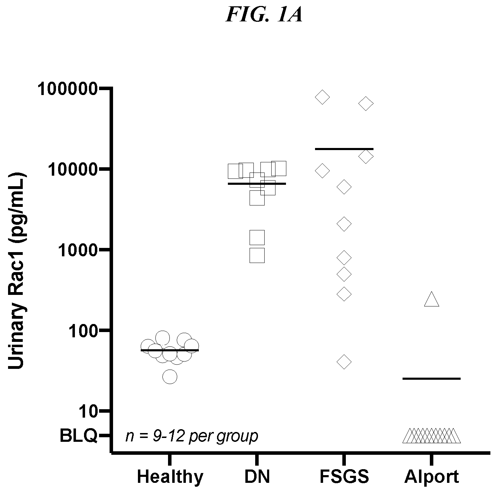

As shown in Fig. 1A, Rac1 level in the urine of healthy human subjects was 56.4 ± 15.7 pg/mL, Rac1 level in the urine of DN patients was 6600.0 ± 3677.1 pg/mL, Rac1 level in the urine of FSGS patients was 19,610.4 ± 30,070.6 pg/mL, and Rac1 level in the urine of Alport syndrome patients was 20.6 ± 71.4 pg/mL (all results are mean ± standard deviation). The lower limit of quantitation for the assay is approximately 10 pg/mL with starting urine volumes (prior to concentration) of >3 mL. Limited starting volumes of Alport patient urine samples were available, resulting in a lower limit of quantitation of approximately 100 pg/mL most samples were below the limit of quantitation (BLQ). On an input volume-adjusted bases, Alport patients have Rac1 levels comparable to healthy subjects.

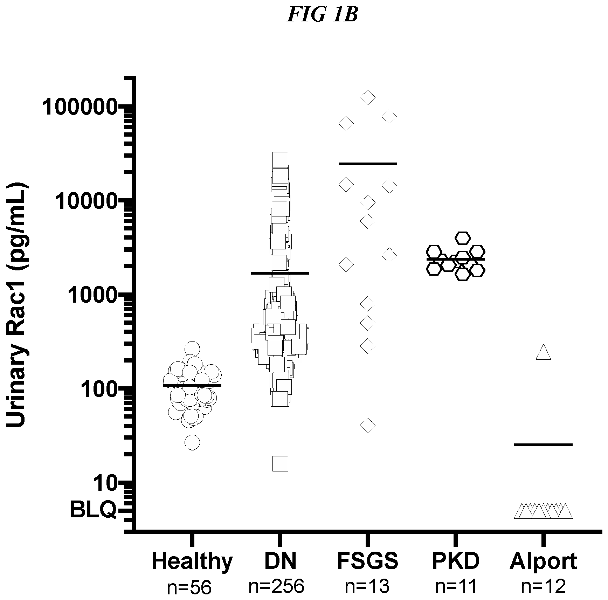

When Rac1 levels in additional healthy, DN and FSGS patients, as well as PKD patients were included in the assay, Rac1 level in the urine of healthy human subjects was determined to be 107.0 ± 44.6 pg/mL, Rac1 level in the urine of DN patients was 1,692.9 ± 3,365.8 pg/mL pg/mL, Rac1 level in the urine of FSGS patients was 24,525.9 ± 39,369.2 pg/mL, and Rac1 level in the urine of PKD patients was 2379.0 ± 654.4 pg/mL (see FIG. 1B).

### Example 3. Urinary Rac1 analysis in naïve rats following treatment with Compound 1

The aim of this study was to measure the amount of Rac1 protein in the urine of healthy rats treated with Compound 1.

Six to seven weeks old Sprague Dawley rats were placed in metabolic cage housing for urine collection. Following two 24-hour periods of pre-dose urine collection, received one daily dose of Compound 1 administered by oral gavage at 10 mg/kg for 7 days; control animals were administered vehicle. Urine was collected for 24-hour periods beginning on the day of dosing inception and on days 3 and 6 of dosing. No adverse effects were observed in the animals administered Compound 1.

Urine samples were concentrated as follows: 20 mL of urine was spun at 1500 x g for 5 min to remove cell debris. Samples were concentrated using a Pierce protein concentrator with 10 kDa MWCO (Cat# 88516, Thermo Scientific, USA) and spun at 6000 x g for 30 min at 4 °C using rotor JA 14.50 (Beckman Coulter, USA) in an AVANTI-JE centrifuge, (Beckman Coulter). A second centrifugation at 6000 x g for 30 min at 4 °C was performed for samples that were concentrated to a volume that was >1 mL, to achieve a volume of <1 mL for all samples. The concentrated urine was collected and stored in -80 °C. Next, the samples were analyzed for urinary Rac1 by ELISA (Cat# abx253084, Abbexa Ltd, UK) and urinary creatinine by ELISA (Cat# ab65340, Abcam, USA) following standard procedures according to the manufacturer's instructions. The amount of Rac1 in the urine was normalized to the amount of creatinine in the urine to control for the volume of urine produced.

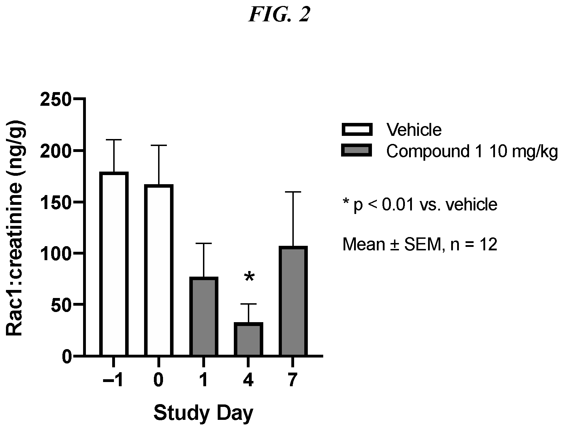

As shown in Fig. 2, Compound 1 reduced urinary Rac1 levels and the decrease reached significance at day 4, compared to pre-dose levels of urinary Rac1 (p value <0.01).

### Example 4. Urinary Rac1 analysis in DOCA-salt hypertensive rats following treatment with Compound 1

The aim of this study was to measure the amount of Rac1 protein in the urine of DOCA-salt hypertensive rats treated with Compound 1.

The DOCA-salt hypertensive rat model is a well-established model of mineralocorticoid hypertension with renal dysfunction leading to an FSGS phenotype, characterized by increased levels of urinary protein and albumin excretion. [Schenk et al., "The pathogenesis of DOCA-salt hypertension," J. Pharmacol. Toxicol. Methods (May 1992) 27(3):161-170; Gomez-Sanchez et al., "Mineralocorticoids, salt and high blood pressure," Steroids (1996) 61:184-188.]

Six to seven weeks old Sprague Dawley rats were unilaterally nephrectomized. After one-week recovery, rats were implanted with a DOCA pellet (45 mg) and provided tap water containing 0.9% NaCl and 0.2% KCl (Day 1) for a 4 weeks treatment. On Day 21, rats received one daily dose of Compound 1 administered by oral gavage at 10 mg/kg for 7 days. Body weight was recorded daily throughout the study. No adverse effects were observed in the animals administered Compound 1. Urine was collected for 24-hour periods beginning on day 17, day 20, day 24 and day 27, and urine protein and albumin were measured using standard methods.

For Rac1 analysis, urine samples were concentrated as follows: 20 mL of urine was spun at 1500 x g for 5 min to remove cell debris. Samples were concentrated using a Pierce protein concentrator with 10 kDa MWCO (Cat# 88516, Thermo Scientific, USA) and spun at 6000 x g for 30 min at 4 °C using rotor JA 14.50 (Beckman Coulter, USA) in an AVANTI-JE centrifuge, (Beckman Coulter). A second centrifugation at 6000 x g for 30 min at 4 °C was performed for samples that were concentrated to a volume that was >1 mL, to achieve a volume of <1 mL for all samples. The concentrated urine was collected and stored in -80 °C. Next, the samples were analyzed for urinary Rac1 by ELISA (Cat# abx253084, Abbexa Ltd, UK) and urinary creatinine by ELISA (Cat# ab65340, Abcam, USA) following standard procedures according to the manufacturer's instructions. The amount of Rac1 in the urine was normalized to the amount of creatinine in the urine to control for the volume of urine produced.

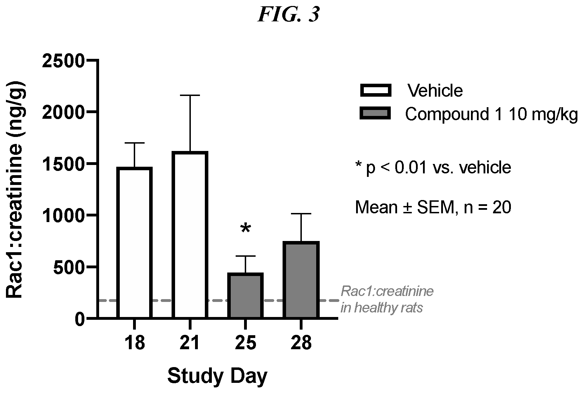

As shown in Fig. 3, following inception of dosing at day 21, Compound 1 reduced urinary Rac1 levels and the decrease reached significance day 25, compared to pre-dose levels of urinary Rac1 (p value <0.01).

### Example 5. Urinary Rac1 analysis in healthy human subjects following treatment with Compound 1

The aim of this study was to measure the amount of Rac1 protein in the urine of healthy human subjects treated with Compound 1.

Healthy human subjects enrolled in a Phase 1 clinical study, "A First-In-Human, Phase 1, Placebo-Controlled Study to Evaluate the Safety, Tolerability, and Pharmacokinetics of Compound 1, a TRPC5 Channel Inhibitor, in Healthy Subjects and Subjects With Renal Impairment," (NCT03970122) were administered a single, oral dose of either a placebo or 20 mg Compound 1 as a tablet. A urine sample was collected prior to administration of drug, and urine pools were then collected from 0-4 hours, 4-8 hours, 8-12 hours, 12-24 hours, 24-48 hours and 48-72 hours after dosing.

For Rac1 analysis, urine samples were concentrated as follows: 20 mL of urine was spun at 1500 x g for 5 min to remove cell debris. Samples were concentrated using a Pierce protein concentrator with 10 kDa MWCO (Cat# 88516, Thermo Scientific, USA) and spun at 6000 x g for 30 min at 4 °C using rotor JA 14.50 (Beckman Coulter, USA) in an AVANTI-JE centrifuge, (Beckman Coulter). A second centrifugation at 6000 x g for 30 min at 4 °C was performed for samples that were concentrated to a volume that was >1 mL, to achieve a volume of <1 mL for all samples. The concentrated urine was collected and stored in -80 °C. Next, the samples were analyzed for urinary Rac1 by ELISA (Cat# abx253084, Abbexa Ltd, UK) and urinary creatinine by ELISA (Cat# ab65340, Abcam, USA) following standard procedures according to the manufacturer's instructions. The amount of Rac1 in the urine was normalized to the amount of creatinine in the urine to control for the volume of urine produced.

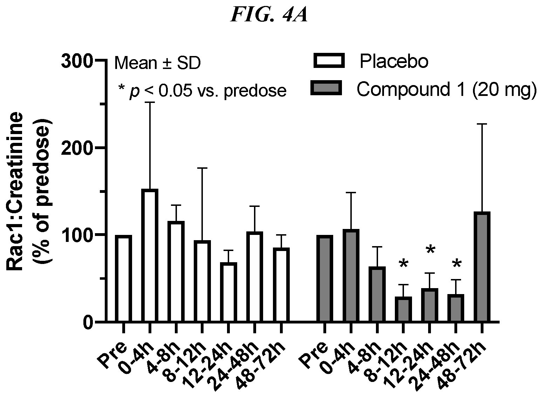

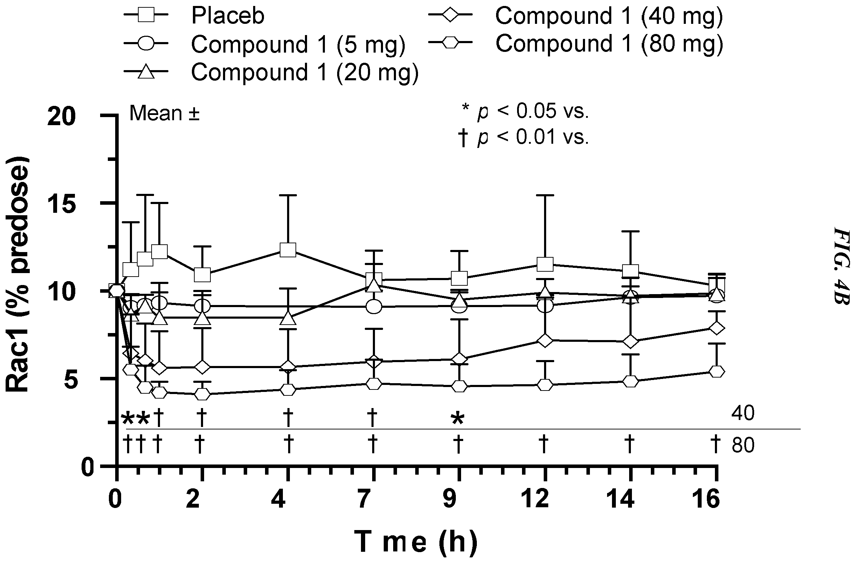

As shown in Fig. 4, Compound 1 reduced urinary Rac1 levels and the decrease reached significance by 8-12 hours after dosing, compared to pre-dose levels of urinary Rac1 (p value <0.05).

Additional data was obtained from human subjects that were administered a single, oral dose of either a placebo, 5 mg of Compound 1 as a liquid suspension or 20, 40 or 80 mg of Compound 1 as tablets. A urine sample was collected prior to administration of drug, and urine pools were then collected from 0-4 hours, 4-8 hours, 8-12 hours, 12-24 hours, and for each 24 hour period from day 2 through day 7 after dosing. Each dose level comprised 2 placebo and 8 treated subjects. These results are shown in FIG 4B.

As shown in Fig. 4B, Compound 1 reduced urinary Rac1 levels and the decrease reached significance by 8-12 hours after dosing with the 40 mg and 80 mg doses, compared to pre-dose levels of urinary Rac1 (p value <0.05). Urinary Rac1 levels remained reduced for up to 4 days with a single 40 mg dose and at least 7 days with a single 80 mg dose, consistent with maintained plasma concentrations based on pharmacokinetic analysis.

### Example 6. Rac1 is found in extracellular vesicles in the urine of healthy human subjects

The aim of this study was to determine whether Rac1 protein is found as a soluble protein in urine or is contained in extracellular vesicles.

Extracellular vesicles are cell-derived, membrane-bound particles that play important roles in intercellular communication [Ståhl et al., "Exosomes and microvesicles in normal physiology, pathophysiology, and renal diseases," Pediatr. Nephrol. (2019) 34: 11-30].

Healthy human subjects enrolled in a Phase 1 clinical study, "A First-In-Human, Phase 1, Placebo-Controlled Study to Evaluate the Safety, Tolerability, and Pharmacokinetics of Compound 1, a TRPC5 Channel Inhibitor, in Healthy Subjects and Subjects With Renal Impairment," (NCT03970122) were administered a single, oral dose of either a placebo or 20 mg Compound 1 as a tablet. A urine sample was collected prior to administration of drug, and urine pools were then collected from 0-4 hours, 4-8 hours, 8-12 hours, 12-24 hours, 24-48 hours and 48-72 hours after dosing.

For Rac1 analysis, urine samples were concentrated as follows: 20 mL of urine was spun at 1500 x g for 5 min to remove cell debris. Samples were concentrated using a Pierce protein concentrator with 10 kDa MWCO (Cat# 88516, Thermo Scientific, USA) and spun at 6000 x g for 30 min at 4 °C using rotor JA 14.50 (Beckman Coulter, USA) in an AVANTI-JE centrifuge, (Beckman Coulter) to achieve a volume of 1-1.5 mL for all samples. 1 mL of concentrated urine was centrifuged at 120,000 x g for 16 hours at 4 °C (Sorvall mx120+ ultracentrifuge, Rotor Type S120-AT2, ThermoFisher, USA) to pellet the extracellular vesicles. A fixed angle rotor was chosen for better pelleting efficiency due to its lower K-factor. Supernatant and pellet were collected and analyzed for Rac1 by ELISA (Cat# abx253084, Abbexa Ltd, UK) following standard procedures according to the manufacturer's instructions.

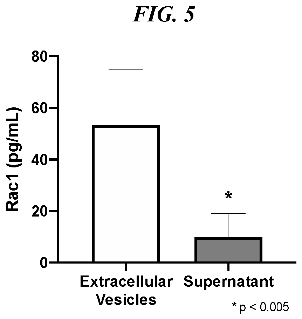

As shown in Fig. 5, the majority of urinary Rac1 is found in the extracellular vesicle pellet, with Rac1 levels significantly higher than in the supernatant (p value <0.005).

### Example 7. Urinary Rac1-GTP analysis in human subjects

The aim of this study is to measure the amount of active Rac1 protein (Rac1-GTP) in the urine of healthy human subjects and patients with kidney disease.

Rac1-GTP is the active form of Rac1, and Rac1 localizes to the cell membrane upon activation [Garcia-Mata et al., "The invisible hand: regulation of RHO GTPases by RHOGDIs," Nat. Rev. Mol. Cell Biol. (2011) 12: 493-504]. The membrane localization of Rac1 is consistent with the presence of Rac1 in extracellular vesicles.

Urine samples are concentrated as follows: 20 mL of urine is spun at 1500 x g for 5 min to remove cell debris. Samples are concentrated using a Pierce protein concentrator with 10 kDa MWCO (Cat# 88516, Thermo Scientific, USA) and spun at 6000 x g for 30 min at 4 °C using rotor JA 14.50 (Beckman Coulter, USA) in an AVANTI-JE centrifuge, (Beckman Coulter). A second centrifugation at 6000 x g for 30 min at 4 °C is performed for samples that are concentrated to a volume that is >1 mL, to achieve a volume of <1 mL for all samples. The concentrated urine is collected and stored in -80 °C. Next, the samples are analyzed for urinary Rac1-GTP by G-LISA (Cat# BK128, Cytoskeleton, USA) and urinary creatinine by ELISA (Cat# ab65340, Abcam, USA) following standard procedures according to the manufacturer's instructions. The amount of Rac1-GTP in the urine is normalized to the amount of creatinine in the urine to control for the volume of urine produced.

### Example 8. Urinary phospho-LIMK1 analysis in human subjects

The aim of this study is to measure the amount of phospho-LIMK1 in the urine of healthy human subjects and patients with kidney disease.

Urine samples are concentrated as follows: 20 mL of urine is spun at 1500 x g for 5 min to remove cell debris. Samples are concentrated using a Pierce protein concentrator with 10 kDa MWCO (Cat# 88516, Thermo Scientific, USA) and spun at 6000 x g for 30 min at 4 °C using rotor JA 14.50 (Beckman Coulter, USA) in an AVANTI-JE centrifuge, (Beckman Coulter). A second centrifugation at 6000 x g for 30 min at 4 °C is performed for samples that are concentrated to a volume that is >1 mL, to achieve a volume of <1 mL for all samples. The concentrated urine is collected and stored in -80 °C. Next, the samples are analyzed for urinary phospho-LIMK1 by ELISA (Cat# 3842S, Cell Signaling Technologies) and urinary creatinine by ELISA (Cat# ab65340, Abcam, USA) following standard procedures according to the manufacturer's instructions. The amount of phospho-LIMK1 in the urine is normalized to the amount of creatinine in the urine to control for the volume of urine produced.

Phospho-LIMK1 is also be assessed by immunoblotting. Concentrated urine is lysed by 1x RIPA lysis buffer (Cat #20-188, EMD Millipore, USA) with protease inhibitor cocktail (Cat# P8340, Sigma, USA) and run on an SDS-polyacrylamide gel, transferred onto polyvinylidene difluoride membrane and immunoblotted with a primary antibody against phospho-LIMK1 (Cat# 3842S, Cell Signaling Technologies) according to standard procedures.

### Example 9. Urinary phospho-cofilin analysis in human subjects

The aim of this study is to measure the amount of phospho-cofilin in the urine of healthy human subjects and patients with kidney disease.

Urine samples are concentrated as follows: 20 mL of urine is spun at 1500 x g for 5 min to remove cell debris. Samples are concentrated using a Pierce protein concentrator with 10 kDa MWCO (Cat# 88516, Thermo Scientific, USA) and spun at 6000 x g for 30 min at 4 °C using rotor JA 14.50 (Beckman Coulter, USA) in an AVANTI-JE centrifuge, (Beckman Coulter). A second centrifugation at 6000 x g for 30 min at 4 °C is performed for samples that are concentrated to a volume that is >1 mL, to achieve a volume of <1 mL for all samples. The concentrated urine is collected and stored in -80 °C. Next, the samples are analyzed for urinary phospho-cofilin by ELISA (Cat# 3318S, Cell Signaling Technologies) and urinary creatinine by ELISA (Cat# ab65340, Abcam, USA) following standard procedures according to the manufacturer's instructions. The amount of phospho-cofilin in the urine is normalized to the amount of creatinine in the urine to control for the volume of urine produced.

Phospho-cofilin is also assessed by immunoblotting. Concentrated urine is lysed by 1x RIPA lysis buffer (Cat #20-188, EMD Millipore, USA) with protease inhibitor cocktail (Cat# P8340, Sigma, USA) and run on an SDS-polyacrylamide gel, transferred onto polyvinylidene difluoride membrane and immunoblotted with a primary antibody against phospho-cofilin (Cat# 3318S, Cell Signaling Technologies) according to standard procedures.

### Example 10. Effect of Compound 1 in the ZDSD Model of Diabetic Nephropathy

The aim of this study was to evaluate the effects of the TRCP5 inhibitor, Compound 1, to attenuate the development and/or progression of albuminuria in ZDSD model of diabetic nephropathy (DN).

The ZDSD model is an established model which recapitulates the major features of diabetes including impaired glucose metabolism, neuropathy, retinopathy and nephropathy [Peterson et al., "Characterization of the ZDSD Rat: A Translational Model for the Study of Metabolic Syndrome and Type 2 Diabetes," J. Diabetes. Res. (2015), Article ID 487816, 10 pages; Peterson et al., "The ZDSD rat: a novel model of diabetic nephropathy," Am. J. Transl. Res. (2017) 9: 4236-4249].

Male ZDSD rats (Crown Bioscience, Indianapolis, IN., *n* = 79) were maintained on standard rodent chow (Purina 5008) from weaning to 15 weeks of age. A diabetogenic diet (Research Diet D124668) was initiated and maintained for three weeks to synchronize development of hyperglycemia. Diabetogenic diet was replaced with Purina 5008 for remainder of the study. Animals were housed two per cage and maintained on a 12 hour light cycle (0600-1800). Room temperature was monitored daily and maintained at 70-74°F. Food and water were provided *ad libitum* for duration of the study.

Hyperglycemic ZDSD rats were selected for study, randomized by body weight into groups of ten and assigned to receive vehicle or Compound 1 (3 or 10 mg/kg/d). All compounds were administered by oral gavage daily (6-8 am) for 12 weeks. Dose volume was maintained at 5 mL/kg.

Body weight was recorded weekly. Food consumption was recorded weekly during the treatment phase from week 0 through 12. Blood samples were obtained from the tail vein three hours following dosing and every two weeks until week 6, then weekly thereafter from week 8 to 11. Whole blood was processed to serum for measurement of BUN, creatinine, albumin, total protein (AU480).

Twenty-four-hour urine samples were collected at baseline, then every two weeks until week six, then weekly thereafter. Samples were collected at room temperature and without additives. Food and water were provided *ad libitum* during the collection period. Urine total protein (AU480), and albumin (ICL kit # E-25AL) were assayed. Animals were terminated after 12 weeks of treatment using CO₂ asphyxiation and cervical dislocation.

Animals receiving Compound 1 at 3 mg/kg and 10 mg/kg demonstrated an increase in body weight compared to animals in vehicle group during the last two weeks of study.

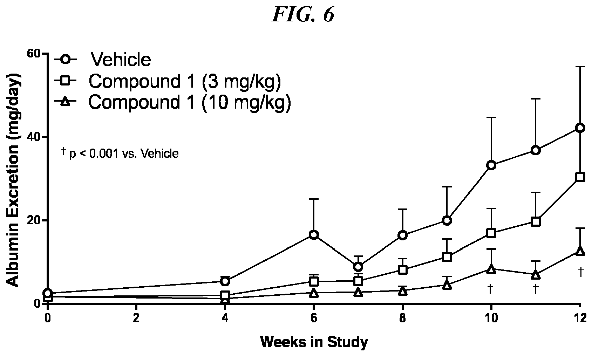

As shown in Fig. 6, Compound 1 attenuated urinary albumin excretion from week 6 to week 12 and the decrease reached significance at weeks 10 to 12, compared to vehicle control rats (p value <0.001).

### Example 11. Effects of Compound 1 in DOCA-salt hypertensive rats

The aim of this study was to evaluate the effects of the TRCP5 inhibitor, Compound 1, to attenuate the development and/or progression of albuminuria in deoxycorticosterone acetate (DOCA)-salt hypertensive rats.

The DOCA-salt hypertensive rat model is a well-established model of mineralocorticoid hypertension with renal dysfunction leading to an FSGS phenotype, characterized by increased levels of urinary protein and albumin excretion. [Schenk et al., "The pathogenesis of DOCA-salt hypertension," J. Pharmacol. Toxicol. Methods (May 1992) 27(3):161-170; Gomez-Sanchez et al., "Mineralocorticoids, salt and high blood pressure," Steroids (1996) 61:184-188.]

Six to seven weeks old Sprague Dawley rats were unilaterally nephrectomized; after one-week recovery, rats were implanted with a DOCA pellet (45 mg) and provided tap water containing 0.9% NaCl and 0.2% KCl (Day 1) for a 4 weeks treatment. On Day 1, DOCA-salt rats received one daily dose of Compound 1 administered by oral gavage at 3 mg/kg or 10 mg/kg for 4 weeks; control animals for DOCA treatment were administered vehicle. Sham animals, implanted with a silicone-water pellet, were given tap water and received oral administration of the vehicle. Body weight was recorded daily and proteinuria, albuminuria and arterial blood pressure were recorded every week.

No adverse effects were observed in the animals administered Compound 1. There was no significant difference in body weight and urinary creatinine excretion in rats treated with DOCA or DOCA and Compound 1. Animals receiving DOCA or DOCA and Compound 1 had elevated mean arterial blood pressure (BP), diastolic and systolic BP, compared to sham animals, from week 1 to 4.

Water intake and urine volume produced per day were also elevated in animals receiving DOCA-salt treatment followed by vehicle or Compound 1.

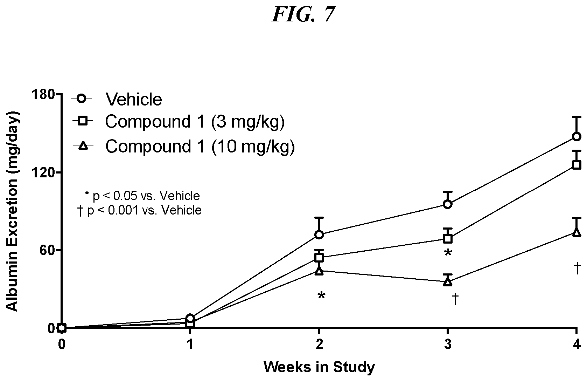

As shown in Fig. 7, Compound 1 at 10 mg/kg attenuated urinary albumin excretion from week 2 to week 4 and the decrease reached significance at week 2, compared to DOCA-vehicle control rats (p value <0.05), and at weeks 3 and 4 (p value <0.001). Compound 1 at 3 mg/kg attenuated urinary albumin excretion from week 2 to week 4 and the decrease was significance at week 3, compared to DOCA-vehicle control rats (p value <0.05).

### Example 12. Effects of Compound 1 in COL4A4 knockout mice

The aim of this study was to evaluate the effects of the TRCP5 inhibitor, Compound 1, to attenuate the development and/or progression of albuminuria in *COL4A3* knockout mice.

The *COL4A4* knockout mouse model is a well-established model of Alport disease, characterized by increased levels of urinary protein and albumin excretion. [Korstanje et al., "A mouse Col4a4 mutation causing Alport glomerulosclerosis with abnormal collagen α3α4α5(IV) trimers," Kidney Int. (2014) 85:1461-1468].

Four to five weeks old *COL4A4* knockout mice were received one daily dose of Compound 1 administered by oral gavage at 3 mg/kg or 10 mg/kg for 4 weeks; control animals were administered vehicle. Body weight was recorded daily and urinary protein and creatinine were recorded every week, and the ratio of urine protein to creatinine (UPCR) was calculated.

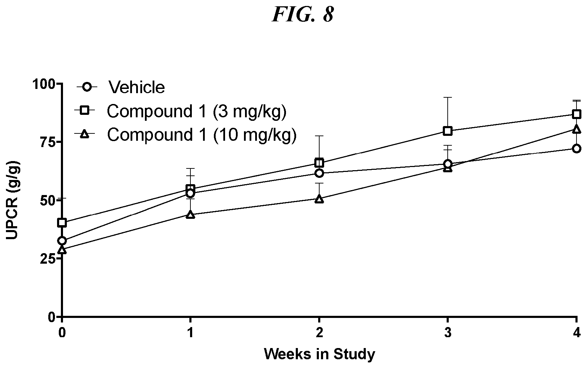

As shown in Fig. 8, Compound 1 at 3 mg/kg or 10 mg/kg had no effect on the urinary protein to creatinine ratio.

### Example 13. Effects of Compound 2 in DOCA-salt hypertensive rats

The aim of this study was to evaluate the effects of the TRCP5 inhibitor, Compound 2, to attenuate the development and/or progression of albuminuria in deoxycorticosterone acetate (DOCA)-salt hypertensive rats.

The DOCA-salt hypertensive rat model is a well-established model of mineralocorticoid hypertension with renal dysfunction leading to an FSGS phenotype, characterized by increased levels of urinary protein and albumin excretion. [Schenk et al., "The pathogenesis of DOCA-salt hypertension," J. Pharmacol. Toxicol. Methods (May 1992) 27(3):161-170; Gomez-Sanchez et al., "Mineralocorticoids, salt and high blood pressure," Steroids (1996) 61:184-188.]

Six to seven weeks old Sprague Dawley rats were unilaterally nephrectomized; after one-week recovery, rats were implanted with a DOCA pellet (45 mg) and provided tap water containing 0.9% NaCl and 0.2% KCl (Day 1) for a 4 weeks treatment. On Day 1, DOCA-salt rats received one daily dose of Compound 2 administered by subcutaneous (SC) injection at 10 mg/kg for 4 weeks or at 60 mg/kg for one week followed by 100 mg/kg for three weeks; control animals for DOCA treatment were administered vehicle. Sham animals, implanted with a silicone-water pellet, were given tap water and received SC administration of the vehicle. Body weight was recorded daily and proteinuria, albuminuria and arterial blood pressure were recorded every week.

No adverse effects were observed in the animals administered Compound 2. There was no significant difference in body weight and urinary creatinine excretion in rats treated with DOCA or DOCA and Compound 2. Animals receiving DOCA or DOCA and Compound 2 had elevated mean arterial blood pressure (BP), diastolic and systolic BP, compared to sham animals, from week 1 to 4.

Water intake and urine volume produced per day were also elevated in animals receiving DOCA-salt treatment with vehicle or Compound 2.

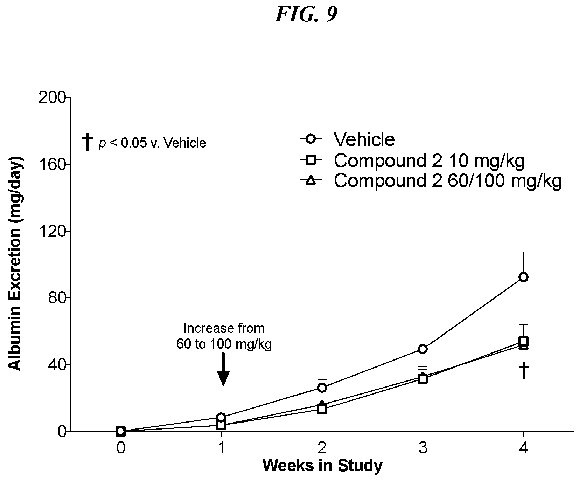

As shown in Fig. 9, Compound 2 at 10 mg/kg and 60/100 mg/kg attenuated urinary albumin excretion from week 2 to week 4 and the decrease reached significance at week 4, compared to DOCA-vehicle control rats (p value <0.05).

### Example 14. Effects of Compound 3 in DOCA-salt hypertensive rats.

The aim of this study was to evaluate the effects of the TRCP5 inhibitor, Compound 3, to attenuate the development and/or progression of albuminuria in deoxycorticosterone acetate (DOCA)-salt hypertensive rats.

The DOCA-salt hypertensive rat model is a well-established model of mineralocorticoid hypertension with renal dysfunction leading to an FSGS phenotype, characterized by increased levels of urinary protein and albumin excretion. [Schenk et al., "The pathogenesis of DOCA-salt hypertension," J. Pharmacol. Toxicol. Methods (May 1992) 27(3):161-170; Gomez-Sanchez et al., "Mineralocorticoids, salt and high blood pressure," Steroids (1996) 61:184-188.]

Six to seven weeks old Sprague Dawley rats were unilaterally nephrectomized; after one-week recovery, rats were implanted with a DOCA pellet (45 mg) and provided tap water containing 0.9% NaCl and 0.2% KCl (Day 1) for a 4 weeks treatment. On Day 1, DOCA-salt rats received one daily dose of Compound 3 administered by oral gavage at 30 mg/kg for 4 weeks; control animals for DOCA treatment were administered vehicle or the mineralocorticoid receptor antagonist (MCRA) eplerenone by twice-daily oral gavage at 50 mg/kg. Sham animals, implanted with a silicone-water pellet, were given tap water and received SC administration of the vehicle. Body weight was recorded daily and proteinuria, albuminuria and arterial blood pressure were recorded every week.

No adverse effects were observed in the animals administered Compound 3. There was no significant difference in body weight and urinary creatinine excretion in rats treated with DOCA or DOCA plus Compound 3 or eplerenone. Animals receiving DOCA, DOCA and Compound 3, or DOCA and eplerenone had elevated mean arterial blood pressure (BP), diastolic and systolic BP, compared to sham animals, from week 1 to 4. Water intake and urine volume produced per day were also elevated in animals receiving DOCA-salt treatment followed by vehicle, Compound 3 or eplerenone.

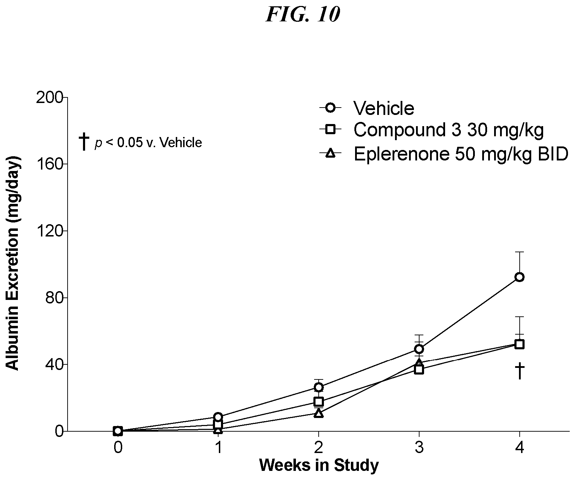

As shown in Fig. 10, Compound 3 at 30 mg/kg significantly attenuated urinary albumin excretion at week 4, compared to DOCA-vehicle control rats (p value <0.05). Eplerenone also significantly attenuated urinary albumin excretion at week 4, compared to DOCA-vehicle control rats (p value <0.05).

### Example 15. Effects of Compound 4 in DOCA-salt hypertensive rats.

The aim of this study was to evaluate the effects of the TRCP5 inhibitor, Compound 4, to attenuate the development and/or progression of albuminuria in deoxycorticosterone acetate (DOCA)-salt hypertensive rats.

The DOCA-salt hypertensive rat model is a well-established model of mineralocorticoid hypertension with renal dysfunction leading to an FSGS phenotype, characterized by increased levels of urinary protein and albumin excretion. [Schenk et al., "The pathogenesis of DOCA-salt hypertension," J. Pharmacol. Toxicol. Methods (May 1992) 27(3):161-170; Gomez-Sanchez et al., "Mineralocorticoids, salt and high blood pressure," Steroids (1996) 61:184-188.]

Six to seven weeks old Sprague Dawley rats were unilaterally nephrectomized; after one-week recovery, rats were implanted with a DOCA pellet (45 mg) and provided tap water containing 0.9% NaCl and 0.2% KCl (Day 1) for a 2 weeks treatment. On Day 1, DOCA-salt rats received one daily dose of Compound 4 administered by intraperitoneal (IP) injection at 20 mg/kg, 50 mg/kg or 100 mg/kg for 2 weeks; control animals for DOCA treatment were administered vehicle. Sham animals, implanted with a silicone-water pellet, were given tap water and received IP administration of the vehicle. Body weight was recorded daily and proteinuria, albuminuria and arterial blood pressure were recorded every week.

No adverse effects were observed in the animals administered Compound 4. There was no significant difference in body weight and urinary creatinine excretion in rats treated with DOCA or DOCA and Compound 4. Animals receiving DOCA or DOCA and Compound 4 had elevated mean arterial blood pressure (BP), diastolic and systolic BP, compared to sham animals, from week 1 to 2.

Water intake and urine volume produced per day were also elevated in animals receiving DOCA-salt treatment with vehicle or Compound 4.

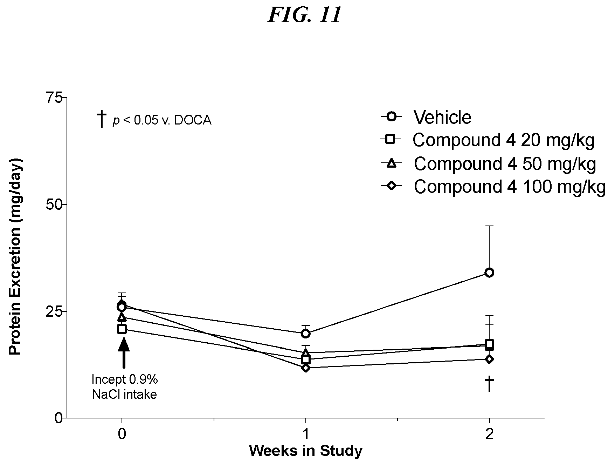

As shown in Fig. 11, Compound 4 at 20 mg/kg, 50 mg/kg and 100 mg/kg significantly attenuated urinary protein excretion at week 2, compared to DOCA-vehicle control rats (p value <0.05).

### Example 16. Effects of cyclosporine A and tacrolimus in DOCA-salt hypertensive rats

The aim of this study was to evaluate the effects of the calcineurin inhibitors, cyclosporine A and tacrolimus, to attenuate the development and/or progression of albuminuria in deoxycorticosterone acetate (DOCA)-salt hypertensive rats.

The DOCA-salt hypertensive rat model is a well-established model of mineralocorticoid hypertension with renal dysfunction leading to an FSGS phenotype, characterized by increased levels of urinary protein and albumin excretion. [Schenk et al., "The pathogenesis of DOCA-salt hypertension," J. Pharmacol. Toxicol. Methods (May 1992) 27(3):161-170; Gomez-Sanchez et al., "Mineralocorticoids, salt and high blood pressure," Steroids (1996) 61:184-188.]

Six to seven weeks old Sprague Dawley rats were unilaterally nephrectomized; after one-week recovery, rats were implanted with a DOCA pellet (45 mg) and provided tap water containing 0.9% NaCl and 0.2% KCl (Day 1) for a 3 weeks treatment. On Day 1, DOCA-salt rats received one daily dose of cyclosporine A by oral gavage at 3 mg/kg for 3 weeks or one daily dose of tacrolimus by oral gavage at 0.3 mg/kg for 2 weeks followed by 0.1 mg/kg for one week; control animals for DOCA treatment were administered vehicle. Sham animals, implanted with a silicone-water pellet, were given tap water and received administration of the vehicle. Proteinuria and albuminuria were recorded every week, and body weight was recorded daily.

No adverse effects were observed in the animals administered DOCA or DOCA and cyclosporine A. There was significant loss of body weight in rats treated with DOCA and tacrolimus, and the dose of tacrolimus was adjusted from 0.3 mg/kg down to 0.1 mg/kg after two weeks of dosing, which reversed the weight loss.

Water intake and urine volume produced per day were also elevated in animals receiving DOCA-salt treatment with vehicle, cyclosporine A or tacrolimus.

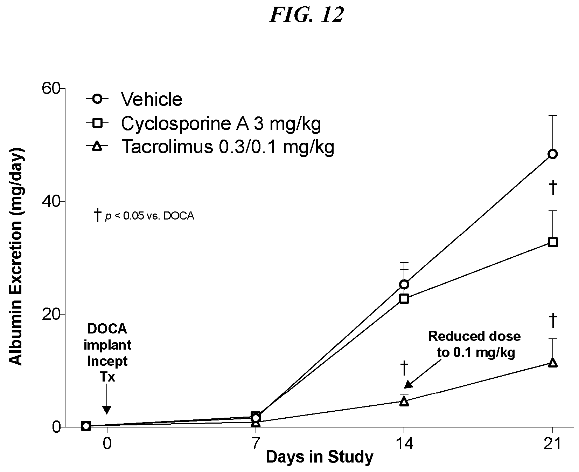

As shown in Fig. 12, cyclosporine A at 3 mg/kg significantly attenuated urinary albumin excretion at week 3, compared to DOCA-vehicle control rats (p value <0.05), and tacrolimus at 0.3/0.1 mg/mg significantly attenuated urinary albumin excretion at weeks 2 and 3, compared to DOCA-vehicle control rats (p value <0.05).

### Example 17. Urinary Rac1 analysis in COVID-19 Positive Patients with Acute Kidney Injury

The aim of this study was to measure the amount of Rac1 protein in the urine of patients with acute kidney injury ("AKI") that had tested positive for COVID-19 by PCR testing.

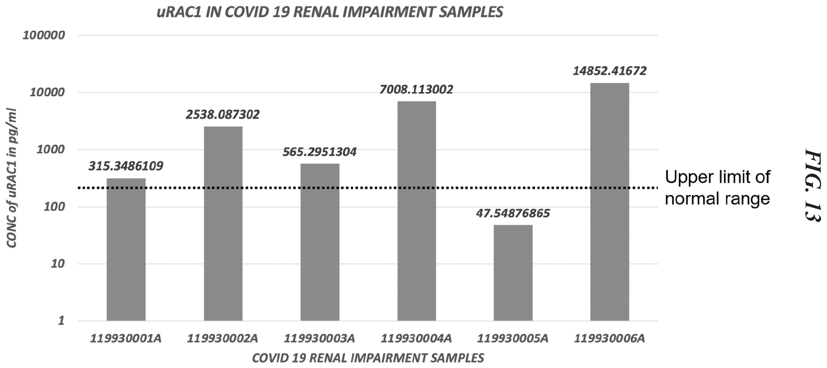

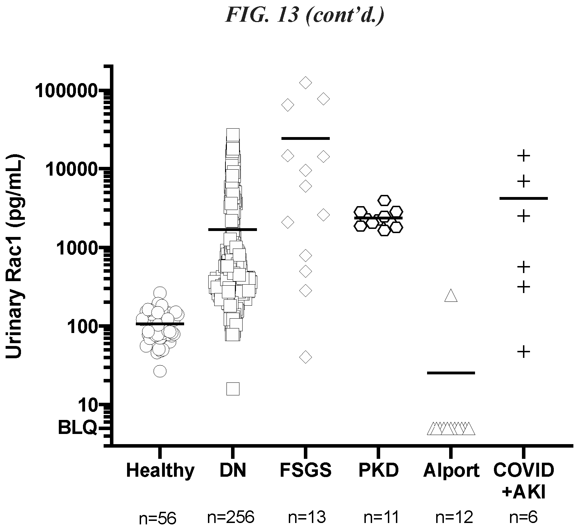

Urine samples from six patients having active AKI following a positive COVID-19 test were obtained, processed, and analyzed as described in Example 2. The mean Rac1 value for the six patients was 4221.13 ± 5825.17 pg/ml (as compared to 107.0 ± 44.6 pg/mL for normal patients). FIG. 13 shows that three of the six patients had Rac1 levels that were elevated by at least 8-fold over the ~300 pg/ml upper limit of normal patients. This suggests that a subset of COVID-19 patients with AKI will have sufficiently high urinary Rac1 concentrations (e.g., above a pre-determined threshold) to be treatable by the methods of this invention.

### INCORPORATION BY REFERENCE

All of the U.S. patents and U.S. and PCT published patent applications cited herein are hereby incorporated by reference.

### EQUIVALENTS

The foregoing written specification is sufficient to enable one skilled in the art to practice the invention. The present invention is not to be limited in scope by examples provided, since the examples are intended as a single illustration of one aspect of the invention and other functionally equivalent embodiments are within the scope of the invention. Various modifications of the invention in addition to those shown and described herein will become apparent to those skilled in the art from the foregoing description and fall within the scope of the appended claims. The advantages and objects of the invention are not necessarily encompassed by each embodiment of the invention.
In some embodiments, the invention relates to the following items:
1. A method of selecting and treating a human subject suffering from a kidney disease comprising the steps of:
   a. selecting the subject if a urinary level of one or more biomarkers selected from Rac1, Rac1-GTP, phospho-LIM kinase 1, and phospho-cofilin in the subject is above a pre-determined threshold; and
   b. administering to the selected subject a pharmaceutical composition comprising a TRPC5 inhibitor or a calcineurin inhibitor; and a pharmaceutically acceptable carrier.
2. A method of treating a human subject suffering from a kidney disease comprising the step of
   administering to the subject a pharmaceutical composition comprising
      a TRPC5 inhibitor or a calcineurin inhibitor, and
      a pharmaceutically acceptable carrier;
   only if the subject is determined to have a pre-treatment urinary level of one or more biomarkers selected from Rac1, Rac1-GTP, phospho-LIM kinase 1, and phospho-cofilin that is above a pre-determined threshold.
3. The method of item 1 or 2, wherein the TRPC5 inhibitor is:
   a. a compound of Formula (I) or Formula (II): 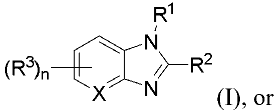 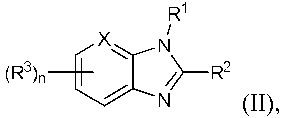 or a pharmaceutically acceptable salt of either of the foregoing, wherein:
      X is CH, C(R³), or N;
      R¹ is selected from the group consisting of H; alkyl; cycloalkyl; heterocycloalkyl; alkenyl; aryl; heteroaryl; alkylene-aryl; alkylene-heteroaryl; -CH₂(O)N(R)-heteroaryl; -CH₂(O)N(R)-alkyl; alkylene-N(alkyl)₂; heterocycloalkyl; alkylene-O-alkyl; alkylene-O-aryl; alkylene-N(R)- C(O)-aryl; alkylene-N(R)-C(O)-alkyl; alkylene-C(O)-N(R)-alkyl; alkylene-C(O)-N(R)-aryl; alkylene-C(O)-cycloalkyl; and alkylene-C(O)-N(R)-heteroaryl;
      R² is selected from the group consisting of H; NH₂, alkyl; cycloalkyl; aryl; heteroaryl; alkylene-aryl, alkylene-N(alkyl)₂; alkylene-heterocycloalkyl; alkylene-cycloalkyl; -N(R)-alkyl; - N(R)-aryl; -N(R)-alkylene-aryl; -N(R)-cycloalkyl; -N(R)-heterocycloalkyl; -O-aryl; alkylene-O-aryl; heterocycloalkyl; -N=C(R)-aryl; -N(R)-alkylene-heteroaryl; -N(R)-alkylene-OH; -S-alkylene-C(O)N(R)-aryl; -S-alkylene-C(O)N(R)-heteroaryl; alkylene-C(O)-heterocycloalkyl; alkylene-N(R)-alkyl; alkylene-N(R)-aryl; and -S-alkyl;
      R³ is independently selected from alkyl, halogen, -CN, -OMe, -OH, -NO₂, -NH₂, -N(Me)₂, -CF₃, -OCF₃, -CHF₂, -OCHF₂, and -O-alkylene-OH;
      R is H, or Me; and
      n is 0, 1, 2, 3, or 4;
   b. a compound of Formula (III), (IV), or (V): 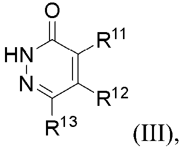 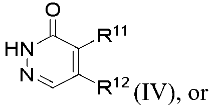 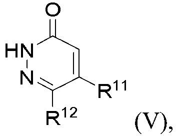 or a tautomer or a pharmaceutically acceptable salt of any of the foregoing, wherein:
      R¹¹ and R¹³ are independently selected from the group consisting of H, alkyl, alkenyl, alkynyl, aryl, heterocyclyl, heteroaryl, halogen, -OH, -CN, -cycloalkyl, -O-alkyl, -O-cycloalkyl, -O-aryl, -aryl-O-aryl -CF₃, -C(H)F₂, alkylene-CF₃, alkylene-C(H)F₂, -SO₂-alkyl, and -O-alkylene-O-alkyl, -heterocyclyl-L-R⁴, and -heteroaryl-L-R⁴;
      R¹² is -heterocyclyl-L-R¹⁴;
      R¹⁴ is absent or selected from the group consisting of alkyl, cycloalkyl, aryl, alkylene-aryl, alkylene-heteroaryl, heteroaryl, heterocyclyl, -C(O)N(R¹⁵)₂, and CF₃;
      R¹⁵ is independently H or alkyl;
      R¹⁶ is selected from the group consisting of alkyl, cycloalkyl, aryl, heterocyclyl, heteroaryl, alkylene-aryl, -C(O)N(R¹⁵)₂, and CF₃;
      L is absent or selected from the group consisting of methylene, -C(O)-, -SO₂-, - CH₂N(Me)-, -N(R¹⁵)(R¹⁶)-, -C(R¹⁵)(R¹⁶)-, and -O-R¹⁶; and
      one and only one of R¹¹, R¹², and R¹³ is -heterocyclyl-L-R¹⁴ or -heteroaryl-L-R¹⁴;
   c. a compound of Formula (VI) or (VII): 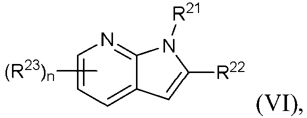 or 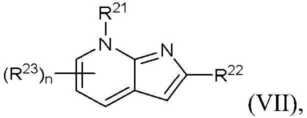 or a pharmaceutically acceptable salt thereof, wherein:
      R²¹ is selected from the group consisting of alkyl; cycloalkyl; heterocycloalkyl; aryl; heteroaryl; alkylene-aryl; alkylene-heteroaryl; alkylene-O-aryl; alkylene-N(alkyl)₂; alkylene-heterocycloalkyl; alkylene-cycloalkyl; -N(alkyl)₂; and -C(O)-aryl;
      R²² is selected from the group consisting of alkyl; cycloalkyl; heterocycloalkyl; aryl; heteroaryl; alkylene-N(alkyl)₂; alkylene-heterocycloalkyl; alkylene-cycloalkyl; alkylene-heterocycloalkyl; and alkylene-OR';
      R²³ is independently selected from alkyl, halogen, OMe, OH, N(Me)₂, CF₃, or OCF₃, -O- and alkylene-OH;
      R is H, or Me;
      R' is H, methyl, ethyl, or isopropyl; and
      n is 0, 1, 2, 3, or 4; or
   d. a compound of Formula (VIII) or (IX): 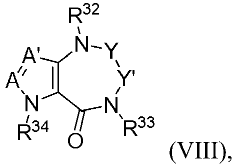 or 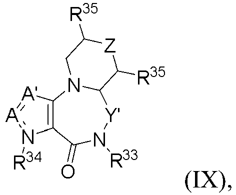 or a pharmaceutically acceptable salt of either of the foregoing, wherein:
      A and A' are independently selected from CR^{a} and N;
      R^{a} is L-R³¹;
      L is absent, CH₂, O, SO₂, or NR³²;
      R³¹ is selected from optionally substituted alkyl, optionally substituted aryl, and optionally substituted heteroaryl;
      each R³² is independently **H,** or alkyl;
      R³³ is selected from optionally substituted alkyl, optionally substituted alkylene-OR³², optionally substituted cycloalkylene-OR³², optionally substituted alkylene-N(R³⁷)₂, optionally substituted cycloalkylene-N(R³⁷)₂, optionally substituted alkylene-C(O)N(R³²)₂, optionally substituted cycloalkylene-C(O)N(R³²)₂, optionally substituted alkylene-S(O)₂N(R³²)₂, and optionally substituted cycloalkylene-S(O)₂N(R³²)₂;
      R³⁴ is selected from alkyl, optionally substituted alkylene-aryl, and optionally substituted alkylene-heteroaryl;
      each R³⁵ is independently selected from **H,** N(R³²)₂, OR³²;
      each R³⁷ is independently selected from **H,** alkyl, (alkyl)C(O)-, (aryl)C(O)-, (alkyl)S(O)₂-, and (aryl)S(O)₂-;
      Y is -C(O)-, CH₂, CHR³⁶, C(R³⁶)₂;
      each R³⁶ is independently selected from H, alkyl, and optionally substituted alkylene-OH;
      Y' is -C(O)-, CH₂, CHR^{33'}, C(R^{33'})₂, or Y' is taken together with R³³ to form a 5- or 6-membered ring;
      each R³³' is independently selected from optionally substituted alkyl, optionally substituted alkylene-OR³², optionally substituted cycloalkylene-OR³², optionally substituted alkylene-N(R³⁷)₂, optionally substituted cycloalkylene-N(R³⁷)₂, optionally substituted alkylene-C(O)N(R³²)₂, optionally substituted cycloalkylene-C(O)N(R³²)₂, optionally substituted alkyleneS(O)₂N(R³²)₂, and optionally substituted cycloalkylene-S(O)₂N(R³²)₂; and
      Z is absent, CH₂, CHR³⁵, O, -NR³²-, or -SO₂-;
      provided that Y and Y' are not both -C(O)-.
4. The method of item 3, wherein the TRPC5 inhibitor a compound of structural formula X: 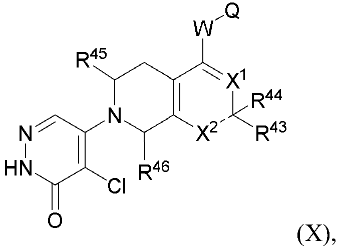 or a pharmaceutically acceptable salt thereof, wherein:
   "---" is a single bond or a double bond;
   X¹ is CH or N;
   when "---" is a double bond, X² is CH or N;
   when "---" is a single bond, X² is N(CH₃),
   when X¹ is CH, X² is N or N(CH₃);
   W is -O-, -N(CH₃)-, -N(CH₂CH₂OH)-, cyclopropan-1,1-diyl, or -CH(CH₃)-;
   Q is 2-trifluoromethyl-4-fluorophenyl, 2-difluoromethyl-4-fluorophenyl, 2-trifluoromethylphenyl, 2-methyl-4-fluorophenyl, 2-chloro-4-fluorophenyl, 2-chlorophenyl, 1-(benzyl)-4-methylpiperidin-3-yl, 4-trifluoromethylpyridin-3-yl, 2-trifluoromethyl-6-fluorophenyl, 2-trifluoromethyl-3-cyanophenyl, 2-ethyl-3-fluorophenyl, 2-chloro-3-cyanophenyl, 2-trifluoromethyl-5-fluorophenyl, or 2-difluoromethylphenyl;
   R⁴³ is hydrogen, -CH₂OH, -CH(OH)-CH₂OH, -NH₂, -CH(OH)CH₃, -OCH₃, or -NH-(CH₂)₂OH; and when "---" is a double bond, R⁴⁴ is absent;
   and when "---" is a single bond, R⁴³ and R⁴⁴ are taken together to form =O; and
   each of R⁴⁵ and R⁴⁶ is independently hydrogen or -CH₃.
5. The method of item 4, wherein the TRPC5 inhibitor is a compound of Formula XI: 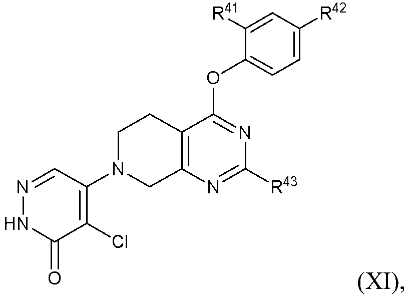 or a pharmaceutically acceptable salt thereof; wherein:
   R⁴¹ is chloro, -CF₃, -CHF₂, or -CH₃;
   R⁴² is hydrogen or fluoro; and
   R⁴³ is hydrogen, -NH₂, -CH₂OH, or CH(OH)-CH₂OH.
6. The method of item 5, wherein the TRPC5 inhibitor is: 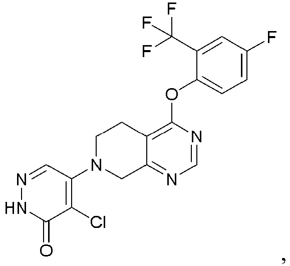 or a pharmaceutically acceptable salt thereof.
7. The method of item 1 or 2, wherein the TRPC5 inhibitor is 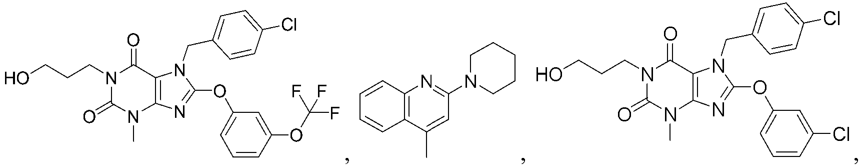 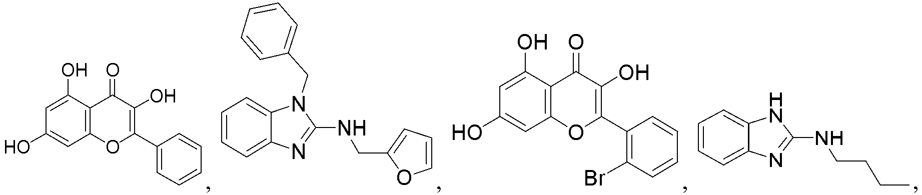 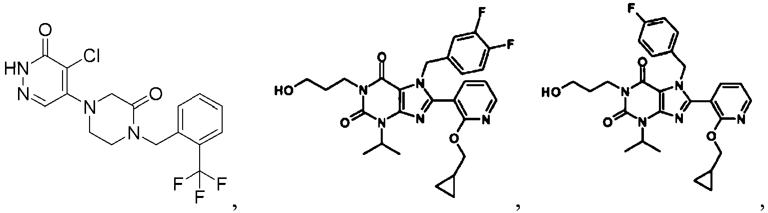 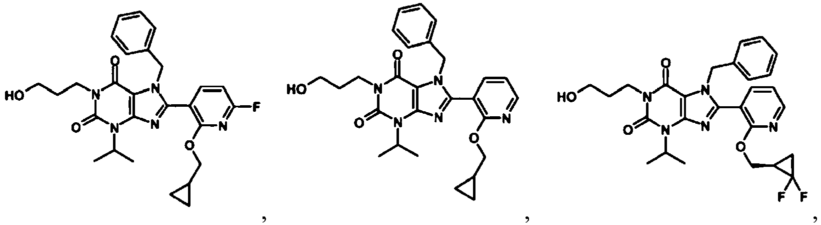 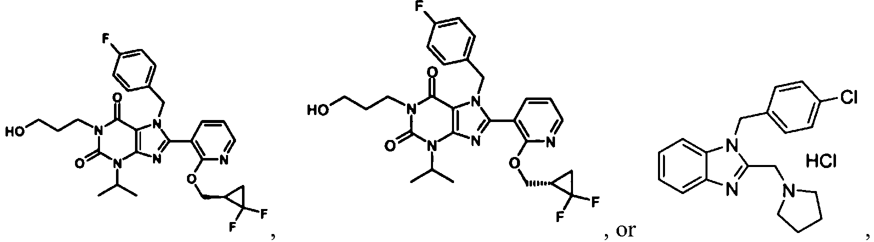 or a pharmaceutically acceptable salt thereof.
8. The method of item 1 or 2, wherein the calcineurin inhibitor is cyclosporin A, tacrolimus, or voclosporin, or a pharmaceutically acceptable salt thereof.
9. The method of any one of items 1-8, wherein the kidney disease is diabetic nephropathy, focal segmental glomerulosclerosis, minimal change disease, membranoproliferative glomerulonephritis (including post-streptococcal glomerulonephritis and bacterial endocarditis-associated glomerulonephritis), membranous nephropathy, other hepatitis C virus-associated glomerulopathies, HIV-associated glomerulopathies, COVID-19-associated acute kidney injury, Alport syndrome, polycystic kidney disease (both autosomal dominant and autosomal recessive), IgA nephropathy, other genetic nephropathies or ciliopathies (e.g., HNF1beta, nephronophthisis, autosomal dominant cystic/tubular kidney disease), lupus nephritis, Goodpasture's syndrome (anti-GBM disease), and another complement- or immune-mediated kidney disease.
10. The method of item 9, wherein the kidney disease is diabetic nephropathy, or focal segmental glomerulosclerosis.
11. The method of item 9, wherein the kidney disease is autosomal dominant polycystic kidney disease of autosomal recessive polycystic kidney disease.
12. The method of any one of items 1-11, wherein the subject is selected on the basis of having a urinary level of Rac1 above a pre-determined threshold.
13. The method of item 12, wherein the urinary Rac1 level in a subject is measured in a fraction of urine comprising extracellular vesicles.
14. The method of item 12 or 13, wherein the pre-determined threshold level is established by determining the range of urinary levels of the selected biomarker in a population of healthy humans; and establishing the pre-determined threshold level for the selected biomarker at a level above the 75^{th} percentile in the population.
15. The method of item 14, wherein the pre-determined threshold level at a level above the 90^{th} percentile in the population.
16. The method of item 15, wherein the pre-determined threshold level at a level above the 95^{th} percentile in the population.
17. The method of item 12 or 13, wherein the pre-determined threshold level for urinary Rac1 is between 100-500 pg/mL.
18. A method of determining the efficacy of a TRPC5 inhibitor therapy in a human subject suffering from a kidney disease, wherein prior to commencing the therapy the subject was determined to have a pre-treatment urinary level of one or more biomarkers selected from Rac1, Rac1-GTP, phospho-LIM kinase 1, and phospho-cofilin that is above a pre-determined threshold, the method comprising:
   a. obtaining the urinary level of the selected biomarker in the human subject at a time after initiation of TRPC5 therapy;
   b. comparing the level of the selected biomarker in step a. with the pre-treatment urinary level of the selected biomarker;
   c. determining that the TRPC5 inhibitor therapy is efficacious if the level of the selected biomarker in step a. is lower than the pre-treatment urinary level of the selected biomarker.
19. A method of determining the efficacy of a TRPC5 inhibitor therapy in a human subject suffering from a kidney disease, wherein prior to commencing the therapy the subject was determined to have a pre-treatment urinary level of one or more biomarkers selected from Rac1, Rac1-GTP, phospho-LIM kinase 1, and phospho-cofilin that is above a pre-determined threshold, the method comprising:
   a. obtaining the urinary level of the selected biomarker in the human subject at a time after initiation of TRPC5 therapy; and
   b. determining that the TRPC5 inhibitor therapy is efficacious if the level of the selected biomarker in step a. is lower than the pre-determined threshold for the selected biomarker.

## Claims

1. A pharmaceutical composition for use in treating anxiety, depression, a kidney disease or cancer in a human subject:
wherein the subject has and is selected for treatment on the basis of having a urinary level of one or more biomarkers selected from Rac1, Rac1-GTP, phospho-LIM kinase 1, and phospho-cofilin above a pre-determined threshold; and
the pharmaceutical composition comprises a TRPC5 inhibitor or a calcineurin inhibitor;
and a pharmaceutically acceptable carrier;
wherein the TRPC5 inhibitor is a compound of structural formula X: 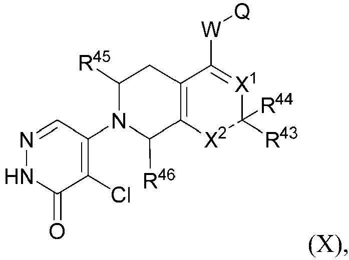 or a pharmaceutically acceptable salt thereof, wherein:
"---" is a single bond or a double bond;
X¹ is CH or N;
when "---" is a double bond, X² is CH or N;
when "---" is a single bond, X² is N(CH₃),
when X¹ is CH, X² is N or N(CH₃);
W is -O-, -N(CH₃)-, -N(CH₂CH₂OH)-, cyclopropan-1,1-diyl, or -CH(CH₃)-;
Q is 2-trifluoromethyl-4-fluorophenyl, 2-difluoromethyl-4-fluorophenyl, 2-trifluoromethylphenyl, 2-methyl-4-fluorophenyl, 2-chloro-4-fluorophenyl, 2-chlorophenyl, 1-(benzyl)-4-methylpiperidin-3-yl, 4-trifluoromethylpyridin-3-yl, 2-trifluoromethyl-6-fluorophenyl, 2-trifluoromethyl-3-cyanophenyl, 2-ethyl-3-fluorophenyl, 2-chloro-3-cyanophenyl, 2-trifluoromethyl-5-fluorophenyl, or 2-difluoromethylphenyl;
R⁴³ is hydrogen, -CH₂OH, -CH(OH)-CH₂OH, -NH₂, -CH(OH)CH₃, -OCH₃, or -NH-(CH₂)₂OH; and when "---" is a double bond, R⁴⁴ is absent;
and when "---" is a single bond, R⁴³ and R⁴⁴ are taken together to form =O; and
each of R⁴⁵ and R⁴⁶ is independently hydrogen or -CH₃.

2. The pharmaceutical composition for use according to claim 1, wherein the TRPC5 inhibitor is a compound of Formula XI: 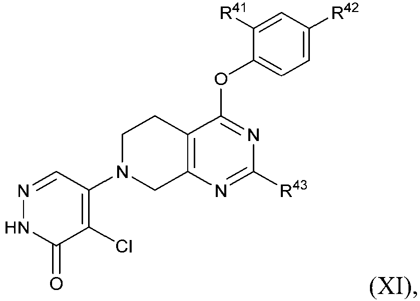 or a pharmaceutically acceptable salt thereof; wherein:
R⁴¹ is chloro, -CF₃, -CHF₂, or -CH₃;
R⁴² is hydrogen or fluoro; and
R⁴³ is hydrogen, -NH₂, -CH₂OH, or CH(OH)-CH₂OH.

3. The pharmaceutical composition for use according to claim 1, wherein the TRPC5 inhibitor is: 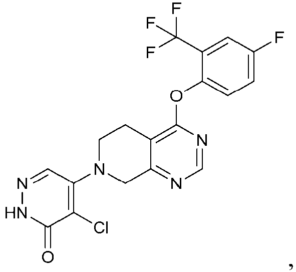 or a pharmaceutically acceptable salt thereof.

4. The pharmaceutical composition for use according to claim 1, wherein the calcineurin inhibitor is cyclosporin A, tacrolimus, or voclosporin, or a pharmaceutically acceptable salt thereof.

5. The pharmaceutical composition for use according to any one of claims 1-4, wherein the composition is for use in the treatment of anxiety or depression.

6. The pharmaceutical composition for use according to any one of claims 1-4, wherein the kidney disease is diabetic nephropathy, focal segmental glomerulosclerosis, minimal change disease, membranoproliferative glomerulonephritis (including post-streptococcal glomerulonephritis and bacterial endocarditis-associated glomerulonephritis), membranous nephropathy, other hepatitis C virus-associated glomerulopathies, HIV-associated glomerulopathies, COVID-19-associated acute kidney injury, Alport syndrome, polycystic kidney disease (both autosomal dominant and autosomal recessive), IgA nephropathy, other genetic nephropathies or ciliopathies (e.g., HNF1beta, nephronophthisis, autosomal dominant cystic/tubular kidney disease), lupus nephritis, Goodpasture's syndrome (anti-GBM disease), and another complement- or immune-mediated kidney disease.

7. The pharmaceutical composition for use according to claim 6, wherein the kidney disease is diabetic nephropathy, or focal segmental glomerulosclerosis; or autosomal dominant polycystic kidney disease of autosomal recessive polycystic kidney disease.

8. The pharmaceutical composition for use according to any one of claims 1-7, wherein the subject has, and is selected on the basis of having, a urinary level of Rac1 above a pre-determined threshold.

9. The pharmaceutical composition for use according to claim 8, wherein the urinary Rac1 level in a subject is measured in a fraction of urine comprising extracellular vesicles.

10. The pharmaceutical composition for use according to claim 8 or 9, wherein the pre-determined threshold level is established by determining the range of urinary levels of the selected biomarker in a population of healthy humans; and establishing the pre-determined threshold level for the selected biomarker at a level above the 75^{th} percentile in the population.

11. The method of claim 10, wherein the pre-determined threshold level is at a level above the 90^{th} percentile in the population; or the pre-determined threshold level is at a level above the 95^{th} percentile in the population.

12. The pharmaceutical composition for use according to claim 8 or 9, wherein the pre-determined threshold level for urinary Rac1 is between 100-500 pg/mL.

13. A method of determining the efficacy of a TRPC5 inhibitor therapy in a human subject suffering from anxiety, depression, a kidney disease or cancer, wherein prior to commencing the therapy the subject was determined to have a pre-treatment urinary level of one or more biomarkers selected from Rac1, Rac1-GTP, phospho-LIM kinase 1, and phospho-cofilin that is above a pre-determined threshold, the method comprising:
a. obtaining the urinary level of the selected biomarker in the human subject at a time after initiation of TRPC5 therapy;
b. comparing the level of the selected biomarker in step a. with the pre-treatment urinary level of the selected biomarker;
c. determining that the TRPC5 inhibitor therapy is efficacious if the level of the selected biomarker in step a. is lower than the pre-treatment urinary level of the selected biomarker.

14. A method of determining the efficacy of a TRPC5 inhibitor therapy in a human subject suffering from anxiety, depression, a kidney disease or cancer, wherein prior to commencing the therapy the subject was determined to have a pre-treatment urinary level of one or more biomarkers selected from Rac1, Rac1-GTP, phospho-LIM kinase 1, and phospho-cofilin that is above a pre-determined threshold, the method comprising:
a. obtaining the urinary level of the selected biomarker in the human subject at a time after initiation of TRPC5 therapy; and
b. determining that the TRPC5 inhibitor therapy is efficacious if the level of the selected biomarker in step a. is lower than the pre-determined threshold for the selected biomarker.
